# EUROPÄISCHE PATENTANMELDUNG

(11) **EP 2 684 562 A1**
(43) Veröffentlichungstag der Anmeldung: **15.01.2014**
(21) Anmeldenummer: 13188161.7
(22) Anmeldetag: 07.08.2009
(51) Int. Cl.: A61K 9/50, C07K 14/435, A61K 9/70

(54) **Wirkstoffhaltige Fasernflächengebilde auf Basis von Biopolymeren, ihre Anwendungen und Verfahren zu ihrer Herstellung**

(30) Priorität: 08.08.2008 EP 08162121; 27.03.2009 EP 09156540
(62) Teilanmeldung aus: 09777749.4
(71) Anmelder: BASF SE, 67056 Ludwigshafen (DE)
(72) Erfinder: Liebmann, Burghard, 64625 Bensheim (DE); Klimov, Evgueni, 67063 Ludwigshafen (DE)
(74) Vertreter: Reitstötter - Kinzebach

(57) **Zusammenfassung**

Die Erfindung betrifft wirkstoffhaltige Faserflächengebilde auf Basis von Biopolymeren, umfassend einen faserförmigen, biopolymeren Wirkstoffträger und wenigstens einen mit dem Träger assoziierten und von dem Faserflächengebilde freisetzbaren Wirkstoff; wirkstoffhaltige Formulierungen, umfassend solche Faserflächengebilde; die Verwendung erfindungsgemäßer wirkstoffhaltiger Faserflächengebilde zur Herstellung wirkstoffhaltiger Formulierungen; und Verfahren zur Herstellung erfindungsgemäßer Faserflächengebilde. Weiterhin betrifft die Erfindung korrespondierende wirkstofffreie Faserflächengebilde und deren Verwendung zur Herstellung von Wundversorgungs- und Hygieneartikeln, sowie die entsprechend hergestellten Wundversorgungs- und Hygieneartikel selbst.

## Beschreibung

Die Erfindung betrifft wirkstoffhaltige Faserflächengebilde auf Basis von Biopolymeren, umfassend einen faserförmigen, biopolymeren Wirkstoffträger und wenigstens einen mit dem Träger assoziierten und von dem Faserflächengebilde freisetzbaren Wirkstoff; wirkstoffhaltige Formulierungen, umfassend solche Faserflächengebilde; die Verwendung erfindungsgemäßer wirkstoffhaltiger Faserflächengebilde zur Herstellung wirkstoffhaltiger Formulierungen; und Verfahren zur Herstellung erfindungsgemäßer Faserflächengebilde. Weiterhin betrifft die Erfindung korrespondierende wirkstofffreie Faserflächengebilde und deren Verwendung zur Herstellung von Wundversorgungs- und Hygieneartikeln, sowie die entsprechend hergestellten Wundversorgungs- und Hygieneartikel selbst.

### Stand der Technik:

Die WO-A-2007/082936 beschreibt die Verwendung amphiphiler, selbstassemblierender Proteine zur Formulierung schwer wasserlöslicher Wirkstoffe durch Dispergieren der Effektstoffe in einem proteinhaltigen Schutzkolloid. Nach Mischung der schwer wasserlöslicher Wirkstoffe und der amphiphilen, selbstassemblierenden Proteine in einer gemeinsamen dispersen Phase und anschließender Phasentrennung in eine protein- und effektstoffreiche Phase sowie eine protein- und effektstoffarme Phase liegen Protein-Microbeads vor, in welche die schwer wasserlöslichen Wirkstoffe verkapselt sind.

Verschiedene Publikationen beschreiben die Herstellung von Fasern durch Spinnverfahren aus chemisch synthetisierten Polymeren und Biopolymeren sowie Proteinen.

Zur Herstellung von Nano- und Mesofasern sind dem Fachmann eine Vielzahl an Verfahren bekannt, von denen dem Elektrospinnverfahren ("Electrospinning") derzeit die größte Bedeutung zukommt. Bei diesem Verfahren, welches beispielsweise von D.H. Reneker, H.D. Chun in Nanotechn. 7 (1996), Seite 216 f. beschrieben ist, wird üblicherweise eine Polymerschmelze oder eine Polymerlösung an einer als Elektrode dienenden Kante einem hohen elektrischen Feld ausgesetzt. Dies kann beispielsweise dadurch erreicht werden, dass die Polymerschmelze oder Polymerlösung in einem elektrischen Feld unter geringem Druck durch eine mit einem Pol einer Spannungsquelle verbundene Kanüle extrudiert wird. Aufgrund der dadurch erfolgenden elektrostatischen Aufladung der Polymerschmelze oder Polymerlösung entsteht ein auf die Gegenelektrode gerichteter Materialstrom, der sich auf dem Wege zur Gegenelektrode verfestigt. In Abhängigkeit von den Elektrodengeometrien werden mit diesem Verfahren Vliese bzw. so genannte Nonwovens oder Ensembles geordneter Fasern erhalten.

In DE-A1-10133393 wird ein Verfahren zur Herstellung von Hohlfasern mit einem Innendurchmesser von 1 bis 100 nm offenbart, bei dem eine Lösung eines wasserunlöslichen Polymers - beispielsweise eine Poly-L-lactid-Lösung in Dichlormethan oder eine Polyamid-46-Lösung in Pyridin - elektroversponnen wird. Ein ähnliches Verfahren ist auch aus WO-A1-01/09414 und DE-A1-10355665 bekannt.

Aus DE-A1-19600162 ist ein Verfahren zur Herstellung von Rasenmäherdraht oder textilen Flächengebilden bekannt, bei dem Polyamid, Polyester oder Polypropylen als fadenbildendes Polymer, ein maleinsäureanhydrid-modifizierter Polyethylen/Polypropylen-Kautschuk sowie ein oder mehrere Alterungsstabilisatoren zusammengegeben, aufgeschmolzen und miteinander vermischt werden, bevor diese Schmelze schmelzversponnen wird.

DE-A1-10 2004 009 887 betrifft ein Verfahren zur Herstellung von Fasern mit einem Durchmesser von < 50 µm durch elektrostatisches Verspinnen oder Versprühen einer Schmelze von mindestens einem thermoplastischen Polymeren.

Durch das Elektrospinnen von Polymerschmelzen lassen sich nur Fasern mit Durchmessern größer 1 µm herstellen. Für eine Vielzahl von Anwendungen, z.B. Filtrationsanwendungen, werden jedoch Nano- und/oder Mesofasern mit einem Durchmesser von weniger als 1 µm benötigt, die sich mit den bekannten Elektrospinnverfahren nur durch Einsatz von Polymerlösungen herstellen lassen.

Ein weiteres geeignetes Verfahren zur Herstellung von Faservliesen ist Zentrifugenspinnen (auch Rotorspinnen genannt). In EP-B1-0624665 und EP-A1-1088918 (beide BASF Anmeldungen) wird ein Verfahren zur Herstellung von Fasergebilden aus Melamin-Formaldehyd-Harz und ihrer Blends mit thermoplastischen Polymeren mittels Zentrifugalspinnverfahren auf einem Schleuderteller offenbart.

Das Verfahren und die Einrichtung zur Herstellung von Fasern aus Schmelze unterschiedlicher Polymermaterialien mit Hilfe der Zentrifugalkräfte werden in DE-A-102005048939 dargestellt.

Die Verarbeitung von Spinnenseidenproteinen der Spinne *Nephila clavipes* aus einer Hexafluoro-2-propanol-Lösung zu Nanofasern mittels des Elektroverspinnens wurde 1998 von Zarkoob und Reneker beschrieben (Polymer 45: 3973-3977, 2004). Spinnversuche von *Bombyx mori* Seide aus einer Ameisensäurelösung werden von Sukigara und Ko (Polymer 44: 5721-572, 2003) offenbart, in denen durch Variation der Elektrospinnparameter die Fasermorphologie beeinflusst wird. Jin und Kaplan berichteten von Wasser basiertem Elektrospinning von Seide bzw. Seide/Polyethylenoxid (Biomacromolecules 3: 1233-1239, 2002).

Die WO-A-03/060099 beschreibt verschiedene Methoden (u.a. Elektroverspinnen) und Apparaturen zum Verspinnen von *Bombyx mori* Seidenproteinen und Spinnenseidenproteinen. Die verwendeten Spinnenseidenproteine wurden dabei rekombinant mit transgenen Ziegen produziert und aus deren Milch aufgereinigt sowie anschließend versponnen.

Die WO-A-01/54667 beschreibt die Herstellung von pharmazeutischen Zusammensetzungen, umfassend einen pharmazeutisch verträglichen polymeren Träger, hergestellt durch Elektroverspinnung von organischen Polymeren, wie insbesondere Polyethylenoxid, wobei in den Trägern ein pharmazeutisches Agens enthalten ist. Die WO 04/014304 beschreibt entsprechende pharmazeutische Zusammensetzungen mit polymeren Trägern, erhalten durch Elektroverspinnung von Polyacrylaten, Polymetacrylaten, Polyvinylpyrrolidolen oder Polyvinylpyrrolidon oder Polyvinylpyrrolidon-Polyvinylacetatcopolymeren.

In der WO-A-2007/082936 ist die Formulierung schwer wasserlöslicher Effektstoffe mit Hilfe von amphiphilen, selbstassemblierenden Proteinen beschrieben. Dabei werden durch induzierte Phasentrennungsprozesse sogenannte Protein-Microbeads gebildet. Eine effiziente Formulierung von wasserlöslichen Wirkstoffen ist mit diesem Verfahren jedoch nicht möglich.

Die bisher bekannten Verfahren zur Formulierung von Wirk- und Effektstoffen erfüllen nicht alle Anforderungen, die an einen insbesondere für pharmazeutischen Einsatz formulierten Wirkstoff gestellt werden, wie mechanische Stabilität, toxische Unbedenklichkeit, Biokompatibilität, hohe Wirkstoff-Bioverfügbarkeit.

Weiterhin liegen die mit bekannten Verfahren formulierten Wirkstoffe oft in kristalliner Form vor, was ihre Bioverfügbarkeit deutlich herabsetzt. Insbesondere die kontinuierliche, verzögerte, gezielte Freisetzung der Wirkstoffe über längere Zeiträume stellt eine besondere Herausforderung bei der Herstellung einer geeigneten Formulierung dar.

Außerdem ist aus dem Stand der Technik bisher noch kein Verfahren bekannt, welches gleichermaßen für die Formulierung verschiedenster Wirkstoffklassen in einem polymeren Träger geeignet ist.

### Zusammenfassung der Erfindung:

Es bestand daher die Aufgabe, ein Verfahren bereitzustellen, das die Formulierung im wesentlichen aller Wirkstoffklassen unter Verwendung geeigneter Träger als Formulierungshilfsstoff erlaubt und dabei gegebenenfalls einzelne oder mehrere der oben genannten Kriterien besser erfüllt als die aus dem Stand der Technik bekannten Verfahren.

Auf dem Gebiet der pharmazeutischen Wirkstoffe, insbesondere der Husten- und Schleimlöser der Guaiacol-Derivate gibt es Referenzprodukte, wie z.B. Tabletten der Handelsmarke Mucinex^{®}, welche kontinuierliche, verzögerte Freisetzungsprofile, wie z.B. des Wirkstoffes Guaiacol Glyceryl Ether (auch Guaifenesin) zeigen. Allerdings wird hier eine Wirkstoff-Freisetzung nur unter Magenbedingungen erzielt. Darmbedingungen führen nicht zur Wirkstoff-Freisetzung. Es werden größtenteils chemisch synthetisierte, nicht biokompatible Polymere als Formulierungshilfsstoff genutzt, welche keinen weiteren Nutzen, z.B. eine Erhöhung der Wirkstoff-Bioverfügbarkeit durch gesteigerte Resorption, besitzen. Es stellte sich demnach die weitere Aufgabe, eine biokompatible Formulierung für husten- und schleimlösende Wirkstoffe, wie z.B. Guaiacol Glyceryl Ether, bereitzustellen, die eine kontinuierliche und verzögerte dabei aber auch proteolytisch, über im Magen-Darmtrakt vorkommende Proteasen, getriggerte Wirkstoff-Freisetzung unter den dort herrschenden Bedingungen erlaubt.

Obige Aufgaben konnten überraschenderweise durch Bereitstellung wirkstoffhaltiger Faserflächengebilde gelöst werden, umfassend einen faserförmigen polymeren Wirkstoffträger und einen mit dem Träger assoziierten freisetzbaren Wirkstoff, wobei der Träger als Polymerkomponente wenigstens ein Biopolymer umfasst.

Insbesondere können erfindungsgemäß die natürlicherweise, z.B. im Magen-DarmTrakt, im Boden (durch Mikroorganismen) oder auf der Haut, vorkommenden Proteasen als gezielter, steuerbarer Triggermechanismus für die kontinuierliche und verzögerte Freisetzung der Wirkstoffe aus den hier beschriebenen neuen Formulierungen genutzt werden. Weiterhin können mit den hier beschriebenen Verfahren Wirkstoff-Formulierungen hergestellt werden, in denen der Wirkstoff auch in amorpher Zustandsform oder als feste Lösung vorliegt. Diese können im Gegensatz zur kristallinen Zustandsform eine erhöhte Wirkstoff-Bioverfügbarkeit bewirken, welche in Kombination mit den biopolymeren Formulierungshilfsstoffen, wie den amphiphilen, selbstassemblierenden Proteinen nochmals gesteigert werden kann.

### Figurenbeschreibung:

In den beiliegenden Figuren zeigt
- Fig. 1: eine elektronenmikroskopische (SEM) Aufnahme von C16-Spinnenseidenprotein-Flächengebilden (Fasern) mit eingeschlossenem Wirkstoff Guaiacol Glyceryl Ether (GGE);
- Fig. 2: Kristallinitätsuntersuchungen (WAXS in Transmission) des Wirkstoffes GGE in den durch Elektrospinnen gewonnenen C16-Spinnenseidenprotein-Formulierungen im Vergleich zu den Reinsubstanzen (GGE bzw C16-Pulver);
- Fig. 3: die Freisetzung des Wirkstoffes GGE aus einer durch Elektrospinnen gewonnenen und zu Tabletten verpressten C16-Spinnenseidenprotein-Formulierung in Kaliumphosphatpuffer (Kontrolle) sowie artifiziellem Magensaft und Darmsaft. Als 100%-Wert wurde die im zugehörigen Ausführungsbeispiel aufgeführte Gesamtwirkstoffkonzentration angesetzt;
- Fig. 4: die Freisetzung des Wirkstoffs GGE aus handelsüblichen Tabletten der Marke Mucinex^{®} (Firma Adams Respiratory Therapeutics);
- Fig. 5: Elektronenmikroskopische (SEM)-Aufnahmen von C16-Spinnenseidenprotein-Flächengebilden (Fasern) mit dem eingeschlossenen Wirkstoff Clotrimazol;
- Fig. 6: Kristallinitätsuntersuchungen (WAXS in Transmission) des Wirkstoffes Clotrimazol in den durch Elektrospinnen gewonnenen C16-Spinnenseidenprotein-Formulierungen im Vergleich zu Clotrimazolreinsubstanz;
- Fig. 7: die Freisetzung des Wirkstoffes Clotrimazol aus einer durch Elektrospinnen gewonnenen und zu Tabletten verpressten C16-Spinnenseidenprotein-Formulierung in Kaliumphosphatpuffer (Kontrolle) sowie artifiziellem Magensaft und Darmsaft. Als 100%-Wert wurde die im zugehörigen Beispiel aufgeführte Wirkstoffkonzentration angesetzt.
- Fig. 8: Elektronenmikroskopische (SEM)-Aufnahmen von C16-Spinnenseidenproteinflächengebilden (Fasern) mit dem eingeschlossenen Wirkstoff Metazachlor.
- Fig. 9: Kristallinitätsuntersuchungen (WAXS in Transmission) des Wirkstoffes Me-tazachlor in den durch Elektrospinnen gewonnenen C16-Spinnenseidenprotein-Formulierungen im Vergleich zu Metazachlor-Reinsubstanz.
- Fig. 10: die Freisetzung des Wirkstoffes Metazachlor aus einer durch Elektrospinnen gewonnenen C16-Spinnenseidenprotein-Formulierung in Kaliumphosphatpuffer (Kontrolle) sowie protolytisch aktiver Proteinase K-Lösung.
- Fig. 11: Elektronenmikroskopische (SEM) Aufnahmen von C16-Spinnenseidenproteinflächengebilden (Fasern) mit eingeschlossenem Wirkstoff Uvinul A+.
- Fig. 12: Kristallinitätsuntersuchungen (WAXS in Transmission) des Wirkstoffes U-vinul A+ in den durch Elektrospinnen gewonnenen C16-Spinnenseidenprotein-Formulierungen im Vergleich zu Uvinul A+-Reinsubstanz.
- Fig. 13: die Freisetzung des Wirkstoffes Uvinul A+ aus einer durch Elektrospinnen gewonnenen C16-Spinnenseidenprotein-Formulierung in Kaliumphosphatpuffer (Kontrolle) sowie in protolytisch aktiver Proteinase K-Lösung.
- Fig. 14: Licht- bzw. Elektronenmikroskopische (SEM) Aufnahmen von (A) R16-Protein-Flächengebilden (Fasern) und (B) S16-Protein-Flächengebilden (Fasern).
- Fig. 15: Elektronenmikroskopische (SEM) Aufnahmen von R16- (vgl. (A)) und S16 (vgl (B)) Protein-Flächengebilden mit eingeschlossenem Wirkstoff Uvinul A+.
- Fig. 16: Kristallinitätsuntersuchungen (WAXS in Transmission) des Wirkstoffes U-vinulA+ im durch Elektrospinnen gewonnenen R16-Protein-Vliesstoff (A) und S16-Protein-Vliesstoff (B) im Vergleich zu Uvinul A+-Reinsubstanz.
- Fig. 17: die Freisetzung des Wirkstoffes Uvinul A+ aus einem durch Elektrospinnen gewonnenen R16-Protein-Vliesstoff (A) und S16-Protein-Vliesstoff (B) in Kaliumphosphatpuffer (Kontrolle) sowie in proteolytisch aktiver Proteinase K-Lösung.

### Detaillierte Beschreibung der Erfindung:

### 1. Definition verwendeter Begriffe:

Werden keine anderen Angaben gemacht, so gelten im Rahmen der vorliegenden Beschreibung folgende Bedeutungen technischer Begriffe:

Unter einem "Trägerpolymer" versteht man Biopolymere bzw. ihre Abmischungen, oder auch Abmischungen aus mindestens einem synthetischen Polymer und einem Biopolymer, wobei das Trägerpolymer die Fähigkeit besitzt, mit dem oder den zu formulierenden Wirkstoffen/Effektstoffen nicht-kovalente Wechselwirkungen einzugehen, bzw. partikuläre Wirkstoffe (dispergiert oder kristallin) zu umschließen oder zu adsorbieren (tragen).

Bei einer "nicht-kovalenten" Wechselwirkung handelt es sich um alle, dem Fachmann bekannten Typen von Bindungen, wobei keine Ausbildung von kovalenten Bindungen zwischen Wirkstoff und Trägerpolymer erfolgt. Als Beispiel können, ohne auf diese beschränkt zu sein, folgende genannt werden: Wasserstoffbrückenbildung, Komplexbildung, Ionenwechselwirkung.

Unter einem "Wirkstoff" oder "Effektstoff" versteht man synthetische oder natürliche, niedermolekulare Substanzen mit hydrophilen, lipophilen oder amphiphilen Eigenschaften, welche in der Agrochemie, Pharmazie, Kosmetik oder Nahrungs- und Futtermittelindustrie Anwendung finden können; ebenso wie biologische aktive Makromoleküle welche in ein erfindungsgemäßes Faserflächengebilde eingebettet oder daran adsorbiert werden können, wie z.B. Peptide (wie Oligopeptide mit 2 bis 10 Aminosäureresten und Polypeptide mit mehr als 10, wie z.B. 11 bis 100 Aminosäureresten) sowie Enzyme und einzel- oder doppelsträngige Nukleinsäuremoleküle (wie Oligonukleotide mit 2 bis 50 Nukleinsäuretesten und Polynukleotide mit mehr als 50 Nukleinsäureresten).

"Niedermolekular" bedeutet dabei Molmassen von weniger als 5000, insbesondere weniger als 2000, wie beispielsweise 100 bis 1000 Gramm pro Mol.

"Hochmolekular" bedeutet dabei Molmassen von mehr als 5000, insbesondere weniger als 10.000, wie beispielsweise 10.000 bis 1.000.000 Gramm pro Mol. Die Begriffe "Wirkstoff" und "Effektstoff" werden als Synonym verwendet.

Der Begriff "Faserflächengebilde" umfasst erfindungsgemäß sowohl einzelne Polymerfasern als auch die geordnete oder ungeordnete ein- oder mehrlagige Zusammenlagerung einer Vielzahl solcher Fasern, beispielsweise zu Faservliesen bzw. Nonwoven-Vliesen.

Ein "Wirkstoffträger" ist faserförmig ausgebildet und trägt, vorzugsweise in adsorbierter, nicht-kovalent gebundener Form an der Faseroberfläche und/oder in das Fasermaterial integriert, den oder die erfindungsgemäß zu verarbeiteten Wirkstoffe. Der Wirkstoff kann dabei über die Faser gleichmäßig oder ungleichmäßig verteilt vorliegen. Der Wirkstoff kann zudem in amorpher, teilkristalliner oder kristalliner Form am/im Wirkstoffträger reversibel adsorbiert sein.

Ein "löslicher" Wirkstoffträger ist in einem wässrigen oder organischen Lösungsmittel, vorzugsweise einem wässrigen Lösungsmittel, wie beispielsweise Wasser oder einem Wasser-basierten Lösungsmittel, in einem pH-Bereich von pH 2 bis 13, wie z.B. 4 bis 11, teilweise oder vollständig löslich. Somit kann die Löslichkeit in Wasser in einem großen Bereich variieren - d.h. von guter, d.h. rascher und vollständiger oder im Wesentlichen vollständiger Löslichkeit bis sehr langsamer und vollständiger oder unvollständiger Löslichkeit.

Als "synthetische" polymere Bestandteile der erfindungsgemäßen Wirkstoffzubereitungen eignen sich prinzipiell alle Polymere, die in einem Temperaturbereich zwischen 0 und 240°C, einem Druckbereich zwischen 1 und 100 bar, einem pH-Bereich von 0 bis 14 oder Ionenstärken bis 10 mol/l in Wasser oder/und in organischen Lösungsmitteln löslich sind.

Eine "wässrige Polymerdispersion" im Sinne der vorliegenden Erfindung bezeichnet, auch in Übereinstimmung mit allgemeinem Fachwissen, eine Mischung von mindestens zwei miteinander nicht oder im Wesentlichen nicht mischbaren Phasen, wobei eine der wenigstens zwei Phasen Wasser ist, und die zweite wenigstens ein im Wesentlichen wasserunlösliches Polymer enthält, insbesondere daraus besteht. Unter "im Wesentlichen wasserunlöslichen Polymeren" sind im Sinne der vorliegenden Erfindung insbesondere Polymere mit einer Löslichkeit in Wasser von weniger als 0,1 Gew.-%, bezogen auf das Gesamtgewicht der Lösung zu verstehen.

Ein "abbaubarer" Wirkstoffträger liegt dann vor, wenn die Faserstruktur durch chemische, biologische oder physikalische Prozesse, wie beispielsweise durch Einwirkung von Licht oder andere Strahlung, Lösungsmittel, chemische oder biochemische Oxidation, Hydrolyse, Proteolyse teilweise oder vollständig zerstört wird. Biochemische Prozesse können dabei von Enzymen oder Mikroorganismen, wie beispielsweise von Prokaryonten oder Eukaryonten, wie z.B. Bakterien, Hefen, Pilzen vermittelt werden.

Unter "Mischbarkeit" von Polymeren versteht man erfindungsgemäß, dass bei einem Gemisch aus wenigstens zwei verschiedenen synthetischen Polymeren oder Biopolymeren ein Polymer für das andere als Lösungsmittel fungieren kann. Dies bedeutet, dass ein einphasiges System zwischen den zwei verschiedenen Polymeren entsteht. Bei nicht-mischbaren Komponenten werden entsprechend zwei unterschiedliche Phasen vorliegen.

Unter einem "Kompositpolymer" versteht man erfindungsgemäß ein homogenes oder inhomogenes Gemisch aus wenigstens einer faserbildenden Polymerkomponente mit wenigstens einem niedermolekularen oder hochmolekularen Zusatz, wie insbesondere einem nicht einpolymerisierbaren Zusatz, wie beispielsweise einem Wirkstoff oder Effektstoff gemäß obiger Definition.

Unter einer "prozessierten Form" eines Faserflächengebildes versteht man, dass das ursprünglich bei der Herstellung des Faserflächengebildes anfallende Produkt weiterverarbeitet wird; beispielsweise, dass die Fasern komprimiert oder tablettiert werden, auf einen weiteren Träger aufgebracht werden und/oder einer Zerkleinerung zur Verkürzung der Faserlänge unterzogen werden.

Werden keine anderen Angaben gemacht, so betreffen Molekulargewichtsangaben für Polymere Mn oder Mw-Werte

### 2. Bevorzugte Ausführungsformen:

Ein erster Gegenstand der Erfindung betrifft ein wirkstoffhaltiges Faserflächengebilde, umfassend einen faserförmigen, polymeren, löslichen und/ oder abbaubaren Wirkstoffträger und einen oder mehrere, wie z. B. 2, 3, 4 oder 5, mit dem Träger assoziierte, und von dem Faserflächengebilde freisetzbare niedermolekulare oder hochmolekulare Wirkstoffe, wobei der Träger als Polymerkomponente ein oder mehrere, wie z.B. 2, 3, 4 oder 5, struktur- oder gerüstbildende, leicht aggregierende, z.T. höhermolekulare Biopolymere umfasst, die gegebenenfalls zusätzlich chemisch und/oder enzymatisch modifiziert sein können, wie z.B. durch Veresterung, Amidierung, Verseifung, Carboxylierung, Acetylierung, Acylierung, Hydroxylierung, Glycosylierung und Farnesylierung.

Dabei ist das Faserflächengebilde insbesondere erhältlich mittels eines Spinnverfahrens, insbesondere durch Elektroverspinnung einer elektroverspinnbaren Lösung, welche das wenigstens eine Biopolymer und den wenigstens einen Wirkstoff insbesondere in gelöster Form umfasst. In dem Faserflächengebilde liegt der wenigstens eine Wirkstoff in amorpher, teilkristalliner oder kristalliner Form vor.

Der Wirkstoff ist dabei in den Träger integriert (eingebettet) und/oder daran adsorbiert.

Das Biopolymer ist vorzugsweise ein Protein, insbesondere ein amphiphiles, selbstassemblierendes Protein.

Vorzugsweise handelt es sich bei den amphiphilen selbstassemblierenden Proteinen um Microbead-bildende Proteine.

Vorzugsweise handelt es sich bei den amphiphilen selbstassemblierenden Proteinen um intrinsisch entfaltete Proteine.

Insbesondere ist das amphiphile selbstassemblierende Protein ein Seidenprotein, wie z.B. ein Spinnenseidenprotein.

Ein Beispiel eines geeigneten Spinnenseidenproteins stellt das C16-Spinnenseidenprotein, umfassend eine Aminosäuresequenz gemäß SEQ ID NO: 2 oder ein von diesem Protein abgeleitetes verspinnbares Protein mit einer Sequenzidentität von wenigstens etwa 60%, wie z.B. wenigstens etwa 70, 80, 90, 95, 96, 97, 98 oder 99% dar.

Beispiele für andere intrinsisch entfaltete, amphiphile selbstassemblierende Proteine sind das R16-Protein, umfassend eine Aminosäuresequenz gemäß SEQ ID NO: 4 oder das S16-Protein, umfassend eine Aminosäuresequenz gemäß SEQ ID NO: 6; oder ein von diesen Proteinen abgeleitetes verspinnbares Protein mit einer Sequenzidentität von wenigstens etwa 60%, wie z.B. wenigstens etwa 70, 80, 90, 95, 96, 97, 98 oder 99%.

Insbesondere sind Faserflächengebilde Gegenstand der Erfindung, wobei wenigstens ein pharmazeutischer Wirkstoff enthalten ist, wie z.B. ein husten- und schleimlösender Wirkstoff (Expektorans); wie insbesondere der Wirkstoff Guaiacol Glyceryl Ether (Guaifenesin; CAS-Nummer 93-14-1) oder ein Derivat davon.

Weiterhin ist Gegenstand der Erfindung ein Faserflächengebilde, wobei der Wirkstoff ein Pflanzenschutzwirkstoff, oder ein haut- und/oder haarkosmetischer Wirkstoff ist.

Weiterhin ist Gegenstand der Erfindung ein Faserflächengebilde, wobei der Träger wenigstens eine weitere Polymerkomponente umfasst, die ausgewählt ist unter synthetischen Polymeren, wie insbesondere synthetischen Homo- oder Copolymeren.

Gegenstand der Erfindung sind auch solche Faserflächengebilde, wobei der polymere Träger ein Kompositpolymer ist, der ausgewählt ist unter
a. Mischungen aus mindestens 2 mischbaren Biopolymeren;
b. Mischungen aus mindestens 2 nicht mischbaren Biopolymeren;
c. Mischungen aus mindestens einem synthetischen Homo- oder Copolymer und mindestens einem Biopolymer, die miteinander mischbar sind;
d. Mischungen aus mindestens einem synthetischen Homo- oder Copolymer und mindestens einem Biopolymer, die miteinander nicht mischbar sind.

In den erfindungsgemäßen Faserflächengebilden weist die synthetische Polymerkomponente eine Molmasse (Mw) im Bereich von etwa 500 bis 10.000.000 auf, wie z.B. 1.000 bis 1.000.000, oder 10.000 bis 500.000 oder 25.000 bis 250.000.

Der Durchmesser der erfindungsgemäßen Wirkstoffträgerfasern beträgt etwa 10 nm bis 100 µm, wie 50 nm bis 10 µm, oder 100 nm bis 2 µm. Deren Wirkstoffbeladung beträgt etwa 0.01 bis 80 Gew.-%, wie z.B. etwa 1 bis 70 Gew.-% oder etwa 10 bis 50 Gew.-%, jeweils bezogen auf den Feststoffgehalt des Faserflächengebildes.

Insbesondere ist das erfindungsgemäße Faserflächengebilde ausgewählt unter Polymerfasern, Polymerfilmen und Polymervliesstoffen.

Erfindungsgemäße Faserflächengebilde können weiterhin dadurch gekennzeichnet sein, dass Trägerpolymerkomponenten und Wirkstoffe nicht-kovalent wechselwirken (d.h. insbesondere eine molekulare Lösung bilden).

Ein weiterer Gegenstand der Erfindung betrifft wirkstoffhaltige Formulierungen, umfassend ein Faserflächengebilde nach obiger Definition in prozessierter Form, gegebenenfalls in Kombination mit wenigstens einem weiteren Formulierungshilfsmittel.

Beispielsweise kann das Faserflächengebilde darin in zerkleinerter oder nichtzerkleinerter Form vorliegen.

Außerdem können die Formulierungen Faserflächengebilde in kompaktierter (gepresster) Form (wie Tabletten oder Kapseln), in Pulverform, oder aufgetragen auf ein Trägersubstrat, umfassen.

Die erfindungsgemäßen Formulierungen sind insbesondere ausgewählt unter kosmetischen (insbesondere haut und haarkosmetischen), human- und tierpharmazeutischen, agrochemischen, insbesondere fungiziden, herbiziden, insektiziden und sonstige Pflanzenschutzformulierungen, Formulierungen sowie Nahrungs- und Futtermittelzusätzen, wie Nahrungs- und Futterergänzungsmitteln.

Ein weiterer Gegenstand der Erfindung betrifft die Verwendung eines wirkstoffhaltigen Faserflächengebildes nach obiger Definition zur Herstellung einer erfindungsgemäßen wirkstoffhaltigen Formulierung; sowie die Verwendung einer wirkstoffhaltigen Formulierung nach obiger Definition zur kontrollierten Abgabe (Freisetzung) eines darin enthaltenen Wirkstoffs.

Schließlich ist Gegenstand der Erfindung ein Verfahren zur Herstellung eines Faserflächengebildes nach obiger Definition, wobei man
a. wenigstens einen Wirkstoff zusammen mit der wenigstens einer Biopolymerkomponente in einer gemeinsamen flüssigen Phase mischt und
b. anschließend die Einbettung (Adsorption) des Wirkstoffes in (an) die Biopolymerfaser mittels Spinnverfahren durchführt.

Insbesondere verfährt man dabei so, dass man wenigstens einen Wirkstoff und die Polymerkomponente in einer Lösungsmittelphase mischt und aus dieser Mischung verspinnt; oder dass man wenigstens einen Wirkstoff und die Polymerkomponente in einem Gemisch aus wenigstens zwei miteinander mischbaren Lösungsmitteln mischt, wobei Wirkstoffe und Polymere mindestens in einem der Lösungsmittel löslich sind, und man aus dieser Mischung verspinnt.

Insbesondere ist Gegenstand der Erfindung ein Verfahren zur Herstellung eines Faserflächengebildes, wobei das Biopolymer ein amphiphiles, selbstassemblierendes Protein ist, das man mit wenigstens einem Wirkstoff in Ameisensäure mischt und anschließend aus dieser Mischung verspinnt.

Vorzugsweise findet als Spinnverfahren ein Elektrospinnverfahren oder ein Zentrifugen(Rotor)spinnverfahren Anwendung.

Dabei arbeitet man insbesondere bei einer Temperatur im Bereich von etwa 5 bis 50°C.

Weiterhin betrifft die Erfindung Faserflächengebilde umfassend oben bezeichnetes Trägermaterial, welche jedoch im wesentlichen frei ist von Wirkstoffen, insbesondere niedermolekularen Wirkstoffen.

Gegenstand der Erfindung ist weiterhin die Verwendung solcher Faserflächengebilde zu Herstellung einer wirkstoffhaltigen oder wirkstofffreien Formulierung, welche z.B. ausgewählt ist unter kosmetischen, human- und tierpharmazeutischen, agrochemischen Formulierungen, Nahrungs- und Futtermittelzusätzen.

Weiterhin betrifft die Erfindung wirkstofffreie Faserflächengebilde, umfassend einen faserförmigen, polymeren löslichen und/ oder abbaubaren Träger, wobei der Träger als Polymerkomponente wenigstens ein Biopolymer umfasst, das gegebenenfalls zusätzlich chemisch und/oder enzymatisch modifiziert ist, und wobei das Biopolymer ein amphiphiles, selbstassemblierendes Protein, ist.; und wobei das Biopolymer insbesondere ein Seidenprotein ist, das ausgewählt ist unter dem R16-Protein, umfassend eine Aminosäuresequenz gemäß SEQ ID NO: 4, und dem S16-Protein, umfassend eine Aminosäuresequenz gemäß SEQ ID NO: 6; oder ein von diesen Proteinen abgeleitetes verspinnbares Protein mit einer Sequenzidentität von wenigstens etwa 60%.

Weiterhin ist Gegenstand der Erfindung die Verwendung solcher wirkstofffreier Faserflächengebilde zur Herstellung von medizinischen Wundbehandlungs- und Wundversorgungsprodukten und Hygieneartikeln.

Gegenstand der Erfindung sind außerdem Wundbehandlungs- und Wundversorgungsprodukte, hergestellt unter Verwendung eines erfindungsgemäßen Faserflächengebildes, wie z.B. Wundauflagen, Pflaster, Tamponaden, Wundkleber, Bandagen, Verbandmaterialien. Die erfindungsgemäßen Wundmaterialen können beispielsweise verwendet werden zur oberflächlichen Abdeckung kleinerer Wunden, wie Schnittwunden, oder größerer Wunden, wie diabetische Wunden, Geschwüren, wie Druckgeschwüre, Operationswunden, Brandwunden, Ekzemen und dergleichen. Beispielsweise können erfindungsgemäße Produkte zum Einsatz gelangen bei der Behandlung von blutenden oder nichtblutenden Wunden oder Verletzungen im Bereich der Haut, der Augen, der Ohren, der Nase, der Mundhöhle, der Zähne, sowie im Körperinneren, wie Operation im Intestinalbereich (Magen, Darm, Leber, Nieren, Harnwege), Thorax (Herz, Lunge), Genitalbereich, Schädel, Muskulatur; bei der Behandlung und Nachsorge von Wunden im Zusammenhang mit der Transplantation von Geweben, Gefäßen oder Organen.

Gegenstand der Erfindung sind außerdem Hygieneartikel, hergestellt unter Verwendung eines erfindungsgemäßen Faserflächengebildes, wie sie üblicherweise im Personal Care Bereich eingesetzt werden, wie Windeln, Inkontinenz-Produkten, Slipeinlagen, Monatsbinden, Tampons, Pads zur Haut- und Gesichtspflege, Wischtücher und dergleichen.

### 3. Weitere Ausgestaltungen der Erfindung:

### (i) Biopolymere

Grundsätzlich zur Ausbildung erfindungsgemäßer Trägerstukturen geeignet sind solche Biopolymere, welche die Fähigkeit besitzen Gerüststrukturen auszubilden und/ oder leicht zu aggregieren. Meist ist hierzu ein hohes Molekulargewicht nötig, was zum nachfolgenden intermolekularen Verschlingen der Molekülketten führen kann. Aber auch intramolekulare, nicht kovalente Wechselwirkungen, wie Wasserstoffbrücken oder hydrophobe Interaktionen, können an der Ausbildung der erfindungsgemäßen Trägerstrukturen beteiligt sein.

Als nicht limitierende Beispiele sind zu nennen: Cellulose, Celluloseether wie z.B. Methylcellulose (Substitutionsgrad 3 - 40 %), Ethylcellulose, Butylcellulose, Hydroxymethylcellulosen; Hydroxyethylcellulosen; Hydroxypropylcellulosen, Isopropylcellulose, Cellulose-Ester, wie z.B. Celluloseacetat, bakterielle Cellulosen, Stärken, modifizierte Stärken wie z.B. Methylether-Stärke, Gummi arabicum, Chitin, Schellak, Gelatine, Chitosan, Pektin, Casein, Alginat, sowie Copolymere und Blockcopolymere aus den Monomeren der o.g. Verbindungen.; sowie Nukleinsäuremoleküle.

Als geeignet Biopolymers besonders zu nennen sind amphiphile, selbstassemblierende Proteine. Amphiphile, selbstassemblierende Proteine bestehen aus Polypeptiden, die aus Aminosäuren, insbesondere aus den 20 natürlich vorkommenden Aminosäuren, aufgebaut sind. Die Aminosäuren können auch modifiziert, beispielsweise acetyliert, glycosyliert, farnesyliert, sein.

Ihre selbstassemblierenden Eigenschaften ermöglichen bestimmten, erfindungsgemäß verwendbaren Proteinen, höhermolekulare Strukturen einzunehmen und damit Wirkstoffe dauerhaft zu verkapseln. Diese amphiphilen, selbstassemblierenden Proteine eignen sich als Formulierungshilfsstoffe in erster Linie für schwer wasserlösliche, hydrophobe Wirkstoffe. Durch ihren amphiphilen Molekülcharakter interagieren diese Proteine stark mit hydrophoben Wirkstoffen und können diese in wässrigen Lösungen stabilisieren. Nachfolgende Phasentrennungsprozesse können zur Verkapselung der hydrophoben Wirkstoffe in eine Protein-Matrix genutzt werden. Die Interaktion von amphi-philen, selbstassemblierenden Proteinen mit stärker wasserlöslichen Wirkstoffen ist deutlich schwächer, weshalb induzierte Phasentrennungsprozesse aus wässriger Lösung z.B. durch Zugabe lyotroper Salze nicht zur effektiven Verkapselung der wasserlöslichen Wirkstoffe z.B. in Microbeads führen. Durch Spinnprozesse können aus wässrigen Lösungen oder organischen Lösungsmitteln, in denen amphiphile, selbstassemblierende Proteine und wasserlösliche Wirkstoffe gelöst oder dispergiert vorliegen, höhermolekulare Proteinstrukturen, wie Protein-Flächengebilde (z.B. Protein-Filme, Protein-Fasern, Protein-Vliesstoffe) hergestellt werden. Auch nicht oder schwer wasserlösliche Wirkstoffe können so verkapselt werden.

Die dabei hergestellten protein- und wirkstoffreichen Phasen können später ausgehärtet und als mechanisch stabile Wirkstoff enthaltende Proteinstrukturen abgetrennt und ggf. getrocknet sowie zu Tabletten oder Kapseln verarbeitet werden.

Geeignete amphiphile, selbstassemblierende Proteine für die Formulierung sowohl wasserlöslicher als auch schwer wasserlöslicher Effektstoffe sind solche Proteine, welche Protein-Microbeads ausbilden können. Protein-Microbeads besitzen eine globuläre Gestalt mit einem mittleren Teilchendurchmesser von 0,1 bis 100, insbesondere von 0,5 bis 20, bevorzugt von 1 bis 5 und besonders bevorzugt von 2 bis 4 p m.

Protein-Microbeads lassen sich bevorzugt durch das im Folgenden beschriebene Verfahren herstellen:

Das Protein wird in einem ersten Lösungsmittel gelöst. Als Lösungsmittel können beispielsweise wässrige Salzlösungen verwendet werden. Insbesondere eignen sich hoch konzentrierte Salzlösungen mit einer Konzentration größer 2, insbesondere größer 4 und besonders bevorzugt größer 5 molar, deren Ionen stärker ausgeprägte chaotrope Eigenschaften aufweisen als Natrium- und Chloridionen. Ein Beispiel für eine solche Salzlösung ist 6 M Guanidiniumthiocyanat oder 9 M Lithiumbromid. Des Weiteren können organische Lösungsmittel zum Lösen der Proteine verwendet werden. Insbesondere eignen sich fluorierte Alkohole oder zyklische Kohlenwasserstoffe oder organische Säuren. Beispiele dafür sind Hexafluorisopropanol, Cyclohexan und Ameisensäure. Die Herstellung der Protein-Microbeads kann in den beschriebenen Lösungsmitteln erfolgen. Alternativ kann dieses Lösungsmittel durch ein weiteres Lösungsmittel z.B. niedrig konzentrierte Salzlösungen (c < 0,5 M) durch Dialyse oder Verdünnung ersetzt werden. Die Endkonzentration des gelösten Proteins sollte zwischen 0,1-100 mg/ml betragen. Die Temperatur, bei der das Verfahren durchgeführt wird, beträgt üblicherweise 0-80, bevorzugt 5-50 und besonders bevorzugt 10 40 °C.

Bei Verwendung von wässrigen Lösungen können diese auch noch mit einem Puffer, bevorzugt im Bereich von pH 4-10, besonders bevorzugt 5 -9, ganz besonders bevorzugt 6 - 8,5 versetzt sein.

Durch Zugabe eines Additivs wird eine Phasentrennung induziert. Dabei entsteht eine in der Mischung von Lösungsmittel und Additiv emulgierte proteinreiche Phase. Aufgrund von Oberflächeneffekten nehmen emulgierte proteinreiche Tröpfchen eine runde Form an. Durch die Wahl des Lösungsmittels, des Additivs und der Proteinkonzentration kann der mittlere Durchmesser der Protein-Microbeads auf Werte zwischen 0,1 µm bis 100 µm eingestellt werden.

Als Additiv können alle Substanzen verwendet werden, die einerseits mit dem ersten Lösungsmittel mischbar sind und andererseits die Bildung einer proteinreichen Phase induzieren. Wird die Microbeadbildung in organischen Lösungsmitteln durchgeführt, so eignen sich dafür organische Substanzen, die eine geringere Polarität als das Lösungsmittel aufweisen, z.B. Toluol. In wässrigen Lösungen können Salze als Additiv verwendet werden, deren Ionen stärker ausgeprägte kosmotrope Eigenschaften aufweisen als Natrium- und Chloridionen (z.B. Ammoniumsulfat; Kaliumphosphat). Die Endkonzentration des Additivs sollte abhängig von der Art des Additivs zwischen 1% und 50 Gew.-% bezogen auf die Proteinlösung betragen.

Die proteinreichen Tröpfchen werden durch Aushärtung fixiert, wobei die runde Form erhalten bleibt. Die Fixierung beruht dabei auf der Ausbildung starker intermolekularer Wechselwirkungen. Die Art der Wechselwirkungen kann nicht-kovalent, z.B. durch die Bildung intermolekularer β-Faltblattkristalle oder kovalent, z.B. durch chemische Quervernetzung sein. Die Aushärtung kann durch das Additiv und / oder durch die Zugabe einer weiteren geeigneten Substanz erfolgen. Die Aushärtung erfolgt bei Temperaturen zwischen 0 und 80°C, bevorzugt zwischen 5 und 60°C.

Diese weitere Substanz kann ein chemischer Quervernetzer sein. Unter einem chemischen Quervernetzer wird dabei ein Molekül verstanden, bei dem mindestens zwei chemisch reaktive Gruppen über einen Linker miteinander verbunden sind. Beispiele dafür sind Sulfhydryl-reaktive Gruppen (z.B. Maleimide, Pydridyldisulfide, α-Haloacetyle, Vinylsulfone, Sulfatoalkylsulfone (bevorzugt Sulfatoethylsulfone)), Aminreaktive Gruppen (z.B. Succinimidylester, Carbodiimde, Hydroxymethyl- Phosphin, Imidoester, PFP-Ester, Aldehyde, Isothiocyanate etc.), Carboxy-reaktive Gruppen (z.B. Amine etc.), Hydroxyl-reaktive Gruppen (z.B. Isocyanate etc.), unselektive Gruppen (z.B. Arylazide etc.) und photoaktivierbare Gruppen (z.B. Perfluorphenylazid etc.). Diese reaktiven Gruppen können mit in Proteinen vorhandenen Amin-, Thiol-, Carboxyl-oder Hydroxylgruppen kovalente Verknüpfungen bilden.

Die stabilisierten Microbeads werden mit einem geeigneten weiteren Lösungsmittel, z.B. Wasser gewaschen und anschließend durch dem Fachmann geläufige Verfahren getrocknet, z.B. durch Lyophilisierung, Kontakttrocknung oder Sprühtrocknung. Der Erfolg der Kugelbildung wird mit Hilfe der Rasterelektronenmikroskopie überprüft.

Für die Herstellung von Protein-Microbeads sind Proteine geeignet, die in wässriger Lösung überwiegend intrinsisch entfaltet vorliegen. Dieser Zustand kann beispielsweise nach einem Algorithmus berechnet werden, der dem Programm IUpred zugrunde liegt (http://iupred.enzim.hu/index.html; The Pairwise Energy Content Estimated from Amino Acid Composition Discriminates between Folded and Intrinsically Unstructured Proteins; Zsuzsanna Dosztänyi, Veronika Csizmök, Peter Tompa and István Simon; J. Mol. Biol. (2005) 347, 827-839). Ein überwiegend intrinsisch entfalteter Zustand wird dann angenommen, wenn für über 50% der Aminosäurereste nach diesem Algorithmus ein Wert > 0,5 berechnet wird (prediction type: long disorder).

### (ii) Seidenproteine

Weitere geeignete Proteine für die Formulierung von Wirkstoffen mittels Spinnverfahren sind Seidenproteine. Darunter verstehen man erfindungsgemäß im Folgenden solche Proteine, die hoch repetitive Aminosäuresequenzen enthalten und im Tier in einer flüssigen Form gespeichert werden und bei deren Sekretion durch Scherung oder Verspinnen Fasern entstehen (Craig, C. L. (1997) Evolution of arthropod silks. Annu. Rev. Entomol. 42: 231-67).

Besonders geeignete Proteine für die Formulierung von Wirkstoffen mittels Spinnverfahren sind Spinnenseidenproteine, die in ihrer ursprünglichen Form aus Spinnen isoliert werden konnten.

Ganz besonders geeignete Proteine sind Seidenproteine, die aus der "Major Ampullate"-Drüse von Spinnen isoliert werden konnten.

Bevorzugte Seidenproteine sind ADF3 und ADF4 aus der der "Major Ampullate"-Drüse von Araneus diadematus (Guerette et al., Science 272, 5258:112-5 (1996)).

Ebenso geeignete Proteine für die Formulierung von Wirkstoffen mittels Spinnverfahren sind natürliche oder synthetische Proteine, die sich von natürlichen Seidenproteinen ableiten und welche unter Verwendung gentechnologischer Arbeitsmethoden heterolog in prokaryontischen oder eukaryontischen Expressionssystemen hergestellt wurden. Nichtlimitierende Beispiele für prokaryontische Expressionsorganismen sind E-scherichia coli, Bacillus subtilis, Bacillus megaterium, Corynebacterium glutamicum u.a.. Nichtlimitierende Beispiele für eukaryontische Expressionsorganismen sind Hefen, wie Saccharomyces cerevisiae, Pichia pastoris u.a., filamentöse Pilze, wie Aspergillus niger, Aspergillus oryzae, Aspergillus nidulans, Trichoderma reesei, Acremonium chrysogenum u.a., Säugetierzellen, wie Hela-Zellen, COS-Zellen, CHO-Zellen u.a., Insektenzellen, wie Sf9-Zellen, MEL-Zellen u.a..

Weiterhin geeignet für die Formulierung von Wirkstoffen mittels Spinnverfahren sind synthetische Proteine, welche auf Wiederholungseinheiten von natürlichen Seidenproteinen basieren. Neben den synthetischen repetitiven Seidenprotein-Sequenzen können diese zusätzlich eine oder mehrere natürliche nicht-repetitve Seidenprotein-Sequenzen enthalten (Winkler und Kaplan, J Biotechnol 74:85-93 (2000)).

Für die Formulierung von Wirkstoffen mittels Spinnverfahren sind insbesondere solche synthetische Spinnenseidenproteine auch brauchbar, welche auf Wiederholungseinheiten von natürlichen Spinnenseidenproteinen basieren. Neben den synthetischen repetitiven Spinnenseidenprotein-Sequenzen können diese zusätzlich eine oder mehrere natürliche nicht-repetitve Spinnenseidenprotein-Sequenzen enthalten.

Unter den synthetischen Spinnenseidenproteinen ist bevorzugt das sog. C16-Protein zu nennen (Huemmerich et al. Biochemistry, 43(42):13604-13612 (2004)). Dieses Protein hat die in SEQ ID NO: 2 dargestellte Polypeptidsequenz.
Neben der in SEQ ID NO:2 dargestellten Polypeptidsequenz sind auch besonders funktionale Äquivalente, funktionale Derivate und Salze dieser Sequenz bevorzugt. Weiterhin sind für die Formulierung von Wirkstoffen mittels Spinnverfahren synthetische Proteine bevorzugt, welche auf Wiederholungseinheiten von natürlichen Seidenproteinen kombiniert mit Sequenzen von Insektenstrukturproteinen wie dem Resilin (Elvin et al., 2005, Nature 437: 999-1002) basieren.

Von diesen Kombinationsproteinen aus Seidenproteinen und Resilinen sind insbesondere die R16- und S16-Proteine zu nennen. Diese Proteine haben die in SEQ ID NO: 4 bzw. SEQ ID NO: 6 dargestellten Polypeptidsequenzen.

Neben der in SEQ ID NO: 4 und SEQ ID NO: 6 dargestellten Polypeptidsequenzen sind auch besonders funktionale Äquivalente, funktionale Derivate und Salze dieser Sequenzen bevorzugt.

### (iii) Abgewandelte Biopolymere

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem die Eigenschaft zur Verpackung von Effektstoffen besitzt. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne die gewünschte biologische Aktivität.

Beispiele für geeignete Aminosäuresubstitutionen sind folgender Tabelle zu entnehmen:

| | |
|---|---|
| Ursprünglicher Rest | Beispiele der Substitution |
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Erfindungsgemäß mit umfasste "funktionale Äquivalente" sind Homologe zu den hierin konkret offenbarten Proteinen/Polypeptiden. Diese besitzen wenigstens 60%, wie z.B. 70, 80 oder 85%, wie z.B. 90, 91, 92, 93, 94, 95, 96, 97, 98 oder 99%, Identität zu einer der konkret offenbarten Aminosäuresequenzen.

Unter "Identität" zwischen zwei Sequenzen wird insbesondere die Identität der Reste über die jeweils gesamte Sequenzlänge verstanden, insbesondere die Identität, die durch Vergleich mit Hilfe der Vector NTI Suite 7.1 (Vector NTI Advance 10.3.0, Invitrogen Corp.) (bzw. Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:

| | |
|---|---|
| Gap opening penalty | 10 |
| Gap extension penalty | 0,05 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| | |
|---|---|
| FAST algorithm | off |
| K-tuple size | 1 |
| Gap penalty | 3 |
| Window size | 5 |
| Number of best diagonals | 5 |

### (iv) Formulierung von Wirkstoffen

Formulierungen von Wirkstoffen können, z.B. unter Verwendung eines Biopolymers, wie eines amphiphilen selbstassemblierenden Proteins auf verschiedene Art und Weise hergestellt werden. Wirkstoffe können durch Spinnverfahren in Protein-Flächengebilde (z.B. Protein-Filme, Protein-Fasern, Protein-Vliesstoffe) verpackt oder verkapselt werden.

Die Fasern und Flächengebilde aus Protein-Wirkstoff-Kombinationen können mit allen, dem Fachmann bekannten Spinnverfahren aus Lösung oder feinteiliger Dispersion (Trockenspinnen, Nassspinnen) und Gel hergestellt werden. Besonders geeignet sind Spinnverfahren aus der Lösung oder einer feinteiligen Dispersion, darunter besonders bevorzugt sind Zentrifugenspinnen (Rotorspinnen) und Elektrospinnen (elektrostatisches Spinnen).

Bei der Verspinnung von Proteinen zu Fasern sind Faserdurchmessern von 10 nm bis 100 µm, bevorzugt mit Durchmesser von 50 nm bis 10 µm, besonders bevorzugt von 100 nm bis 2 µm, geeignet.

Beim Elektroverspinnen (elektrostatisches Spinnen) wird die zu formulierende Lösung oder feinteilige Dispersion in ein elektrisches Feld mit der Stärke zwischen 0,01 bis 10 kV/cm, besonders bevorzugt zwischen 1 und 6 kV/cm und ganz besonders bevorzugt zwischen 2 und 4 kV/cm, eingebracht. Sobald die elektrischen Kräfte die Oberflächenspannung der Formulierung übersteigen, erfolgt der Massentransport in Form eines Jets auf die gegenüberliegende Elektrode. Das Lösungsmittel verdampft im Zwischenelektrodenraum und Feststoff der Formulierung liegt dann als Fasern auf der Gegenelektrode vor. Die Spinnelektrode kann Düsen- oder Spritzen- basiert sein oder Walzengeometrie haben. Das Spinnen kann in beiden vertikalen Richtungen (von unten nach oben und von oben nach unten) und in horizontaler Richtung erfolgen.

Ein weiteres geeignetes Verfahren ist das Zentrifugenspinnen (Rotorspinnen). Bei diesem Verfahren wird die Formulierung oder feinteilige Dispersion in ein Feld mit Gravitationskräften eingebracht. Dazu wird das Faserrohmaterial in ein Behältnis gegeben und das Behältnis in Rotation versetzt, wobei das fluidisierte Faserrohmaterial durch Zentripetal- bzw. Zentrifugalkräfte aus dem Behältnis in Form von Fasern ausgetragen wird. Die Fasern können anschließend durch Gasstrom abtransportiert und zu Flächengebilden zusammengelegt werden.

Die Formulierung der Wirkstoffe kann durch Einschließen in die durch die erfindungsgemäßen Verfahren hergestellten Protein-Flächengebilde (z.B. Protein-Filme, Protein-Fasern, Protein-Vliesstoffe) erfolgen. Dieser Prozess umfasst zwei Schritte. Im ersten Schritt wird eine Spinnlösung aus Wirkstoff und Biopolymer, wie z.B. amphiphilem selbstassemblierenden Protein, durch Mischen der Komponenten in einer gemeinsamen Phase hergestellt. Dazu können der Wirkstoff und das Protein direkt durch ein Lösungsmittel oder eine Lösungsmittelmischung in Lösung gebracht werden. Alternativ können der Wirkstoff und das Protein zunächst in unterschiedlichen Lösungsmitteln gelöst und die Lösungen im Anschluss miteinander vermischt werden, so dass wiederum eine gemeinsame Phase entsteht. Bei der gemeinsamen Phase kann es sich auch um eine molekular-disperse Phase oder eine kolloidal-disperse Phase handeln.

Der Spinnlösung können zusätzlich weitere Stoffe zugegeben werden, um z.B. die Viskosität der Lösung zu erhöhen oder deren sonstige Verarbeitbarkeit zu verbessern bzw. bevorzugte strukturelle Materialeigenschaften wie z.B. Kristallinitäten oder bevorzugte Anwendungseigenschaften wie z.B. gezielte, verzögerte oder kontinuierliche Freisetzungsprofile der formulierten Wirkstoffe zu erreichen. Bevorzugte Zusatzstoffe sind dabei wasserlösliche Polymere oder insbesondere wässrige Polymerdispersionen. Geeignete Mengen der Zusatzstoffe in der Spinnlösung sind > 0,1 Gew. %, bevorzugt > 0,5 Gew. %, besonders bevorzugt > 1 %, ganz besonders bevorzugt > 5 %.

Zusätzlich können der Spinnlösung oder den daraus hergestellten Protein-Flächengebilden (z.B. Protein-Filme, Protein-Fasern, Protein-Vliesstoffe) Stoffe zugesetzt werden, welche eine Sprengung der Tabletten oder Kapseln und damit eine verbesserte Dispergierung der zu den Tabletten oder Kapseln verpressten Protein-Flächengebilde (z.B. Protein-Filme, Protein-Fasern, Protein-Vliesstoffe) sowie der darin enthaltenen Wirkstoffe ermöglicht.

Das Lösen des Wirkstoffes und des Proteins in verschiedenen Lösungsmitteln und das anschließende Mischen beider Lösungen sind insbesondere dann von Vorteil, wenn sich der Wirkstoff und das Protein nicht in einem gemeinsamen Lösungsmittel oder Lösungsmittelgemisch lösen lassen. Auf diese Art und Weise lassen sich auch kolloidal-disperse Lösungen hydrophober Wirkstoffe herstellen, indem der in einem geeigneten Lösungsmittel gelöste Wirkstoff in ein anderes Lösungsmittel verdünnt wird, in dem dieser Wirkstoff unlöslich ist.

Da Proteine in der Regel gut wasserlöslich sind, wird bevorzugt mit wässrigen Lösungen gearbeitet. Es sind aber auch Mischungen aus Wasser und wassermischbaren, organischen Lösungsmitteln bzw. die ausschließliche Verwendung organischer Lösungsmittel möglich. Bespiele für geeignete, wassermischbare Lösungsmittel sind Alkohole wie Methanol, Ethanol und Isopropanol, fluorierte Alkohole wie Hexafluorisopropanol und Trifluorethanol, Alkanone wie Aceton oder auch Sulfoxide wie z.B. Dimethylsulfoxid oder Formamide wie Dimethylformamid oder andere organische Lösungsmittel wie z.B. Tetrahydrofuran und Acetonitril oder N-Methyl-2-pyrrolidon oder Formiat. Im allgemeinen kann mit allen Lösungsmitteln und Lösungsmittelgemischen gearbeitet werden, in denen sich die Proteine lösen lassen. Beispiele für geeignete Lösungsmittel sind Wasser oder auf Wasser basierende Puffersysteme und Salzlösungen, fluorierte Alkohole wie z.B. Hexafluorisopropanol oder Trifluorethanol, ionische Flüssigkeiten wie z.B. 1-Ethyl-(3-methyl(imidezol) (EMIM)-Acetat, wässrige Lösungen chaotroper Salze wie z.B. Harnstoff, Guanidiuniumhydrochlorid und Guanidiniumthiocyanat oder organische Säuren wie z.B. Ameisensäure sowie Mischungen dieser Lösungsmittel mit anderen organischen Lösungsmitteln. Beispiele für Lösungsmittel, die sich mit den Lösungsmitteln für das Protein mischen lassen sind u.a. Wasser, Alkohole wie Methanol, Ethanol und Isopropanol, Alkanone wie Aceton, Sulfoxide wie z.B. Dimethylsulfoxid, Formamide wie Dimethylformamid, Halogenalkane wie Methylenchlorid oder auch weitere organische Lösungsmittel wie z.B. Tetrahydrofuran.

Der zweite Schritt der Formulierung der Wirkstoffe ist eine Assemblierung des Proteins, induziert z.B. durch Verdampfen des Lösungsmittels, ein elektrisches Feld, durch Scherkräfte oder Zentrifugalkräfte, zu einer gemeinsamen festen oder hoch viskosen, gelartigen Phase, die anschließend aushärtet. Dabei wird der Wirkstoff in die Assemblierungsform des Proteins eingeschlossen. Die assemblierten Proteinstrukturen können als wirkstoff-enthaltende Protein-Flächengebilde (z.B. Protein-Filme, Protein-Fasern, Protein-Vliesstoffe) hergestellt und während des Spinnvorganges auf Substraten wie z. B. Mikrofaser-Vliesen abgelegt werden. Anschließend können die assemblierten Proteinstrukturen zu Tabletten oder Kapseln verpresst werden.

Der Wirkstoff kann an die Oberfläche gebunden sein, in die Protein-Flächengebilde (z.B. Protein-Filme, Protein-Fasern, Protein-Vliesstoffe) eingeschlossen sein oder auch auf beide Arten mit den Protein-Flächengebilden assoziiert sein. Die Bindung des Wirkstoffs an die mit den erfindungsgemäßen Verfahren hergestellten Protein-Flächengebilde kann durch die Verarmung des Assemblierungsansatzes an gelöstem Wirkstoff bestimmt werden. Die Konzentration des Wirkstoffs kann durch eine quantitative Analyse seiner Eigenschaften gemessen werden. So kann die Bindung von lichtabsorbierenden Wirkstoffen z.B. durch photometrische Methoden analysiert werden. Dazu werden z.B. die Färbung der Proteinflächengebilde (z.B. Protein-Filme, Protein-Fasern, Protein-Vliesstoffe) oder die Entfärbung der protein- und wirkstoffarmen Phase des Formulierungsansatzes durch Messen der Absorption eines farbigen oder lichtabsorbierenden Wirkstoffs bestimmt. Mit Hilfe dieser Methoden kann auch bestimmt werden, wie hoch der Wirkstoffanteil in den Microbeads ist. Dazu werden die Protein-Flächengebilde (z.B. Protein-Filme, Protein-Fasern, Protein-Vliesstoffe) mit einem für den verkapselten Wirkstoff geeigneten Lösungsmittel versetzt und dabei der Wirkstoff herausgelöst. Anschließend wird der Wirkstoffgehalt im Lösungsmittel z.B. absorptionsphotometrisch bestimmt. Alternativ kann die Proteinassemblierungsstruktur auch mittels proteolytisch aktiven Enzymen abgebaut werden, wobei der enthaltene Wirkstoff freigesetzt und anschließend quantifiziert wird.

### (v) Synthetische Polymerkomponenten

Geeignete synthetische Polymere sind z. B. ausgewählt aus der Gruppe bestehend aus Homo- und Copolymerisaten von aromatischen Vinylverbindungen, Homo- und Copolymerisaten von Alkylacrylaten, Homo- und Copolymerisaten von Alkylmethacrylaten, Homo- und Copolymerisaten von α-Olefinen, Homo- und Copolymere von aliphatischen Dienen, Homo- und Copolymerisaten von Vinylhalogeniden, Homo- und Copolymerisaten von Vinylacetaten, Homo- und Copolymerisaten von Acrylnitrilen, Homo-und Copolymerisaten von Urethanen, Homo- und Copolymerisaten von Vinylamiden und Copolymeren aufgebaut aus zwei oder mehr der die vorstehend genannten Polymere bildenden Monomereinheiten.

Als Trägerpolymere kommen insbesondere Polymere auf Basis der folgenden Monomere in Betracht:
Acrylamid, Adipinsäure, Allylmethacrylat, alpha-Methylstyrol, Butadien, Butandiol, Butandioldimethacrylat, Butandioldivinylether, Butandioldimethacrylat, Butandiolmonoacrylat, Butandiolmonomethacrylat, Butandiolmonovinylether, Butylacrylat, Butylmethacrylat, Cyclohexylvinylether, Diethylenglycoldivinylether, Diethylenglycolmonovinylether, Ethylacrylat, Ethyldiglycolacrylat, Ethylen, Ethylenglycolbutylvinylether, Ethylenglycoldimethacrylat, Ethylenglycoldivinylether, Ethylhexylacrylat, Ethylhexylmethacrylat, Ethylmethacrylat, Ethylvinylether, Glycidylmethacrylat, Hexandioldivinylether, Hexandiolmonovinylether, Isobuten, Isobutylacrylat, Isobutylmethacrylat, Isopren, Isopropylacrylamid, Methylacrylat, Methylenbisacrylamid, Methylmethacrylat, Methylvinylether, n-Butylvinylether, NMethyl-N-vinylacetamid, N-Vinylcaprolactam, N-Vinylimidazol, N-Vinylpiperidon, NVinylpyrrolidon, Octadecylvinylether, Phenoxyethylacrylat, Polytetra-hydrofuran-2-divinylether, Propylen, Styrol, Terephthalsäure, tert-Butylacrylamid, tert-Butylacrylat, tert-Butylmethacrylat, Tetraethylenglycoldivinylether, Triethylenglycoldimethylacrylat, Triethylenglycoldivinylether, Triethylenglycoldivinylmethylether, Trimethylolpropantrimethacrylate, Trimethylolpropantrivinylether, Vinyl-(2-ethylhexyl)ether, Vinyl-4-tertbutyl-benzoat, Vinylacetat, Vinylchlorid, Vinyldodecylether, Vinylidenchlorid, Viny-lisobutylether, Vinylisopropylether, Vinylpropylether und Vinyl-tert-butylether.

Der Begriff "synthetische Polymere" umfasst sowohl Homo- als auch Copolymere. Als Copolymere kommen sowohl statistische als auch alternierende Systeme, Blockcopolymere oder Pfropfcopolymere in Frage. Der Begriff Copolymere umfasst Polymere, die aus zwei oder mehr verschiedenen Monomeren aufgebaut sind, oder bei denen sich der Einbau mindestens eines Monomers in die Polymerkette auf verschiedene Art und Weise realisieren lässt, wie es z.B. bei den Stereoblockcopolymeren der Fall ist.

Es können auch Abmischungen von Homo- und Copolymeren sein. Die Homo- und Copolymere können mischbar und nicht mischbar sein.

Folgende Polymere sind vorzugsweise zu nennen:
Polyvinylether wie z.B. Polybenzyloxyethylen, Polyvinylacetale, Polyvinylester wie z.B. Polyvinylacetat, Polyoxytetramethylen, Polyamide, Polycarbonate, Polyester, Polysiloxane, Polyurethane, Polyacrylamide, wie z.B. Poly(N-isopropylacrylamid), Polymethacrylamide, Polyhydroxybutyrate, Polyvinylalkohole, acetylierte Polyvinylalkohole, Polyvinylformamid, Polyvinylamine, Polycarbonsäuren (Polyacrylsäure, Polymethacrylsäure), Polyacrylamid, Polyitaconsäure, Poly(2-hydroxyethylacrylat), Poly(N-isopropylacryl-amid), Polysulfonsäure (Poly(2-acrylamido-2-methyl-1-propanesulfonsäure) oder PAMPS), Polymethacrylamid, Polyalkylenoxiden, z. B. Polyethylenoxiden; Poly-N-vinylpyrrolidon; Maleinsäuren, Poly(ethylenimin), Polystyrolsulfonsäure, Polyacrylate, wie z.B. Polypheno xyethylacrylat, Polymethylacrylat, Polyethylacrylat, Polydodecylacrylat, Poly(ibornylacrylat),Poly(n-butylacrylat), Poly(t-butylacrylat), Polycyclohexylacrylat, Poly(2-ethylhexylacrylat), Polyhydroxypropylacrylat, Polymethacrylate, wie z.B. Polymethylmethacrylat, Poly(n-amylmethacrylat), Poly(n-butylmethacrylat), Polyethylmethacrylat, Poly(hydroxypropylmethacrylat), Polycyclohexylmethacrylat, Poly(2-ethylhexylmethacrylat), Polylaurylmethacrylat, Poly(t-butylmethacrylat), Polybenzylmethacrylat, Poly(ibornylmethacrylat), Polyglycidylmethacrylat und Polystearylmethacrylat, Polystyrol, sowie Copolymere auf Basis von Styrol, z.B. mit Maleinsäureanhydrid, Styrol-Butadien-Copolymere, Methylmethacrylat-Styrol-Copolymere, N-Vinylpyrrolidon-Copolymere, Polycaprolactone, Polycaprolactame, Poly(N-vinylcaprolactam).

Insbesondere sind Poly-N-vinylpyrrolidon, Polymethylmethacrylat, Acrylat-StyrolCopolymere, Polyvinylalkohol, Polyvinylacetat, Polyamid, Polyester zu nennen. Anwendbar sind weiterhin synthetische, biologisch abbaubare Polymere.

Die Angabe "biologisch abbaubare Polymere" soll alle Polymere umfassen, die die in DIN V 54900 gegebene Definition der Bioabbaubarkeit erfüllen, insbesondere kompostierbare Polyester.

Im Allgemeinen bedeutet die biologische Abbaubarkeit, dass die Polymere, wie z.B. Polyester, in einer angemessenen und nachweisbaren Zeitspanne zerfallen. Der Abbau kann hydrolytisch und/oder oxidativ erfolgen und zum überwiegenden Teil durch die Einwirkung von Mikroorganismen, wie Bakterien, Hefen, Pilzen und Algen, bewirkt werden. Die biologische Abbaubarkeit lässt sich z.B. dadurch bestimmen, dass Polyester mit Kompost gemischt und für eine bestimmte Zeit gelagert werden. Gemäß ASTM D 5338, ASTM D 6400 und DIN V 54900 wird CO₂-freie Luft beispielsweise durch gereiften Kompost während des Kompostierens strömen gelassen und dieser einem definierten Temperaturprogramm unterworfen. Hierbei wird die biologische Abbaubarkeit über das Verhältnis der Netto-CO₂-Freisetzung der Probe (nach Abzug der C02-Freisetzung durch den Kompost ohne Probe) zur maximalen CO₂-Freisetzung der Probe (berechnet aus dem Kohlenstoffgehalt der Probe) als biologische Abbaubarkeit definiert. Biologisch abbaubare Polyester zeigen in der Regel schon nach wenigen Tagen der Kompostierung deutliche Abbauerscheinungen wie Pilzbewuchs, Riss- und Lochbildung. Beispiele für biologisch abbaubare Polymere sind biologisch abbaubare Polyester wie zum Beispiel Polylactid, Polycaprolacton, Polyalkylenadipatterepthalate, Polyhydroxyalkonoate (Polyhydroxybutyrat) und Polylactidglycosid. Besonders bevorzugt sind biologisch abbaubare Polyalkylenadipatterephthalate, vorzugsweise Polybutylenadipatterephtalate. Geeignete Polyalkylenadipatterephthalate sind z.B. in der DE 4 440 858 beschrieben (und sind kommerziell erhältlich, z.B. Ecoflex® von BASF).

### (vi) Wirkstoffe

Im folgenden werden die Begriffe Wirkstoffe und Effektstoffe synonym verwendet. Dabei handelt es sich sowohl um wasserlösliche als auch schwer-wasserlösliche Effektstoffe. Die Begriffe schwer-wasserlösliche und hydrophobe Wirk- oder Effektstoffe werden synonym verwendet. Als schwer wasserlöslichen Wirkstoffe werden im folgenden solche Verbindungen bezeichnet, deren Wasserlöslichkeit bei 20°C < 1 Gew.-%, bevorzugt < 0,5 Gew.-%, besonders bevorzugt < 0,25 Gew.-%, ganz besonders bevorzugt < 0,1 Gew.-% beträgt. Als wasserlösliche Wirkstoffe werden im folgenden solche Verbindungen bezeichnet, deren Wasserlöslichkeit bei 20°C > 1 Gew.-%, bevorzugt > 10 Gew.-%, besonders bevorzugt > 40 Gew.-%, ganz besonders bevorzugt > 70 Gew.-% beträgt.

Geeignete Effektstoffe sind Farbstoffe, insbesondere die in der folgenden Tabelle genannten:

Besonders vorteilhafte Farbstoffe sind die in der folgenden Liste genannten öllöslichen oder in Öl dispergierbaren Verbindungen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Pigment Yellow 16 | 20040 | gelb |
| Pigment Yellow 13 | 21100 | gelb |
| Pigment Yellow 83 | 21108 | gelb |
| Solvent Yellow | 21230 | gelb |
| Food Yellow | 40800 | orange |
| trans-β-Apo-8'-Carotinaldehyd (C30) | 40820 | orange |
| trans-Apo-8'-Carotinsäure(C30)-ethylester | 40825 | orange |
| Canthaxanthin | 40850 | orange |
| Solvent Dye | 45396 | orange |
| Chinophthalon | 47000 | gelb |
| Pigment Violet 23 | 51319 | violett |
| 1,2-Dioxyanthrachinon, Calcium-Aluminiumkomplex | 58000 | rot |
| 1-Hydroxy-4-N-phenyl-aminoanthrachinon | 60724 | violett |
| 1-Hydroxy-4-(4'-methylphenylamino)-anthrachinon | 60725 | violett |
| 1,4-Di(4'-methyl-phenylamino)-anthrachinon | 61565 | grün |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| 1,7-Bis-(4-hydroxy-3-methoxyphenyl)1,6-heptadien-3,5-dion | 75300 | gelb |

Weitere bevorzugte Effektstoffe sind Fettsäuren, insbesondere gesättigte Fettsäuren, die eine Alkylverzweigung tragen, besonders bevorzugt verzweigte Eicosansäuren, wie 18-Methyl-eicosansäure.

Weitere bevorzugte Effektstoffe sind Carotinoide. Unter Carotinoide sind erfindungsgemäß folgende Verbindungen sowie deren veresterte oder glykosylierte Derivate zu verstehen: β-Carotin, Lycopin, Lutein, Astaxanthin, Zeaxanthin, Cryptoxanthin, Citranaxanthin, Canthaxanthin, Bixin, β-Apo-4-carotinal, β-Apo-8-carotinal, β-Apo-8-carotinsäureester, Neurosporen, Echinenon, Adonirubin, Violaxanthin, Torulen, Torularhodin, einzeln oder als Mischung. Bevorzugt verwendete Carotinoide sind β-Carotin, Lycopin, Lutein, Astaxanthin, Zeaxanthin, Citranaxanthin und Canthaxanthin. Weitere bevorzugte Effektstoffe sind Vitamine, insbesondere Retinoide und deren Ester.

Unter Retinoide sind im Rahmen der vorliegenden Erfindung Vitamin A Alkohol (Retinol) und seine Derivate wie Vitamin A Aldehyd (Retinal), Vitamin A Säure (Retinsäure) und Vitamin A Ester (z.B. Retinylacetat, Retinylpropionat und Retinylpalmitat) gemeint. Der Begriff Retinsäure umfasst dabei sowohl all-trans Retinsäure als auch 13-cis Retinsäure. Die Begriffe Retinol und Retinal umfassen bevorzugt die all-trans Verbindungen. Als bevorzugtes Retinoid verwendet man für die erfindungsgemäßen Formulierungen all-trans-Retinol, im Folgenden als Retinol bezeichnet.

Weitere bevorzugte Effektstoffe sind Vitamine, Provitamine und Vitaminvorstufen aus den Gruppen A, B, C, E und F, insbesondere 3,4-Didehydroretinol, β-Carotin (Provitamin des Vitamin A), Palmitinsäureester der Ascorbinsäure, Tocopherole, insbesondere α-Tocopherol sowie seine Ester, z.B. das Acetat, das Nicotinat, das Phosphat und das Succinat; weiterhin Vitamin F, worunter essentielle Fettsäuren, besonders Linolsäure, Linolensäure und Arachidonsäure, verstanden werden.

Weitere bevorzugte Effektstoffe sind lipophile, öllösliche Antioxidantien aus der Gruppe Vitamin E, d.h. Tocopherol und dessen Derivate, Gallussäureester, Flavonoide und Carotinoide sowie Butylhydroxytoluol/anisol.

Ein weiterer bevorzugter Effektstoff ist Liponsäure und geeignete Derivate (Salze, Ester, Zucker, Nukleotide, Nukleoside, Peptide und Lipide).

Weitere bevorzugte Effektstoffe sind UV-Lichtschutzfilter. Darunter sind organische Substanzen zu verstehen, die in der Lage sind, ultraviolette Strahlen zu absorbieren und die aufgenommene Energie in Form längerwelliger Strahlung, z.B. Wärme, wieder abzugeben.

Als öllösliche UV-B-Filter können z.B. folgende Substanzen verwendet werden:
3-Benzylidencampher und dessen Derivate, z.B. 3-(4-Methylbenzyliden)campher; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)-benzoesäureamylester; Ester der Zimtsäure, vorzugsweise 4-Methoxyzimtsäure-2-ethylhexylester, 4 Methoxyzimtsäurepropylester, 4-Methoxyzimtsäureisoamylester, 4 Methoxyzimtsäureisopentylester, 2-Cyano-3-phenyl-zimtsäure-2-ethylhexylester (Oc-tocrylene);

Ester der Salicylsäure, vorzugsweise Salicylsäure-2-ethylhexylester, Salicylsäure-4 isopropylbenzylester, Salicylsäurehomomenthylester; Derivate des Benzophenons, vorzugsweise 2-Hydroxy-4-methoxybenzophenon, 2-Hydroxy-4-methoxy-4'-methylben-zophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin (Octyltriazone) und Dioctyl Butamido Triazon (Uvasorb® HEB):
Propan-1,3-dione, wie z.B. 1 -(4-tert. Butylphenyl)-3-(4'-methoxyphenyl)propan-1,3-dion.
Besonders bevorzugt ist die Verwendung von Estern der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene).

Des Weiteren ist die Verwendung von Derivaten des Benzophenons, insbesondere 2-Hydroxy-4-methoxybenzophenon, 2-Hydroxy-4-methoxy-4'-methylbenzophenon, 2,2'-Dihydroxy-4-methoxybenzophenon sowie der Einsatz von Propan-1,3-dionen, wie z.B. 1-(4-tert. Butylphenyl)-3-(4-'methoxyphenyl)propan-1,3-dion bevorzugt.

Als typische UV-A-Filter kommen in Frage:
Derivate des Benzoylmethans, wie beispielsweise 1-(4'-tert.Butylphenyl)-3-(4'-methoxy-phenyl)propan-1,3-dion, 4-tert.-Butyl-4'-methoxydibenzoylmethan oder 1-Phenyl-3-(4'-isopropylphenyl)-propan-1,3-dion;
Amino-hydroxy-substituierte Derivate von Benzophenonen wie z.B. N,N-Diethylamino-hydroxybenzoyl-n-hexylbenzoat.

Die UV-A und UV-B-Filter können selbstverständlich auch in Mischungen eingesetzt werden.

Geeignete UV-Filtersubstanzen sind in der folgenden Tabelle genannt.

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Tocopherole (Vitamin E) und öllösliche Ascorbinsäurederivate (Vitamin C).

Erfindungsgemäß können geeignete Derivate (Salze, Ester, Zucker, Nukleotide, Nukleoside, Peptide und Lipide) der genannten Verbindungen als Effektstoffe verwendet werden.

Weiter bevorzugt sind sogenannte Peroxydzersetzter, d.h. Verbindungen die in der Lage sind Peroxyde, besonders bevorzugt Lipidperoxide zu zersetzen. Darunter sind organische Substanzen zu verstehen, wie z.B. 5-Pyrimidinol- sowie 3-Pyridinolderivate und Probucol.

Weiterhin handelt es sich bei den genannten Peroxidzersetzern bevorzugt um die in den Patentanmeldungen WO-A-02/07698 und WO-A03/059312, auf deren Inhalt hiermit ausdrücklich Bezug genommen wird, beschriebenen Substanzen, bevorzugt die dort beschriebenen Bor-enthaltenden oder Stickstoff-enthaltenden Verbindungen, die Peroxide oder Hydroperoxide zu den entsprechenden Alkoholen ohne Bildung radikalischer Folgestufen reduzieren können. Ferner können für diesen Zweck sterisch gehinderte Amine eingesetzt werden.

Eine weitere Gruppe sind Antiirritantien, die eine entzündungshemmende Wirkung auf durch UV-Licht geschädigte Haut besitzen. Solche Stoffe sind beispielsweise Bisabolol, Phytol und Phytantriol.

Eine weitere Gruppe von Effektstoffen sind Wirkstoffe, die im Pflanzenschutz eingesetzt werden können, beispielsweise Herbizide, Insektizide und Fungizide.

Die folgende Liste von Insektiziden zeigt mögliche Pflanzenschutzwirkstoffe auf, soll aber nicht auf diese beschränkt sein:
A.1. Organo(thio)phosphate: azinphos-methyl, chlorpyrifos, chlorpyrifos-methyl, chlorfenvinphos, diazinon, disulfoton, ethion, fenitrothion, fenthion, isoxathion, malathion, methidathion, methyl-parathion, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
A.2. Carbamate: alanycarb, bendiocarb, benfuracarb, carbaryl, carbofuran, carbosulfan, fenoxycarb, furathiocarb, methiocarb, methomyl, oxamyl, pirimicarb, thiodicarb, triazamate;
A.3. Pyrethroide: allethrin, bifenthrin, cyfluthrin, cyhalothrin, cyphenothrin, cyper-5 methrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin;
A.4. Wachstumsregulatoren: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, a tetronic acid derivative of formula D1, 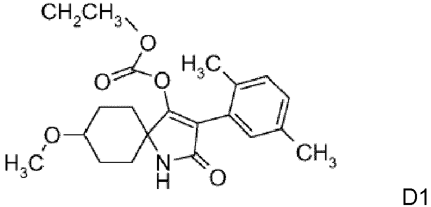
A.5. Nicotin Rezeptor Agonisten1 Antagonisten: clothianidin, dinotefuran, thiacloprid;
A.6. GABA Antagonisten: acetoprole, endosulfan, ethiprole, fipronil, vaniliprole;
A.7. Macrolid-Insektizide: abamectin, emamectin, milbemectin, lepimectin, spinosad;
A.8. MET1 I Acarizide: fenazaquin, pyridaben, tebufenpyrad, tolfenpyrad;
A.9. MET1 II and III Verbindung: acequinocyl, fluacyprim, hydramethylnon;
A.10. Entkoppler-Verbindungen: chlorfenapyr;
A.11 . Hemmer der oxidativen Phosphorylierung: cyhexatin, diafenthiuron, fenbutatin oxide, propargite;
A.12. Häutungsstörende Verbindungen: cryomazine;
A.13. Hemmer der Mixed-Function-Oxidase: piperonyl butoxide;
A.14. Natriumkanalblocker: indoxacarb, metaflumizone;
A.15. Verschiedene: benclothiaz, bifenazate, flonicamid, pyridalyl, pymetrozine, sulfur, thiocyclam und aminoisothiazole Verbindungen der Formel D2, 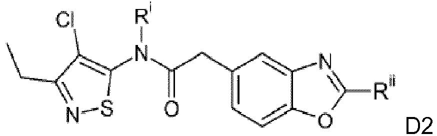 wobei Rⁱ für -CH₂OCH₂CH₃ oder H und R" für CF₂CF₂CF₃ oder CH₂CH(CH₃)₃ steht, anthranilamide Verbindungen der Formel D3: 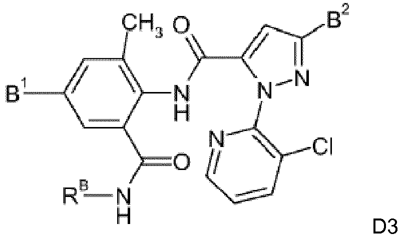 wobei B¹ für Wasserstoff oder Chlor, B² für Brom oder CF₃, und R^{B} für CH₃ oder CH(CH₃)₂ steht, und malononitrile Verbindungen wie in JP 2002 284608, WO 02/189579, WO 02/190320, WO 02/190321, WO 04/106677, WO 04/120399, oder der JP 2004 99597 beschrieben, N-R'-2,2-Dihalo-I-R"cyclo-propancarboxamid-2-(2,6-dichlor-*α*,*α*,*α*,*α*-trifluor-p-tolyl)hydrazon oder N-R'-2,2-Di(R"')propionamid-2-(2,6-dichlor-*α*,*α*,*α*,*α*-trifluor-p-tolyl)-hydrazon, worin R' für Methyl oder Ethyl steht, Halo für Chlor oder Brom steht, R" für Wasserstoff oder Methyl und R'" für Methyl oder Ethyl stehen;

Die folgende Liste von Fungiziden zeigt mögliche Wirkstoffe auf, soll aber nicht auf diese beschränkt sein:
1. Strobilurine
   Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-me-thyl-pyridin-2-ylmethoxyimino)-ethyl]-benzyl)-carbaminsäuremethylester, 2-(ortho-((2,5-Dimethylphenyl-oxymethylen)phenyl)-3-methoxy-acrylsäuremethylester;
2. Carbonsäureamide
   - Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluor-2-methyl-triazol-5-carbonsäure-(4'-trifluor-methyl-biphenyl-2-yl)-amid, 4-Difluor-2-methyl-triazol-5-carbonsäure-(4'-chlor-3'-fluor-biphenyl-2-yl)-amid, 3-Difluor-1-methyl-pyrazol-4-carbonsäure-(3',4'-dichlor-4-fluor-biphenyl-2-yl)-amid;
   - Carbonsäuremorpholide: Dimethomorph, Flumorph;
   - Benzoesäureamide: Flumetover, Fluopicolide (Picobenzamid), Zoxamide;
   - Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;
3. Azole
   - Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol,Hexaconazol, Imibenconazole, Ipconazole, Metconazole, Myclobutanil, Penconazole,Propiconazole, Prothioconazole, Simeconazole, Tebuconazole, Tetraconazole,Triadimenol, Triadimefon, Triticonazole;
   - Imidazole: Cyazofamid, Imazalil, Pefurazoate, Prochloraz, Triflumizole;
   - Benzimidazole: Benomyl, Carbendazim, Fuberidazole, Thiabendazole;
   - Sonstige: Ethaboxam, Etridiazole, Hymexazole;
4. Stickstoffhaltige Heterocyclylverbindungen:
   - Pyridine: Fluazinam, Pyrifenox, 3-[5-(4-Chlor-phenyl)-2,3-dimethyl-isoxazolidin-3-yl]-pyridin;
   - Pyrimidine: Bupirimate, Cyprodinil, Ferimzone, Fenarimol, Mepanipyrim, Nuarimol, Pyrimethanil;
   - Piperazine: Triforine;
   - Pyrrole: Fludioxonil, Fenpiclonil;
   - Morpholine: Aldimorph, Dodemorph, Fenpropimorph, Tridemorph;
   - Dicarboximide: Iprodione, Procymidone, Vinclozolin;
   - sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Quinoxyfen, Tricyclazole, 5-Chlor-7-(4-methyl-piperidin-1-yl)-6-(2,4,6-trifluor-phenyl)-[1,2,4]triazolo[1,5-alpyrimidin, 2-Butoxy-6-iodo-3-propyl-chromen-4-on, 3-(3-Brom-6-fluor-2-methyl-indol-1-sulfonyl)-[1,2,4]triazol-1-sulfonsäuredimethylamid;
5. Carbamate und Dithiocarbamate
   - Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluor-25 phenyl)ester;
6. Sonstige Fungizide
   - Organometallverbindungen: Fentin-Salze;
   - Schwefelhaltige Heterocyclylverbindungen: Isoprothiolane, Dithianon;
   - Organophosphorverbindungen: Edifenphos, Fosetyl, Fosetyl-aluminium, Iprobenfos, Pyrazophos, Tolclofos-methyl, Phosphorige Säure und ihre Salze;
   - Organochlorverbindungen: Thiophanate Methyl, Chlorothalonil, Dichlofluanid, Tolylfluanid, Flusulfamide, Phthalide, Hexachlorbenzene, Pencycuron, Quintozene;
   - Nitrophenylderivate: Binapacryl, Dinocap, Dinobuton;
   - Sonstige: Spiroxamine, Cyflufenamid, Cymoxanil, Metrafenone.

Die folgende Liste von Herbiziden zeigt mögliche Wirkstoffe auf, soll aber nicht auf diese beschränkt sein:
Verbindungen, die die Biosynthese von Lipiden inhibieren, z.B. Chlorazifop, Clodinafop, Clofop, Cyhalofop, Ciclofop, Fenoxaprop, Fenoxaprop-p, Fenthiaprop, Fluazifop, Fluazifop-P, Haloxyfop, Haloxyfop-P, Isoxapyrifop, Metamifop, Propaquizafop, Quizalofop, Quizalofop-P, Trifop, bzw. deren Esters, Butroxydim, Cycloxydim, Profoxydim, Sethoxydim, Tepraloxydim, Tralkoxydim, Butylate, Cycloat, Diallat, Dimepiperat, EPTC, Esprocarb, Ethiolate, Isopolinate, Methiobencarb, Molinate, Orbencarb, Pebulate, Prosulfocarb, Sulfallat, Thiobencarb, Thiocarbazil, Triallat, Vernolat, Benfuresat, Ethofu-5 mesat und Bensulid;
ALS-Inhibitoren wie Amidosulfuron, Azimsulfuron, Bensulfuron, Chlorimuron, Chlorsulfuron, Cinosulfuron, Cyclosulfamuron, Ethametsulfuron, Ethoxysulfuron, Flazasulfuron, Flupyrsulfuron, Foramsulfuron, Halosulfuron, Imazosulfuron, lodosulfuron, Mesosulfuron, Metsulfuron, Nicosulfuron, Oxasulfuron, Primisulfuron, Prosulfuron, Pyrazosulfuron, Rimsulfuron, Sulfometuron, Sulfosulfuron, Thifensulfuron, Triasulfuron, Tribenuron, Trifloxysulfuron, Triflusulfuron, Tritosulfuron, Imazamethabenz, Imazamox, Imazapic, Imazapyr, Imazaquin, Imazethapyr, Cloransulam, Diclosulam, Florasulam, Flumetsulam, Metosulam, Penoxsulam, Bispyribac, Pyriminobac, Propoxycarbazone, Flucarbazone, Pyribenzoxim, Pyriftalid und Pyrithiobac; sofern der pH Wert 8 < ist;
Verbindungen, die die Photosynthese inhibieren wie Atraton, Atrazine, Ametryne, Aziprotryne, Cyanazine, Cyanatryn, Chlorazine, Cyprazine, Desmetryne, Dimethametryne, Dipropetryn, Eglinazine, Ipazine, Mesoprazine, Methometon, Methoprotryne, Procyazine, Proglinazine, Prometon, Prometryne, Propazine, Sebuthylazine, Secbumeton, Simazine, Simeton, Simetryne, Terbumeton, Terbuthylazine und Terbutryne;
Protoporphyrinogen-IX Oxidase-Inhibitoren wie Acifluorfen, Bifenox, Chlomethoxyfen, Chlornitrofen, Ethoxyfen, Fluorodifen, Fluoroglycofen, Fluoronitrofen, Fomesafen, Furyloxyfen, Halosafen, Lactofen, Nitrofen, Nitrofluorfen, Oxyfluorfen, Fluazolate, Pyraflufen, Cinidon-ethyl, Flumiclorac, Flumioxazin, Flumipropyn, Fluthiacet, Thidiazimin, Oxadiazon, Oxadiargyl, Azafenidin, Carfentrazone, Sulfentrazone, Pentoxazone, Benzfendizone, Butafenacil, Pyraclonil, Profluazol, Flufenpyr, Flupropacil, Nipyraclofen und Etnipromid;
Herbizide wie Metflurazon, Norflurazon, Flufenican, Diflufenican, Picolinafen, Beflubutamid, Fluridone, Flurochloridone, Flurtamone, Mesotrione, Sulcotrione, Isoxachlortole, Isoxaflutole, Benzofenap, Pyrazolynate, Pyrazoxyfen, Benzobicyclon, amitrole, clomazone, Aclonifen, 4-(3-trifluormethylphenoxy)- 2-(4-trifluoromethylphenyl)pyrimidin, und 3-heterocyclyl-substituierte Benzoylderivate der Formel (vgl. WO-A-96/26202, WO-A-97/41116, WO-A-97/41117 und WO-A-97/41118) 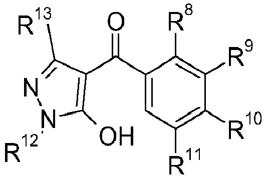 worin die Substituenten R⁸ bis R¹³ folgende Bedeutung haben:
   R⁸, R¹⁰ Wasserstoff, Halogen, C₁-C₅-Alkyl, C₁-C₅-Haloalkyl, C₁-C₅-Alkoxy, Haloalkoxy, C₁-C₅-Alkylthio, C₁-C₅-Alkylsulfinyl oder C₁-C₅-Alkylsulfonyl;
   R⁹ bedeutet ein heterocyclisches Radikal aus der Gruppe bestehend aus Thiazol-2-yl, thiazol-4-yl, Thiazol-5-yl, Isoxazol-3-yl, Isoxazol-4-yl, Isoxazol-5-yl, 4,5-dihydroisoxazol-3-yl, 4,5-dihydroisoxazol-4-yl und 4,5-dihydroisoxazol-5-yl, worin die genannten Radikale einen oder mehrere Substituenten tragen können z.B. mono-, di-,5 tri- oder tetrasubstituiert sein können durch Halogen, C₁-C₄-Alkyl, C₁-C₄-Alkoxy, C₁-C₄-Haloalkyl, C₁-C₄-Haloalkoxy oder C₁-C₄-Alkylthio;
   R¹¹ = Wasserstoff, Halogen oder C₁-C₅-Alkyl;
   R¹² = C₁-C₆-Alkyl;
   R¹³ = Wasserstoff oder C₁-C₆-Alkyl, sofern der pH Wert < 8 ist;
   Mitose-Inhibitoren wie Benfluralin, Butralin, Dinitramine, Ethalfluralin, Fluchloralin, i-Sopropalin, Methalpropalin, Nitralin, Oryzalin, Pendimethalin, Prodiamine, Profluralin, Trifluralin, Amiprofos-methyl, Butamifos, Dithiopyr, Thiazopyr, Propyzamide, Chlorthal, Carbetamide, Chlorpropham and Propham;
VLCFA-Inhibitoren wie Acetochlor, Alachlor, Butachlor, Butenachlor, Delachlor, Diethatyl, Dimethachlor, Dimethenamid, Dimethenamid-P, Metazachlor, Metolachlor, SMetolachlor, Pretilachlor, Propisochlor, Prynachlor, Terbuchlor, Thenylchlor, Xylachlor, CDEA, Epronaz, Diphenamid, Napropamide, Naproanilide, Pethoxamid, Flufenacet, Mefenacet, Fentrazamide, Anilofos, Piperophos, Cafenstrole, Indanofan und Tridiphan; Inhibitoren für die Biosynthese von Cellulose wie Dichlobenil, Chlorthiamid, Isoxaben und Flupoxam;
Herbizide wie Dinofenat, Dinoprop, Dinosam, Dinoseb, Dinoterb, DNOC, Etinofen und Medinoterb;
außerdem: Benzoylprop, Flamprop, Flamprop-M, Bromobutide, Chlorflurenol, Cinmethylin, Methyldymron, Etobenzanid, Pyributicarb, Oxaziclomefone, Triaziflam und Methyl bromide.

Im Pflanzenschutz verwendete Wirkstoffe können auch zur Bekämpfung von Schädlingen (z.B. Schaben, Ameisen, Termiten u.a.) im urbanen Raum (z.B. Wohnsiedlungen, Haus- und Gartenbereich, Gaststätten, Parkanlagen, Hotelanlagen, Industrieflächen u.a.) eingesetzt werden und sind speziell für diese Anwendungen eine weitere Gruppe von geeigneten Effektstoffen.

Auch Wirkstoffe zur Bekämpfung von Schädlingen aus dem Bereich der Wirbeltiere (z.B. Ratten, Mäuse u.a.) können mit den erfindungsgemäßen Verfahren formuliert werden und die resultierenden Wirkstoffzubereitungen zur entsprechenden Schädlingsbekämpfung im landwirtschaftlichen und urbanen Raum angewendet werden.

Des weiteren geeignet sind Wirkstoffe für die pharmazeutische Anwendung, insbesondere solche für die orale Verabreichung. Das erfindungsgemäße Verfahren ist prinzipiell unabhängig von der medizinischen Indikation auf eine beliebige Vielzahl von Wirkstoffen anwendbar.

Insbesondere sind wasserlösliche Wirkstoffe für die pharmazeutische Anwendung zu nennen, insbesondere solche für die orale Verabreichung. Dies betrifft sowohl verschreibungspflichtige als auch nicht verschreibungspflichtige Wirkstoffe. Die Erfindung ist prinzipiell unabhängig von der medizinischen Indikation auf eine Vielzahl von therapeutischen, prophylaktischen oder diagnostischen Wirkstoffen anwendbar. Nichtlimitierende Beispiele anwendbarer Wirkstoffklassen umfassen antiinflammatorische Mittel, vasoaktive Mittel, infektionshemmende Mittel, anästhetisierende Mittel, wachstumsfördernde Mittel. Grundsätzliche anwendbare Verbindungsklassen sind Proteine, Peptide, Nukleinsäuren, Mono-, Di-, Oligo- und Polysaccharide, Proteoglycane, Lipide, niedermolekulare synthetische oder natürliche organische Wirkstoffe. oder anorganische Verbindungen oder Elemente, wie z.B. Silber.

Nicht limitierende Beispiele für geeignete schwer wasserlösliche pharmazeutische Wirkstoffe sind in der folgenden Tabelle genannt.

| **Wirkstoff** | **Summenformel** | **Löslichkeit in Wasser [g/L]** |
|---|---|---|
| Felodipine | C₁₈H₁₉Cl₂NO₄ | 4,53 E-03 (22 °C) |
| Indomethacin | C₁₈H₁₆ClNO₄ | 1,4 E-02 (25 °C) |
| Piroxicam | C₁₅H₁₃N₃O₄S | 2,3 E-02 (RT) |
| Carbamazipine | C₁₅H₁₂N₂O | 9,451 E-01 (RT) |
| 17-β-Estradiol | C₁₈H₂₄O₂ | 1,836 E-05 (25 °C) |
| Clotrimazol | C₂₂H₁₇ClN₂ | < 1,0 E-02 (25 °C) |
| Ketoconazole | C₂₆H₂₈Cl₂N₄O₄ | 8,0 E-02 (37 °C) |
| Cinnarizine | C₂₆H₂₈N₂ | 7,5 E-01 |
| Griseofulvin | C₁₇H₁₇ClO₆ | 3,685 E-05 (25 °C) |
| Ibuprofen | C₁₃H₁₈O₂ | 2,1 E-02 (25 °C) |

Beispiele für wasserlösliche pharmazeutische Wirkstoffe sind insbesondere husten- und schleimlösende Wirkstoffe, wie z. B. Guaiacol Glykol Ether (auch Guaifenesin genannt) und dessen Derivate.

Weitere bevorzugte pharmazeutische Wirkstoffe sind Antikörper und andere in der Pharmazie verwendete Proteine, z. B. Enzyme oder Peptide, oder Nukleinsäuren.

### (vii) Wirkstoff-Freisetzung aus den Formulierungen

Die Freisetzung der Wirkstoffe aus den mit den erfindungsgemäßen Verfahren hergestellten Formulierungen kann durch Desorption in geeignete Lösungsmittel, durch den Abbau der erfindungsgemäßen Biopolymer-Flächengebilde (z.B. Protein-Filme, Protein-Fasern, Protein-Vliesstoffe) durch Proteasen oder durch Auflösen der erfindungsgemäßen Biopolymer-Flächengebilde (z.B. Protein-Filme, Protein-Fasern, Protein-Vliesstoffe) durch geeignete Lösungsmittel erfolgen. Geeignete Lösungsmittel für die Desorption sind alle Lösungsmittel oder Lösungsmittelgemische, in denen sich der Wirkstoff lösen lässt. Geeignete Proteasen können als technische Proteasen einer Suspension von den erfindungsgemäßen Biopolymer-Flächengebilden (z.B. Protein-Filme, Protein-Fasern, Protein-Vliesstoffe) gezielt zugesetzt werden oder am gewünschten Wirkort der Effektormoleküle natürlicherweise vorkommen, wie z.B. Proteasen des Verdauungstraktes, z.B. Magen- oder Darmproteasen oder von Mikroorganismen freigesetzte Proteasen. Lösungsmittel, die die erfindungsgemäßen Biopolymer-Flächengebilde auflösen können, sind z.B. fluorierte Alkohole wie z.B. Hexafluorisopropanol oder Trifluorethanol, ionische Flüssigkeiten wie z.B. EMIM Acetat, wässrige Lösungen chaotroper Salze wie z.B. Harnstoff, Guanidiuniumhydrochlorid und Guanidiniumthiocyanat oder organische Säuren wie z.B. Ameisensäure sowie Mischungen dieser Lösungsmittel mit anderen organischen Lösungsmitteln. Die Geschwindigkeit und die Kinetik der Freisetzung der Effektormoleküle können z.B. durch die Beladungsdichte mit Wirkstoffen und die Größe der erfindungsgemäßen Biopolymer-Flächengebilde bzw. ihrem Verhältnis von Volumen zur Oberfläche gesteuert werden.

Ein weiterer Gegenstand der Erfindung ist die Verwendung der unter Benutzung der beschriebenen amphiphilen selbstassemblierenden Proteine hergestellten proteinhaltigen Flächengebilde (z.B. Protein-Filme, Protein-Fasern, Protein-Vliesstoffe) zur Speicherung, zum Transport oder zur Freisetzung von Wirkstoffen in pharmazeutischen Produkten, kosmetischen Produkten, Pflanzenschutzprodukten, Nahrungs- und Futtermitteln. Dabei dienen die erfindungsgemäßen Flächengebilde weiterhin dem Schutz der verpackten Wirkstoffe vor Umwelteinflüssen, wie z.B. oxidativen Prozessen oder UV-Strahlung, oder vor Zerstörung durch Reaktion mit anderen Bestandteilen der Produkte oder vor Abbau durch bestimmte Proteasen. Der Wirkstoff kann durch Desorption, proteolytischen Abbau, gezielte Freisetzung oder langsame Freisetzung oder Kombination dieser Mechanismen aus den proteinhaltigen Flächengebilden freigesetzt werden.

Bevorzugt sind die erfindungsgemäßen proteinhaltigen Flächengebilde (z.B. Protein-Filme, Protein-Fasern, Protein-Vliesstoffe) und damit formulierte Wirkstoffe in pharmazeutischen Produkten für eine per orale Aufnahme. Dabei kann die Stabilität der Wirkstoffe bei Magenpassage erhöht werden, da unter den dort vorherrschenden Bedingungen kein proteolytischer Abbau der erfindungsgemäßen Flächengebilde erfolgt. Die Freisetzung der Wirkstoffe aus den per oral aufgenommenen Wirkstoffenthaltenden Protein-Flächengebilden, welche auch zu Tabletten oder Kapseln verpresst sein können, erfolgt dann im Darm. Eine Freisetzung der Wirkstoffe unter Magenbedingungen kann aber durch Desorption oder Diffusion erfolgen.

In pharmazeutischen Produkten, Nahrungs- und Futtermitteln bzw. Pflanzenschutzprodukten kann eine Formulierung von Wirkstoffen mit den erfindungsgemäßen Verfahren unter Verwendung der beschriebenen Biopolymere, insbesondere amphiphilen, selbstassemblierenden Proteine weiterhin zu einer erhöhten Bioverfügbarkeit der Wirkstoffe führen. Die Verpackung von pharmazeutischen Wirkstoffen in Protein-Flächengebilde kann weiterhin zur verbesserten Aufnahme über die Darmschleimhaut führen. Pflanzenschutzprodukte können durch Verkapselung bzw. Einbettung in ProteinFlächengebilde vor Auswaschprozessen geschützt werden. Bestimmte Wirkstoffpartikelgrößen, welche besser aufgenommen oder resorbiert werden bzw. besser bioverfügbar sind, können durch Verpackung in Protein-Flächengebilde eingestellt werden.

Durch Variation der Aminosäuresequenz der beschriebenen amphiphilen selbstassemblierenden Proteine bzw. Fusionierung mit zusätzlichen Protein- oder Peptidsequenzen ist es möglich, Strukturen zu generieren, welche bestimmte Oberflächen, z.B. Haut, Haar, Blätter, Wurzeln oder Darm- oder Blutgefäßoberflächen, spezifisch erkennen bzw. von diesen Oberflächen oder den enthaltenen Rezeptoren erkannt und gebunden werden.

Dadurch ist es möglich, die mit den beschriebenen amphiphilen selbstassemblierenden Proteinen formulierten Wirkstoffe effektiver an den gewünschten Wirkort zu bringen bzw. die Wirkstoffaufnahme zu verbessern. Die Bioverfügbarkeit von pharmazeutischen Wirkstoffen bzw. Wirkstoffen in Nahrungs- und Futtermitteln kann erhöht werden, wenn diese in Protein-Flächengebilde (z.B. Protein-Filme, Protein-Fasern, Protein-Vliesstoffe) verpackt werden, welche zusätzlich mit Proteinen fusioniert bzw. assoziert vorliegen, die an bestimmte Oberflächenmarker (z.B. Rezeptoren) von Zellen des Magen- und Darmtraktes (z.B. Mucosazellen) binden. Solche Proteine sind z.B. das MapA-Protein oder das Kollagen-bindende Protein CnBP aus Lactobacillus reuteri (Miyoshi et al., 2006, Biosci. Biotechnol. Biochem. 70:1622-1628) oder funktional vergleichbare Proteine aus anderen Mikroorganismen, vor allem der natürlichen Magen-Darmflora. Die beschriebenen Bindeproteine vermitteln ein Anhaften der Mikroorganismen an Zelloberflächen. Durch Kopplung bzw. Fusionierung der Bindeproteine an die beschriebenen amphiphilen selbstassemblierenden Proteine würden daraus hervorgehende Wirkstoff-beinhaltende Protein-Flächengebilde gezielter an entsprechende Aufnahmeorte gelenkt werden bzw. an diesen Orten länger verweilen, was eine verlängerte und verbesserte Wirkstoff-Freisetzung und -aufnahme zur Folge hat.

Weiterhin ist es durch Variation der Aminosäuresequenz der für die Wirkstoffformulierung beschriebenen amphiphilen selbstassemblierenden Proteine bzw. Fusionierung mit zusätzlichen Protein- oder Peptidsequenzen möglich, Wirkstoffe gezielt an gewünschte Wirkorte zu lenken, um damit z.B. eine höhere Spezifität, geringeren Wirkstoffverbrauch oder Wirkstoffdosis, eine schnellere oder stärkere Wirkung zu erzielen. Der Spinnlösung können zusätzlich weitere Stoffe zugegeben werden, um z.B. Kristallisation des Wirkstoffes in den Flächengebilden später zu beeinflussen (z.B. zu hemmen) oder bevorzugte Anwendungseigenschaften, wie veränderte Bioverfügbarkeit, zu erreichen. Bevorzugte Zusatzstoffe sind dabei z.B. ionische (kationische oder anionisch) und nichtionische Tenside. Geeignete Mengen der Zusatzstoffe in der Spinnlösung sind 0,01 Gew. % bis 5 Gew.-% .

Zusätzlich können der Spinnlösung oder den daraus hergestellten Flächengebilden Stoffe zugesetzt werden, welche eine Sprengung der Tabletten oder Kapseln und damit eine verbesserte Dispergierung der zu den Tabletten oder Kapseln verpressten Biopolymer-Flächengebilde ermöglicht.

### (viii) Faserflächengebilde (Vliese) zur Wundbehandlung und Körperpflege

Die erfindungsgemäßen Vliese können mit an sich bekannten Wundbehandlungsprodukten bzw. Körperpflegemittel kombiniert, das heißt in diese eingearbeitet oder auf diese aufgebracht werden. Konventionelle Wundauflagen, wie z. B. Mull oder Vliesstoff- oder Saugkompressen sind meist Gewebe oder Nonwoven aus Baumwolle, Viskose oder synthetischen Fasern, wie Polyamid, Polyethylen oder Polypropylen. Diese können mit hydrophoben Fettsalben imprägniert sein und zeigen eine hohe Saugfähigkeit, was das Ableiten von überschüssigem Wundexsudat, Gewebetrümmern und Bakterien begünstigt.

Allerdings ist ein häufiger Verbandwechsel notwendig und zuweilen ist ein Austrocknen der Wunde zu beobachten. Dies kann zum Verkleben der Wunde mit eingetrocknetem Wundsekret oder zum Einwachsen von jungem Epithelgewebe in die Kompresse führen. Der Verbandwechsel führt in diesem Fall zu erneuten Läsionen, was den Heilungsprozess deutlich verzögern.

Moderne Wundauflagen sollten daher für eine ideal feuchte Wundumgebung sorgen. Die eingesetzten Materialien sollten in der Lage sein, unter Gelbildung große Mengen an Feuchtigkeit aufzunehmen, wie dies z.B. bei Polyacrylaten und Alginaten Hydrokolloidprodukte auf Basis von Carboxymethyl-zellulose der Fall ist oder. Aufgrund ihrer hohen Saugkapazität kommen diese Produkte in erster Linie bei mäßig bis stark nässenden Wunden zum Einsatz. Beim Austrocknen und im Fall nekrotischer Wunden können diese Auflagen verkleben und es besteht aufgrund der großen Schrumpfung die Gefahr, dass die Wunde durch Abreißen des darunter liegenden Gewebes erneut traumatisiert wird.

Es ist ein umfangreiches Sortiment an Wundmaterialien und Konzepten zur Steuerung der Wundheilung beschrieben, die allerdings sehr spezifisch auf besondere Einsatzgebiete und weitgehend auf eine klinische Anwendung abgestimmt sind. In der Regel werden so genannte Sandwichverbände mit dem gewünschten Eigenschaftsprofil bereitgestellt; so besteht die erste Lage meist aus einer nicht verklebenden Schicht (z.B. Polyurethan basierte Schäume oder Fettgaze) und einer zweiten Lage mit einer hohen Aufnahmekapazität für Wundsekret, wie z.B. Zellulosekompressen.

Die erfindungsgemäßen Vliese stellen nun ein preiswertes, leicht drapierbares Produkt dar, das als heilungsfördernde textile Trennschicht zu der Wunde hin eingesetzt werden kann, welches aufgrund ihrer Porosität die Diffusion von Sauerstoff und Wundsekret zulässt, jedoch die Wunde elastisch verschließt und während der Heilung resorbiert wird.

Die erfindungsgemäßen Materialen können auch bei einfacheren Wundversorgungen eingesetzt werden und den Verzicht auf den Einsatz von mehrlagigen, kostenintensiven Verbänden gestatten.

Besondere Vorteile erfindungsgemäßer Faserflächengebilde, wie Biokompatibilität, Dehnbarkeit, Nicht-Toxizität, biologischen (insbesondere proteolytische) Abbaubarkeit, gute Regulierung von Feuchtigkeit, machen diese zu geeigneten Kandidaten für die Herstellung von Produkten zur Behandlung chronischer oder nichtchronischer Wunden und zur Körperpflege.

Erfindungsgemäß hergestellte wirkstofffreie oder wirkstoffhaltige Faserflächengebilde eignen sich besonders zur Herstellung von Wundversorgungsprodukten und Hygieneartikeln. Dort können sie als solches oder aufgebracht auf einem geeigneten, an sich bekannten textilen Geweben oder polymeren Trägermaterialen eingesetzt werden.

Hierzu können unterschiedliche Materialien in an sich bekannter Weise zusammengeführt und zu mehrschichtigen Produkten weiterverarbeitet werden. Je nach Verwendungszweck können Materialien, wie PE-, PET-, bzw. PU-Folien und AluminiumVerbundfolie, Nonwoven, Substrate, Silikonpapiere, Laminate etc. mit dem erfindungsgemäßen Faserflächengebilde zusammengeführt werden.

Im Falle der Herstellung der die erfindungsgemäßen Biopolymer-Flächengebilde enthaltenden Medikalprodukte (z.B. Wundauflagen oder Pflastern) oder Hygieneprodukten (Wischtücher, Windeln, Binden etc.) bzw. der Verwendung der erfindungsgemäßen Biopolymer-Vliesstoffe in entsprechenden Anwendungen können als Trägersubstrat oder als Trägerstoff für das Flächengebilde das zu beschichtende Medikal- oder Hygieneprodukt selbst oder Teile bzw. einzelne Schichten davon verwendet werden.

Die Erfindung wird nun unter Bezugnahme auf folgende nichtlimitierende Beispiele näher erläutert.

### EXPERIMENTELLER TEIL

### Allgemeiner Teil:

### a) Elektrospinnverfahren

Die zur Durchführung des erfindungsgemäßen Verfahrens geeignete Vorrichtung zum Elektrospinnen umfasst eine an deren Spitze mit einer mit einem Pol einer Spannungsquelle verbundenen Kapillardüse versehene Spritze zur Aufnahme der erfindungsgemäßen Formulierung. Gegenüber dem Ausgang der Kapillardüse ist in einem definierten Abstand eine mit dem anderen Pol der Spannungsquelle verbundene quadratische Gegenelektrode angeordnet, die als Kollektor für die gebildeten Fasern fungiert.

Eine weitere mögliche Vorrichtung zur Durchführung des erfindungsgemäßen Verfahrens umfasst eine Walze, die sich in einem Behälter mit Spinnlösung dreht. Die Walze kann glatt sein oder eine physikalische Strukturierung, z.B. Nadeln oder Riefen aufweisen. Die Spinnlösung gerät bei jeder Drehung der Walze in das starke elektrische Feld und mehrere Materialströme werden gebildet. Die Gegenelektrode befindet sich über der Spinnelektrode. Die Fasern werden auf einen Trägervlies, z.B. Polypropylen abgeschieden.

Beispielsweise kann eine Nanospider-Apparatur der Firma Elmarco verwendet werden Die Spannung beträgt dabei etwa 82 kV bei einem Elektrodenabstand von 18cm. Die Temperatur beträgt etwa 23 °C und die relative Luftfeuchtigkeit 35 %. Es wird eine gezackte Elektrode zum Verspinnen verwendet. Um ein möglichst dickes Protein-Flächengebilde (z.B. Protein-Filme, Protein-Fasern, Protein-Vliesstoffe) zu erreichen wird das Trägervlies im Stillstand belassen. Alternativ kann das Trägervlies aber auch unter Vorschub bewegt werden, um definiert dünnere Protein-Flächengebilde-Schichten zu erzielen. Die aus dem Ansatz gewonnenen Protein-Flächengebilde (z.B. Protein-Filme, Protein-Fasern, Protein-Vliesstoffe) werden anschließend bei 40°C und unter Vakuum über Nacht getrocknet. Die Schichtdicke der Protein-Flächengebilde wird mit dem Schichtdickenmessgerät Millitron (Firma: Mahr Feinprüf, Deutschland) bestimmt.

### b) Wirkstofffreisetzungsversuche

Die Freisetzung von Wirkstoffen aus den Protein-Flächengebilden wurde in zwei verschiedenen Ansätzen überprüft.

Per oral aufzunehmende Wirkstoff-Formulierungen, z.B. Guaiacol Glyceryl Ether und Clotrimazol (gepresst zu Tabletten) wurden in künstlichem Magensaft (0,1 g NaCl; 0,16 g Pepsin; 0,35 ml HCl auf 50 ml auffüllen, pH 1-2) und künstlichem Darmsaft (3,4 g KH₂PO₄ in 12,5 ml Wasser lösen + 3,85 ml 0,2N NaOH auf 25 ml auffüllen + 0,5 g Pankreatin auf 50 ml auffüllen, pH 6,8) analysiert, um die Wirkstoff-Freisetzung unter proteolytisch aktiven Bedingungen im Verdauungstrakt zu simulieren. Kontrollansätze (ohne Proteasen) erfolgten in 5 mM Kaliumphosphatpuffer (pH 8,0), wobei unter diesen Bedingungen nur eine geringe Wirkstoff-Freisetzung beobachtet werden sollte. Pro Tablette wurden 20 ml des jeweiligen Verdauungssaftes oder Puffers zugegeben und die Ansätze bei 37 °C und 80 upm leicht schüttelnd inkubiert. Zu verschiedenen Zeitpunkten werden je 500 µl Probe für eine Wirkstoff-Quantifizierung mittels HPLC oder Photometer entnommen. Um bei schlecht wasserlöslichen Wirkstoffen, z.B. Clotrimazol, auch nach der Freisetzung entstandene Wirkstoff-Aggregate zu erfassen, wurde die absorptionsphotometrische Quantifizierung nach Extraktion mit THF (3 ml Überstand + 3 ml THF + Spatelspitze NaCl, kräftiges vortexen, 1 min 15.000 x g, Oberphase vermessen, ggf. verdünnen) durchgeführt.

Bei anderen Wirkstoffen (nicht per oral aufgenommene pharmazeutische oder andere beispielsweise kosmetische oder Pflanzenschutz-Wirkstoffe), z.B. Uvinul A+ und Metazachlor, erfolgte die Freisetzungsanalyse durch Versetzen definierter Mengen an Protein-Wirkstoff-Flächengebilden mit unspezifischer Proteinase K-Lösung. Die Protein-Wirkstoff-Flächengebilde wurden in 0,25-0,5 % [w/v] Proteinase K (Roche, Germany; gelöst in 5 mM Kaliumphosphatpuffer) bei 120-150 upm schüttelnd inkubiert. Zu verschiedenen Zeitpunkten wurden die noch intakten Protein-Wirkstoff-Flächengebilde durch Zentrifugation abgetrennt, die Überstände mit einem 4-5-fachen Überschuss an THF versetzt und der Wirkstoffgehalt anschließend absorptionsphotometrisch bestimmt. Bei allen Ansätzen wurden die freigesetzten Wirkstoff-Mengen nach Abgleich mit einer Wirkstoff-spezifischen Eichreihe ermittelt.

### Beispiel 1 - Herstellung des C16-Spinnenseidenproteins

Die Herstellung des C16-Spinnenseidenproteins erfolgte biotechnologisch unter Verwendung plasmidhaltiger *Escherichia coli* Expressionsstämme. Design und Klonierung des C16-Spinnenseidenproteins (auch ADF4 genannt) sind in Hümmerich et al. (Biochemistry 43, 2004, 13604-13012) beschrieben. Im Gegensatz zum dort beschriebenen Verfahren wurde C16-Spinnenseidenprotein im *E. coli*-Stamm BL21 Gold (DE3) (Stratagene) hergestellt. Die Anzucht erfolgt in Techfors-Fermentern (Infors HAT, Schweiz) unter Verwendung eines Minimalmediums und Fed-Batch Techniken.

| | |
|---|---|
| Minimalmedium: | 2,5 g/l Citronensäuremonohydrat |
| | 4 g/l Glycerin |
| | 12,5 g/l Kaliumdihydrogenphosphat |
| | 6,25 g/l Ammoniumsulfat |
| | 1,88 g/l Magnesiumsulfatheptahydrat |
| | 0,13 g/l Calciumchloriddihydrat |
| | 15,5 ml/l Spurenelementelösung (40 g/l Citronensäuremonohydrat; 11 g/l Zink(II)sulfatheptahydrat; 8,5 g/l Diammoniumeisen(II)sulfatheptahydrat; 3 g/l Mangan(II)sulfatmonohydrat; 0,8 g/l Kupfer(II)sulfatpentahydrat; 0,25 g/l Kobalt(II)sulfatheptahydrat) |
| | 3 ml/l Vitaminlösung (6,3 mg/ml Thiaminhydrochlorid; 0,67 mg/ml Vitamin B12) |
| | pH 6,3 |

| | |
|---|---|
| Feed-Lösung: | 790 g/l Glycerin |
| | 6,9 g/l Citronensäuremonohydrat |
| | 13,6 g/l Natriumsulfat |
| | 1,05 g/l Diammoniumeisen(II)sulfatheptahydrat |
| | 13 mg/l Thiaminhydrochlorid |

Die Zellen wurden bei 37°C bis zu einer OD₆₀₀ von 100 angezogen, danach erfolgte die Induktion der Proteinexpression mit 0,1mM Isopropyl β-D-1-thiogalactopyranoside (IPTG). Am Ende der Fermentation (8 bis 12 Stunden nach Induktion) wurden die Kulturen geerntet. Der Hauptanteil des Proteins befand sich in "Inclusion Bodies".

Nach der Zellernte wurde das Pellet in 20mM 3-(-N-Morpholino)propanesulfonic acid (MOPS) pH 7,0 resuspendiert (5L Puffer pro Kilogramm Feuchtmasse). Anschließend erfolgte der Zell-Aufschluss unter Verwendung eines Micofluidizer M-110EH (Microfluidics, US) bei Drücken von 1200 bis 1300 bar. Nach Sedimentation enthielt das Pellet nach Aufschluss neben den "Inclusion Bodies" noch Zelltrümmer und Membranbestandteile, welche durch zwei Waschschritte entfernt wurden. In einem ersten Waschschritt wurde das Pellet in 2,5 Volumen Tris-Puffer (50 mM Tris/HCl, 0,1% Triton X-100, pH 8,0) resuspendiert und anschließend der verbleibende Feststoff durch Zentrifugation sedimentiert. Ein zweiter Waschschritt erfolgte unter Verwendung von Tris-Puffer (50 mM Tris/HCl, 5mM EDTA, pH 8,0). Das abermals nach Sedimentation erhaltene Pellet war nahezu frei von Membran- und Zelltrümmern.

Die gereinigten "Inclusion Bodies" wurden in Guanidiniumthiocyanat (Roth, Germany) gelöst, wobei pro 1 g Pellet (Feuchtmasse) 1,6 g Guanidiniumthiocyanat zugegeben wurden. Die "Inclusion Bodies" lösten sich unter Rühren bei leichter Erwärmung (50°C). Zur Abtrennung evtl. vorhandener nicht-löslicher Bestandteile wurde anschließend noch eine Zentrifugation durchgeführt. Um eine wässrige C16-Spinnenseidenproteinlösung zu erhalten, wurde dann eine 16-stündige Dialyse gegen 5 mM Kaliumphosphatpuffer (pH 8,0) durchgeführt (Verdünnungsfaktor der Dialyse: 200).

Verunreinigende *E. coli*-Proteine bildeten bei der Dialyse Aggregate, welche durch Zentrifugation abgetrennt werden konnten. Die erhaltene Proteinlösung wies eine Reinheit von ∼95% C16-Spinnenseidenprotein auf.

Die erhaltene wässrige Proteinlösung kann entweder direkt zum Elektroverspinnen verwendet oder zwecks besserer Lagerfähigkeit zu Protein-Microbeads weiterverarbeitet werden. Zur Herstellung von C16-Protein-Microbeads wurde die wässrige C16-Spinnenseidenproteinlösung wird mit 0,25 Volumen einer 4-molaren Ammoniumsulfatlösung versetzt. Unter Einwirkung des Ammoniumsulfats assemblieren die Proteinmonomere zu kugelförmigen Gebilden, welche hier als Microbeads bezeichnet werden. Die Microbeads wurden durch Zentrifugation abgetrennt, drei Mal mit destilliertem Wasser gewaschen und anschließend gefriergetrocknet.

### Beispiel 2 - Formulierung von Guaiacol Glyceryl Ether als Effektstoff mittels Elektroverspinnen

Um die Verwendbarkeit des beschriebenen Verfahrens für die Formulierung von pharmazeutisch aktiven Substanzen, insbesondere solchen zur Behandlung von Husten- und Atemwegserkrankungen, zu zeigen, wurde beispielhaft der Wirkstoff Guaiacol Glyceryl Ether (auch Guaifenesin) mittels Elektroverspinnen in C16-Spinnenseidenprotein-Flächengebilde (z.B. Protein-Filme, Protein-Fasern, Protein-Vliesstoffe) verkapselt.

Für die Herstellung einer verspinnbaren Lösung wurden C16-Spinnenseidenprotein-Microbeads (14 % [w/w]) und der Wirkstoff Guaiacol Glyceryl Ether (10 % [w/w]) zusammen in Ameisensäure (98-100% p.a.) gelöst. Es wurden 200 ml Ameisensäure in einem Becherglas vorgelegt und anschließend sukzessive 50,4 g C16-Spinnenseidenprotein und 36 g Guaiacol Glyceryl Ether (Fa. Sigma, Deutschland) eingerührt. Nachdem die Stoffe vollständig gelöst waren, wurde die Lösung mit Ameisensäure (98-100%) auf 360 g aufgefüllt.

Alternativ kann auch wässrige C16-Spinnenseidenproteinlösung (siehe Beispiel 1) als Ausgangsstoffbasis genutzt werden. Der Wirkstoff wird dann direkt in der wässrigen Proteinlösung gelöst bzw. bei Einsatz höherer Wirkstoffkonzentrationen in einem alternativen Lösungsmittel (z.B. Ameisensäure) vorgelöst und dann mit der Proteinlösung gemischt. Um die Viskosität der Spinnlösung zu erhöhen, können dann zusätzlich wasserlösliche Polymere oder wässrige Polymerdispersionen zugemischt werden.

Die Lösung aus C16-Spinnenseidenprotein und Guaiacol Glyceryl Ether wurde drei Stunden lang in einer Nanospider-Apparatur der Firma Elmarco wie oben beschrieben versponnen. Die Schichtdicke der erhaltenen Protein-Flächengebilde wurde mit dem Schichtdickenmessgerät Millitron (Firma: Mahr Feinprüf, Deutschland) bestimmt und lag bei 0,01 - 0,2 mm.

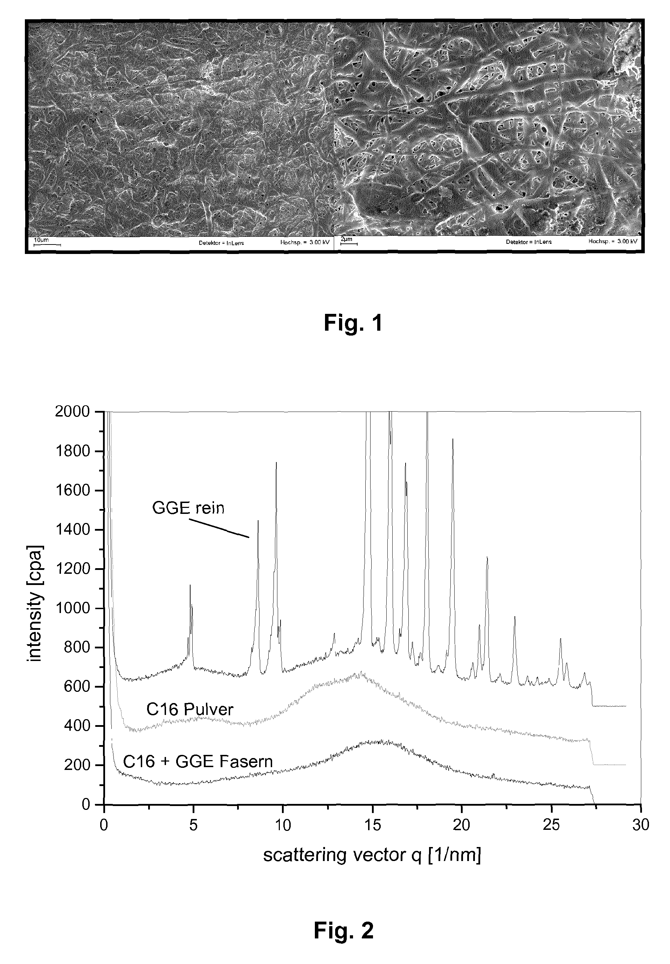

Die elektronenmikroskopische Analyse der so hergestellten C16-Spinnenseidenprotein-Flächengebilde mit eingeschlossenem Guaiacol Glyceryl Ether ergab, dass es sich hauptsächlich um Fasern mit einem Durchmesser bis zu 2 µm und darunter handelt (Figur 1).

Im Gegensatz zum reinen Guaiacol Glyceryl Ether zeigen sich in der C16-Spinnenseidenprotein / Guaiacol Glyceryl Ether-Formulierung in der Röntgenbeugung keine kristallinen Peaks (Figur 2). Demnach ist davon auszugehen, dass der Wirkstoff amorph oder als feste Lösung verkapselt wurde, was seine Bioverfügbarkeit positiv beeinflussen kann.

Um die Wirkstoff-Freisetzung aus einer möglichst relevanten Applikationsform zu überprüfen, wurden aus den C16-Spinnenseidenprotein-Flächengebilden Tabletten verpresst. Es wurden jeweils 300 mg Material unter Vakuum und 100 Bar Druck ca. 10 min lang in einer KBr-Presse (Firma: Paul-Otto-Weber, Deutschland) verpresst. Die Tabletten hatten einen Durchmesser von etwa 13 mm und eine Dicke von etwa 2 mm.

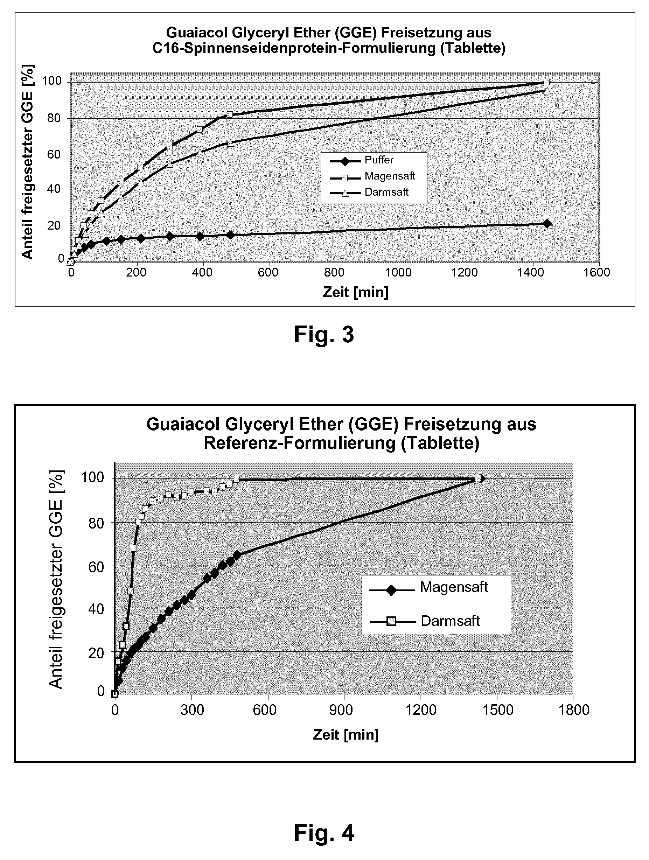

Die Freisetzung von Guaiacol Glyceryl Ether aus den Tabletten wurde nach Behandlung mit künstlichem Magensaft und künstlichem Darmsaft mittels HPLC (Säule: Luna C8(2), 150*3,0mm [Fa. Phenomenex, Deutschland]; Vorsäule: C18 ODS; Detektion: UV 210nm; Eluent A: 10mM KH₂PO₄, pH2,5; Eluent B: Acetonitril) bestimmt. Während in den Kontrollansätzen (Puffer) lediglich maximal 20 % der verkapselten Wirkstoffmenge freigesetzt werden, wird in Magen- und Darmsaft gesteuert durch die vorhandenen enzymatischen Aktivitäten (Proteasen) eine 100 %ige Freisetzung innerhalb 24 h erzielt (Figur 3). Sowohl in Magen- als auch in Darmsaft wird der Wirkstoff Guaiacol Glyceryl Ether kontinuierlich freigesetzt. In den ersten 8 h werden in den Ansätzen mit Darmsaft etwa 65 % des Wirkstoffes freigesetzt, während im Magensaft in dieser Zeitspanne schon etwa 80 % des Wirkstoffes frei werden (Figur 3).

Um den nach 24 h aus der Formulierung noch nicht freigesetzten Anteil an Guaiacol Glyceryl Ether zu bestimmen, wurden die Ansätze mit den verbliebenen C16-Spinnenseidenprotein-Aggregaten auf pH 7,0 eingestellt, jeweils 100 µl Proteinase K (435 U/ml, Roche, Deutschland) hinzu gegeben und die Ansätze bei 37 °C und 120 upm bis zur vollständigen Auflösung aller Aggregate weiter inkubiert. Anschließend wurde der Wirkstoffgehalt in Lösung mittels HPLC-Analytik quantifiziert. Aus dem Endwert und den vorher bestimmten Zwischenwerten konnte dadurch die Beladungsdichte der C16-Spinnenseidenproteinformulierung mit dem Wirkstoff Guaiacol Glyceryl Ether bestimmt werden. Die Beladungsdichte lag bei allen untersuchten Tabletten zwischen 31 und 33 % [w/w], woraus sich eine durchschnittliche Beladungsdichte des zu Tabletten verpressten C16-Spinnenseidenprotein-Flächengebildes mit 32,2 % [w/w] Guaiacol Glyceryl Ether ergab (Tab. 1).

**Tab. 1: Beladungsdichten der C16-Spinnenseidenprotein-Formulierung (Tabletten) mit dem Wirkstoff Guaiacol Glyceryl Ether.**

| Ansatz | Masse Tablette [mg] | GGE in Lösung [mg] | mg GGE pro mg Tablette | Beladungsdichte [%] |
|---|---|---|---|---|
| Puffer | 300 | 97,4 | 0,325 | 32,5 |
| Magensaft | 299 | 94,7 | 0,317 | 31,7 |
| Darmsaft | 300 | 97,4 | 0,325 | 32,5 |
| | Durchschnittliche Beladunqsdichte | | | **32,2** |

Kontrollansätze mit einer formulierten Referenzprobe des Wirkstoffes Guaiacol Glyceryl Ether (Tabletten der Handelsmarke Mucinex^{®}, Fa. Adams Respiratory Therapeutics) zeigen eine kontinuierliche, verzögerte Wirkstoff-Freisetzung nur unter Magenbedingungen (Figur 4). Bei einer durchschnittlichen Magenverweildauer der Wirkstoff-Formulierung von 2-5 Stunden wären zu diesem Zeitpunkt maximal 50 % Wirkstoff freigesetzt. Unter Darmbedingungen wird etwa 90 % des Wirkstoffes innerhalb kurzer Zeit (2-3 Stunden) aus der Mucinex^{®}-Formulierung freigesetzt (Figur 4).

Aufgrund der in Figur 4 dargestellten Versuchsergebnisse kann geschlussfolgert werden, dass eine kontinuierliche, verzögerte Guaiacol Glyceryl Ether-Freisetzung und damit auch dessen Aufnahme bei Mucinex^{®}-Tabletten unter Darmbedingungen nicht mehr stattfindet und ein Großteil des Wirkstoffes somit über Ausscheidungsprozesse verloren geht. Erfindungsgemäße C16-Spinnenseidenproteinformulierungen des Wirkstoffes Guaiacol Glyceryl Ether zeigen dagegen unter Magenbedingungen und auch unter Darmbedingungen eine kontinuierliche, verzögerte Freisetzung, die eine länger anhaltende Wirkstoffaufnahme begünstigen würde. Demnach wären Formulierungen des Wirkstoffes Guaiacol Glyceryl Ether mit amphiphilen selbstassemblierenden Proteinen deutlich universeller einsetzbar und erlauben selbst nach Magenpassage noch kontinuierliche, verzögerte Wirkstoff-Freisetzung und anschließend WirkstoffAufnahme.

### Beispiel 3 - Formulierung von Clotrimazol als Effektstoff mittels Elektroverspinnen

Um die Verwendbarkeit des beschriebenen Verfahrens für die Formulierung von weiteren pharmazeutisch aktiven, insbesondere schwer wasserlöslichen Substanzen zu zeigen, wurde beispielhaft der Wirkstoff Clotrimazol mittels Elektroverspinnen in C16-Spinnenseidenprotein-Flächengebilde (z.B. Protein-Filme, Protein-Fasern, Protein-Vliesstoffe) verkapselt.

Für die Herstellung einer verspinnbaren Lösung wurden C16-Spinnenseidenprotein-Microbeads (14 % [w/w]) und der Wirkstoff Guaiacol Glyceryl Ether (10 % [w/w]) zusammen in Ameisensäure (98-100% p.a.) gelöst. Es wurden 200 ml Ameisensäure in einem Becherglas vorgelegt und anschließend sukzessive 50,4 g C16-Spinnenseidenprotein und 36 g Clotrimazol (Fa. Sigma, Deutschland) eingerührt. Nachdem die Stoffe vollständig gelöst waren wurde die Lösung mit Ameisensäure auf 360 g aufgefüllt.

Alternativ kann auch wasserlösliche C16-Spinnenseidenproteinlösung (siehe Beispiel 1) als Ausgangsstoffbasis genutzt werden. Der Wirkstoff wird dann direkt in der wässrigen Proteinlösung gelöst bzw. bei Einsatz höherer Wirkstoffkonzentrationen in einem alternativen Lösungsmittel (z.B. Ameisensäure) vorgelöst und dann mit der Proteinlösung gemischt. Um die Viskosität der Spinnlösung zu erhöhen können dann zusätzlich wasserlösliche Polymere oder Polymerdispersionen zugemischt werden.

Die Lösung aus C16-Spinnenseidenprotein und Clotrimazol wurde drei Stunden lang in einer Nanospider-Apparatur der Firma Elmarco wie oben beschrieben versponnen.

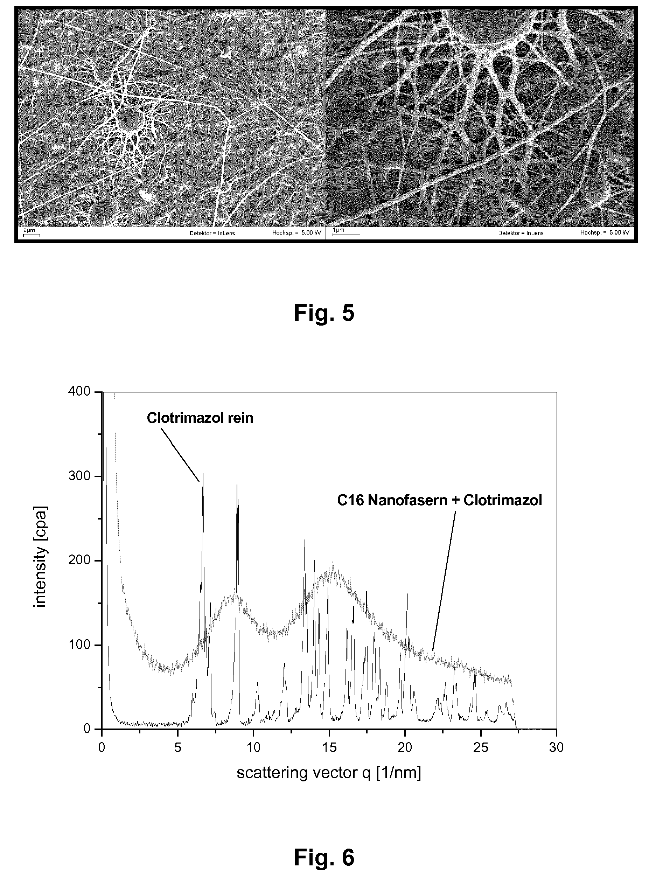

Die elektronenmikroskopische Analyse der so hergestellten C16-Spinnenseidenprotein-Flächengebilde mit eingeschlossenem Clotrimazol ergab, dass es sich hauptsächlich um Fasern mit einem Durchmesser etwa 50 nm bis zu 1 µm handelt (Figur 5).

Im Gegensatz zum reinen Clotrimazol zeigen sich in der C16-Spinnenseidenprotein / Clotrimazol Formulierung in der Röntgenbeugung keine kristallinen Peaks (Figur 6). Demnach ist davon auszugehen, dass der Wirkstoff amorph oder als feste Lösung verkapselt wurde, was seine Bioverfügbarkeit positiv beeinflussen kann.

Wie schon in Beispiel 2 beschrieben, wurden auch aus den C16-Spinnenseidenprotein-Flächengebilden mit verkapseltem Wirkstoff Clotrimazol Tabletten gepresst. Um die Freisetzungskinetik des Wirkstoffes zu bestimmen wurden wie in Beispiel 2 beschrieben die Tabletten in künstlichem Magensaft, künstlichem Darmsaft und 5 mM Kaliumphosphatpuffer (Kontrolle) inkubiert. Die Quantifizierung des freigesetzten Clotrimazols erfolgte aufgrund seiner schlechten Wasserlöslichkeit (und damit Neigung zu Aggregatbildung in wässrigen Systemen) nach Extraktion des Überstandes mit THF durch absorptionsphotometrische Bestimmung bei 262 nm.

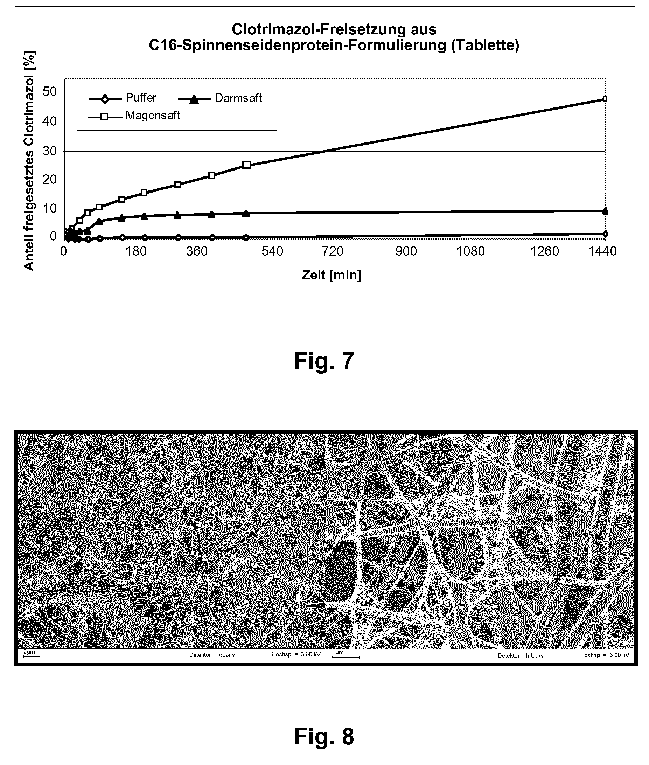

Während im Kontrollansatz (Puffer ohne Proteasen) lediglich maximal 2 % der verkapselten Wirkstoffmenge freigesetzt werden, wird in Magensaft gesteuert durch die vorhandene enzymatische Aktivität (Proteasen) etwa 50 % Freisetzung innerhalb 24 h erzielt (Figur 7). Dabei wird der Wirkstoff Clotrimazol kontinuierlich freigesetzt. Im Darmsaft hingegen werden nach 24 h nur etwa 20 % des Wirkstoffes freigesetzt (Figur 7). Die C16-Spinnenseidenprotein / Clotrimazol-Formulierung scheint unter den dabei vorherrschenden eher neutralen pH-Werte im betrachteten Zeitbereich so stabil, dass nur eine abgeschwächte Freisetzung zu beobachten ist.

Um den nach 24 h aus der Formulierung noch nicht freigesetzten Anteil an Clotrimazol zu bestimmen, wurden die Ansätze mit den nicht proteolytisch abgebauten C16-Spinnenseidenprotein-Flächengebilden mit 3 ml THF versetzt und für max. 48 weiter schüttelnd inkubiert. Anschließend wurde der Wirkstoffgehalt absorptionsphotometrisch bei 262 nm quantifiziert. Aus dem Endwert und den vorher bestimmten Zwischenwerten konnte dadurch die Beladungsdichte der C16-Spinnenseidenproteinformulierung mit dem Wirkstoff Clotrimazol bestimmt werden. Die Beladungsdichte lag bei allen untersuchten Tabletten zwischen 27 und 33 % [w/w], woraus sich eine durchschnittliche Beladungsdichte des zu Tabletten verpressten C16-Spinnenseidenprotein-Flächengebildes mit etwa 30 % [w/w] Clotrimazol ergab (Tab. 2).

**Tab. 2: Beladungsdichten der C16-Spinnenseidenprotein-Formulierung (Tabletten) mit dem Wirkstoff Clotrimazol.**

| Ansatz | Masse Tablette [mg] | Clotrimazol in Lösung [mg] | mg Clotrimazol pro mg Tablette | Beladungsdichte [%] |
|---|---|---|---|---|
| Puffer | 304 | 92,2 | 0,303 | 30,3 |
| Magensaft | 302 | 99,1 | 0,328 | 32,8 |
| Darmsaft | 299 | 82,0 | 0,274 | 27,4 |
| | Durchschnittliche Beladungsdichte | | | **30,2** |

### Beispiel 4 - Formulierung von Metazachlor als Effektstoff mittels Elektroverspinnen

Um die Verwendbarkeit des beschriebenen Verfahrens für die Formulierung von Pflanzenschutzwirkstoffen zu zeigen, wurde beispielhaft der Wirkstoff Metazachlor mittels Elektroverspinnen in C16-Spinnenseidenprotein-Flächengebilde (z.B. Protein-Filme, Protein-Fasern, Protein-Vliesstoffe) verkapselt.

Für die Herstellung einer verspinnbaren Lösung wurden C16-Spinnenseidenprotein-Microbeads (14 % [w/w]) und der Wirkstoff Metazachlor (10 % [w/w]) zusammen in Ameisensäure (98-100% p.a.) gelöst. Es wurden 200 ml Ameisensäure in einem Becherglas vorgelegt und anschließend sukzessive 50,4 g C16-Spinnenseidenprotein und 36 g Metazachlor eingerührt. Nachdem die Stoffe vollständig gelöst waren wurde die Lösung mit Ameisensäure (98-100%) auf 360 g aufgefüllt.

Alternativ kann auch wässrige C16-Spinnenseidenprotein-Lösung (siehe Beispiel 1) als Ausgangsstoffbasis genutzt werden. Der Wirkstoff wird dann direkt in der wässrigen Proteinlösung gelöst bzw. bei Einsatz höherer Wirkstoffkonzentrationen in einem alternativen Lösungsmittel (z.B. Ameisensäure) vorgelöst und dann mit der Proteinlösung gemischt. Um die Viskosität der Spinnlösung zu erhöhen können dann zusätzlich wasserlösliche Polymere oder Polymerdispersionen zugemischt werden.

Die Lösung aus C16-Spinnenseidenprotein und Metazachlor wurde drei Stunden lang in einer Nanospider-Apparatur der Firma Elmarco wie oben beschrieben versponnen.

Die elektronenmikroskopische Analyse der so hergestellten C16-Spinnenseidenprotein-Flächengebilde mit eingeschlossenem Metazachlor ergab, dass es sich hauptsächlich um Fasern mit einem Durchmesser etwa 50 nm bis zu 500 nm handelt (Figur 8).

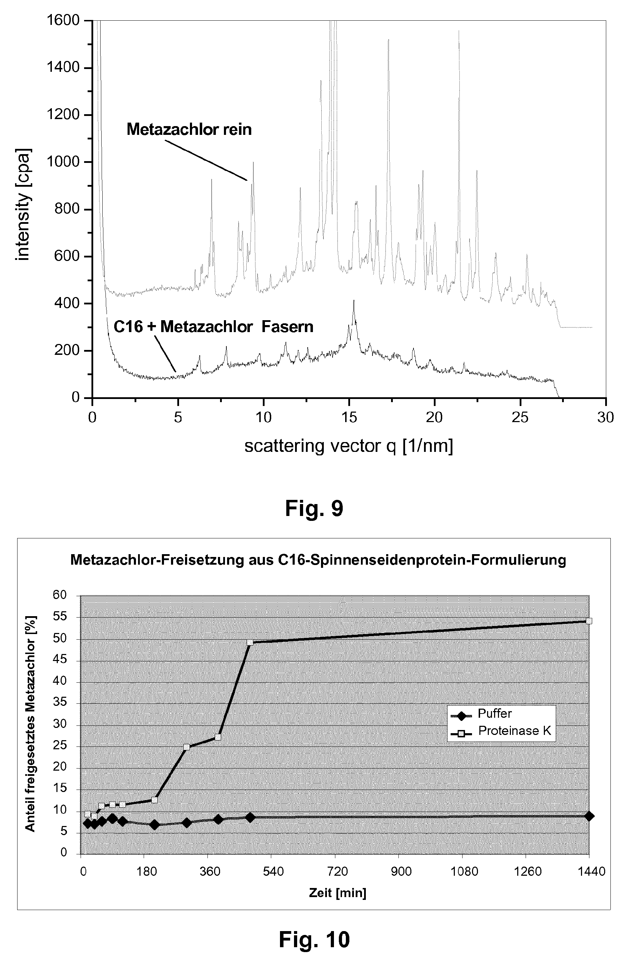

Reines Metazachlor zeigt in der Röntgenbeugung stark kristalline Anteile (Figur 9). Im Gegensatz dazu weist die C16-Spinnenseidenprotein / Metazachlor-Formulierung in der Röntgenbeugung geringer ausgeprägte teilkristalline Bereiche auf, die auf den Wirkstoff Metazachlor zurückzuführen sind (Figur 9).

Zur Bestimmung der Beladungsdichte mit dem Wirkstoff Metazachlor wurden in zwei Ansätzen (1. Ansatz: 25 mg; 2. Ansatz: 26 mg) das hergestellte Spinnenseidenprotein-Flächengebilde mit jeweils 2 ml THF versetzt und für 5 h schüttelnd bei 1800 upm inkubiert. Der durch die THF-Behandlung quantitativ herausgelöste Wirkstoff Metazachlor wurde anschließend absorptionsphotometrisch bei 264 nm bestimmt. Es zeigte sich, dass im Ansatz 1 eine Beladungsdichte von etwa 40 % [w/w], im zweiten Ansatz von etwa 45 % [w/w] vorlag.

Um die Freisetzungskinetik des Wirkstoffes zu bestimmen wurden die C16-Spinnenseidenprotein-Flächengebilde mit verkapseltem Metazachlor in 5 mM Kaliumphosphatpuffer versetzt mit 0,5 % [w/v] Proteinase K inkubiert. Die Quantifizierung des freigesetzten Metazachlors erfolgte nach Abtrennung der noch intakten C16-Spinnenseidenprotein-Flächengebilde durch Extraktion des Überstandes mit THF und anschließende absorptionsphotometrische Bestimmung bei 264 nm.

Während im Kontrollansatz (Puffer ohne Proteinase K) nach 24 h nur etwa 10 % der verkapselten Wirkstoffmenge freigesetzt wurde, konnte im Proteinase K-enthaltenden Ansatz die Freisetzung von etwa 55 % Metazachlor in der gleichen Zeitspanne erzielt werden (Figur 10). Nach 7 Tagen konnten aus einer solchen C16-Spinnenseidenprotein / Metazachlor-Formulierung etwa 70 % kontinuierliche Wirkstoff-Freisetzung erreicht werden (nicht gezeigt).

### Beispiel 5 - Formulierung von Uvinul A+ als Effektstoff mittels Elektroverspinnen

Um die Verwendbarkeit des beschriebenen Verfahrens für die Formulierung von kosmetischen Wirkstoffen zu zeigen, wurde beispielhaft der Wirkstoff Uvinul A+ mittels Elektroverspinnen in C16-Spinnenseidenprotein-Flächengebilde (z.B. Protein-Filme, Protein-Fasern, Protein-Vliesstoffe) verkapselt.

Für die Herstellung einer verspinnbaren Lösung wurden C16-Spinnenseidenprotein-Microbeads (14 % [w/w]) und der Wirkstoff Uvinul A+ (10 % [w/w]) zusammen in Ameisensäure (98-100% p.a.) gelöst. Es wurden 200 ml Ameisensäure in einem Becherglas vorgelegt und anschließend sukzessive 50,4 g C16-Spinnenseidenprotein und 36 g Uvinul A+ eingerührt. Nachdem die Stoffe vollständig gelöst waren wurde die Lösung mit Ameisensäure (98-100%) auf 360 g aufgefüllt.

Alternativ kann auch wässrige C16-Spinnenseidenprotein-Lösung (siehe Beispiel 1) als Ausgangsstoffbasis genutzt werden. Der Wirkstoff wird dann direkt in der wässrigen Proteinlösung gelöst bzw. bei Einsatz höherer Wirkstoffkonzentrationen in einem alternativen Lösungsmittel (z.B. Ameisensäure) vorgelöst und dann mit der Proteinlösung gemischt. Um die Viskosität der Spinnlösung zu erhöhen können dann zusätzlich wasserlösliche Polymere oder Polymerdispersionen zugemischt werden.

Die Lösung aus C16-Spinnenseidenprotein und Uvinul A+ wurde drei Stunden lang in einer Nanospider-Apparatur der Firma Elmarco wie oben beschrieben versponnen.

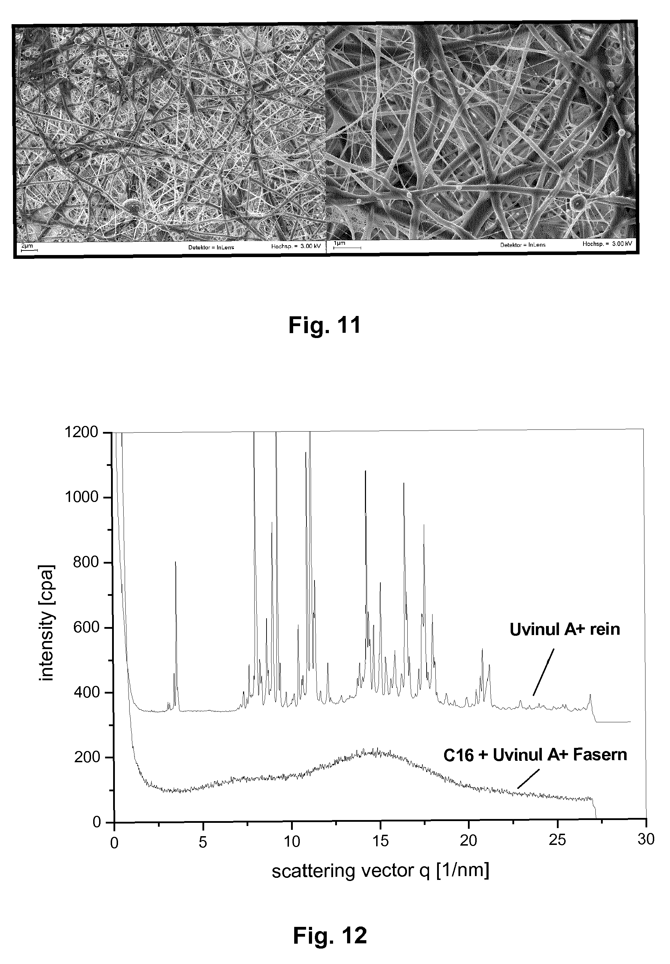

Die elektronenmikroskopische Analyse der so hergestellten C16-Spinnenseidenprotein-Flächengebilde mit eingeschlossenem Uvinul A+ ergab, dass es sich hauptsächlich um Fasern mit einem Durchmesser etwa 50 nm bis zu 400 nm handelt (Figur 11).

Im Gegensatz zum reinen Uvinul A+ zeigen sich in der C16-Spinnenseidenprotein / Uvinul A+-Formulierung in der Röntgenbeugung keine kristallinen Peaks (Figur 12). Demnach ist davon auszugehen, dass der Wirkstoff amorph oder als feste Lösung verkapselt wurde, was seine Bioverfügbarkeit positiv beeinflussen kann.

Zur Bestimmung der Beladungsdichte mit dem Wirkstoff wurden in zwei Ansätzen (1. Ansatz: 7,9 mg; 2. Ansatz: 7,8 mg) das hergestellte C16-Spinnenseidenprotein-Flächengebilde mit jeweils 2 ml THF versetzt und für 5 h schüttelnd bei 1800 upm inkubiert. Der durch die THF-Behandlung quantitativ herausgelöste Wirkstoff Uvinul A+ wurde anschließend absorptionsphotometrisch bei 352 nm bestimmt. Es zeigte sich, dass im Ansatz 1 eine Beladungsdichte von etwa 25 % [w/w], im zweiten Ansatz von etwa 26,2 % [w/w] vorlag.

Um die Freisetzungskinetik des Wirkstoffes zu bestimmen wurden die C16-Spinnenseidenprotein / Uvinul A+-Flächengebilde in 5 mM Kaliumphosphatpuffer versetzt mit 0,25 % [w/v] Proteinase K inkubiert. Die Quantifizierung des freigesetzten Uvinul A+ erfolgte nach Abtrennung der noch intakten C16-Spinnenseidenprotein-Flächengebilde durch Extraktion des Überstandes mit THF und anschließende absorptionsphotometrische Bestimmung bei 352 nm.

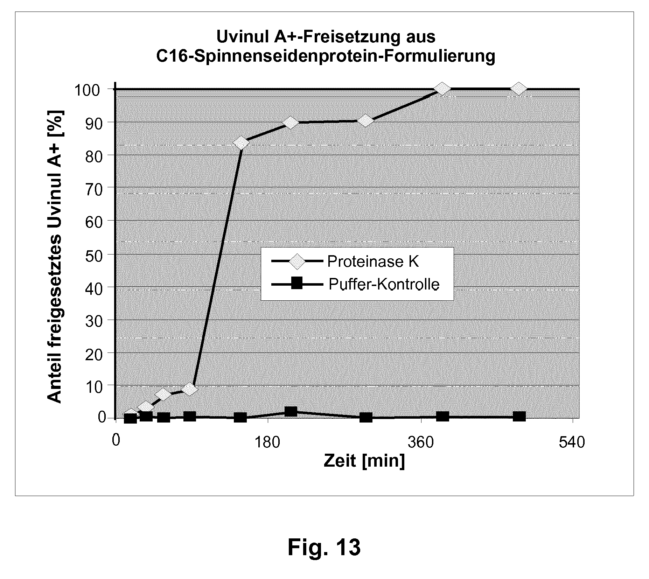

Während im Kontrollansatz (Puffer ohne Proteinase K) auch nach 24 h kein Wirkstoff freigesetzt wurde, konnte im Proteinase K-enthaltenden Ansatz die Freisetzung von 100 % Uvinul A+ nach 6-7 Stunden erzielt werden (Figur 13).

### Beispiel 6 - Herstellung von Fasern aus reinen R16 und S16 Proteinen mittels Elektrospinnen

Für die Herstellung von verspinnbaren R16- bzw. S16-Protein-Lösungen wurden R16-bzw. S16-Protein-Microbeads genutzt. Diese können wie in WO 2008/155304 beschrieben hergestellt werden. Alternativ kann eine Herstellung wie in Beispiel 1 beschrieben erfolgen. Dabei kamen Plasmidvektoren bzw. E. coli-Produktionsstämme zum Einsatz, welche kodierende DNA-Sequenzen für das R16- bzw. S16-Protein enthielten.

Für die Herstellung einer verspinnbaren Lösung wurden R16-Protein-Microbeads; 18 %ig [w/w]) in Ameisensäure (98-100% p.a.) gelöst. Das R16-Protein wurde in einem kleinen Ansatz versponnen, um die Faserbildung nachzuweisen. Dafür wurden 0,36 g R16-Protein-Microbeads in 1,64 g Ameisensäure gelöst und damit die Spritze der Verspinnanlage befüllt.

Die R16-Proteinlösung wurde mit Hilfe der düsenbasierten Elektrospinnanlage versponnen. Die Proteinlösung wurde dafür in einem elektrischen Feld unter geringem Druck durch eine mit einem Pol einer Spannungsquelle verbundene Kanüle extrudiert. Aufgrund der durch das elektrische Feld erfolgenden elektrostatischen Aufladung der Proteinlösung entstand ein auf die Gegenelektrode gerichteter Materialstrom, der sich auf dem Weg zur Gegenelektrode verfestigte und in Form von dünnen Fasern auf einem Glasobjektträger ablagerte.

Folgende Parameter wurden benutzt:
Rel. Luftfeuchtigkeit 27 %,
Spinntemperatur 23 °C,
elektrische Spannung 60 kV,
Elektrodenabstand 15 cm,
Kanülendurchmesser 0,9 mm.
Vorschub manuell

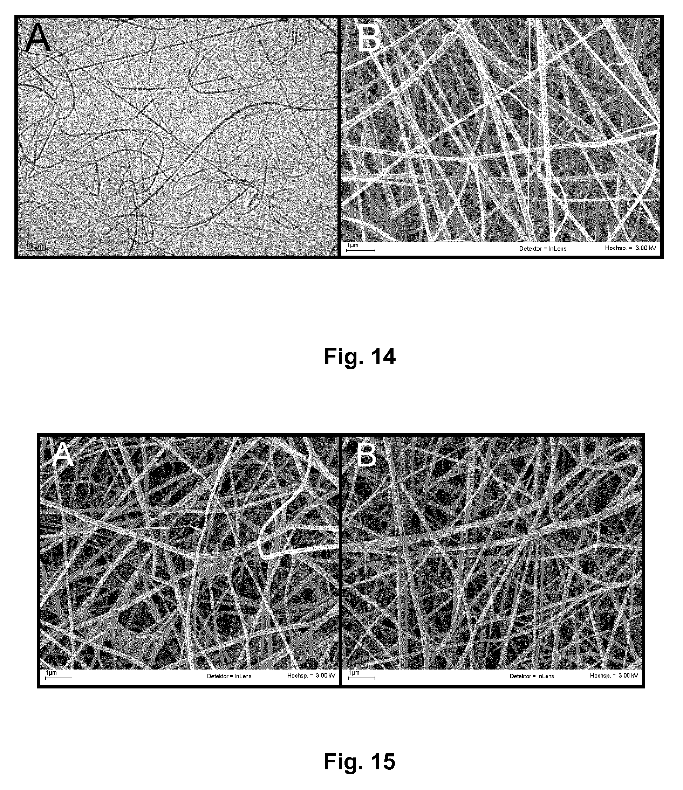

Die elektronenmikroskopische Analyse der so hergestellten R16-Protein-Flächengebilde ergab, dass die Lösung faserbildend war und dass es sich hauptsächlich um Fasern mit einem Durchmesser von etwa 200 nm bis zu 500 nm handelt (Figur 14A).

Die S16-Proteinlösung wurde mit der Nanospider-Apparatur der Firma Elmarco versponnen. Die eingesetzte Lösung befand sich in einem Behälter, in dem eine Spinnelektrode (Walze) permanent rotierte. Die Spinnelektrode war in diesem Fall eine Elektrode auf Basis von Metalldrähten. Dabei befand sich ein Teil der Formulierung beständig auf der Oberfläche der Drähte. Das elektrische Feld zwischen der Walze und der Gegenelektrode (oberhalb der Walze) bewirkte, dass sich aus der Formulierung erst flüssige Jets ausbildeten, die dann auf dem Weg zur Gegenelektrode vorhandenes Lösungsmittel verlieren bzw. erstarren. Der gewünschte Nanofaservlies (textiles Flächengebilde) entstand auf einem Polypropylen-Substrat, das zwischen den beiden Elektroden vorbeizog.

Es wurden Microbeads des S16-Proteins 12 %ig [w/w] in Ameisensäure (98-100% p.a.) gelöst. Für den S16-Ansatz wurden je 200 ml Ameisensäure in einem Becherglas vorgelegt und anschließend sukzessive 40 g S16-Protein eingerührt. Nachdem sich das S16-Protein vollständig gelöst hatte wurde die Lösung mit Ameisensäure (98-100%) auf 340 g aufgefüllt.

Folgende Parameter wurden benutzt:
Temperatur: 24°C
rel. Luftfeuchte: 22%
Spannung: 70-82kV
Elektrodenabstand: 25cm
Spinndauer: 1,5h

Das S16-Protein-Flächengebilde wies Fasern mit einem Durchmesser von etwa 100 nm bis zu 300 nm auf (Figur 14B).

Im Falle der Herstellung von R16- oder S16-Protein-Flächengebilde enthaltenden Medikalprodukten (z.B. Wundauflagen oder Pflastern) oder Hygieneprodukten (Wischtücher, Windeln, Binden etc.) bzw. der Verwendung von R16- bzw. S16-Proteinvliesstoffen in entsprechenden Anwendungen können als Trägersubstrat oder als Trägerstoff für das Flächengebilde das zu beschichtende Medikal- oder Hygieneprodukt selbst oder Teile bzw. einzelne Schichten davon verwendet werden.

Alternativ können auch wässrige R16- oder S16-Proteinlösungen (analog zu C16-Spinnenseidenprotein, Herstellung siehe Beispiel 1) als Ausgangsmaterial für die Herstellung von Fasern/Faserflächengebilden genutzt werden. Um die Viskosität der Spinnlösung zu erhöhen bzw. die Viskoelastizität der Lösungen zu erzeugen, können dann zusätzlich wasserlösliche Polymere, Polymerdispersionen oder Biopolymere (z.B. Proteine) zugemischt werden.

### Beispiel 7 - Formulierung von Uvinul A+ als Effektstoff in R16- und S16-Protein-Vliesstoffe mittels Elektroverspinnen

Um die Verwendbarkeit des beschriebenen Verfahrens für die Formulierung von Wirkstoffen zu zeigen, wurde beispielhaft der Wirkstoff Uvinul A+ mittels Elektroverspinnen in R16- bzw. S16-Protein-Flächengebilde (z.B. Protein-Filme, Protein-Fasern, Protein-Vliesstoffe) verkapselt.

Für die Herstellung einer verspinnbaren Lösung wurden R16-Protein-Microbeads (18 % [w/w]) bzw. S16-Protein-Microbeads (12 % [w/w]) und der Wirkstoff Uvinul A+ (10 % [w/w]) zusammen in Ameisensäure (98-100% p.a.) gelöst. Es wurden 200 ml Ameisensäure in einem Becherglas vorgelegt und anschließend sukzessive 61,2 g R16-Protein bzw. 40,0 g S16-Protein und 34 g Uvinul A+ eingerührt. Nachdem die Stoffe vollständig gelöst waren wurde die Lösung mit Ameisensäure (98-100%) auf 340 g aufgefüllt.

Auch hier kann alternativ wässrige R16- oder S16-Proteinlösungen (analog zu C16-Spinnenseidenprotein, Herstellung siehe Beispiel 1) als Ausgangsstoffbasis genutzt werden. Der Wirkstoff wird dann direkt in der wässrigen Proteinlösung gelöst bzw. bei Einsatz höherer Wirkstoffkonzentrationen in einem alternativen Lösungsmittel (z.B. Ameisensäure oder THF) vorgelöst und dann mit der Proteinlösung gemischt. Um die Viskosität der Spinnlösung zu erhöhen können dann zusätzlich wasserlösliche Polymere, Polymerdispersionen oder Biopolymere (z.B. Proteine) zugemischt werden.

Die Lösung aus R16-Protein bzw. S16-Protein und Uvinul A+ mit folgenden Parametern in der walzenbasierten Nanospider-Apparatur der Firma Elmarco versponnen:

| | **R16-Protein / Uvinul A+** | **S16-Protein / Uvinul A+** |
|---|---|---|
| **Spannung [kV]** | 82 | 82 |
| **Temperatur [°C]** | 24 | 24,5 |
| **Rel. Luftfeuchte [%]** | 33 | 27,5 |
| **Elektrodenabstand [cm]** | 25 | 25 |
| **Spinndauer [Std]** | 1 | 3 |

Die so hergestellten Protein-Flächengebilde mit eingeschlossenem Uvinul A+ wiesen vergleichbare Faserdurchmesser wie die Ansätze ohne Wirkstoff auf (Figur 15).

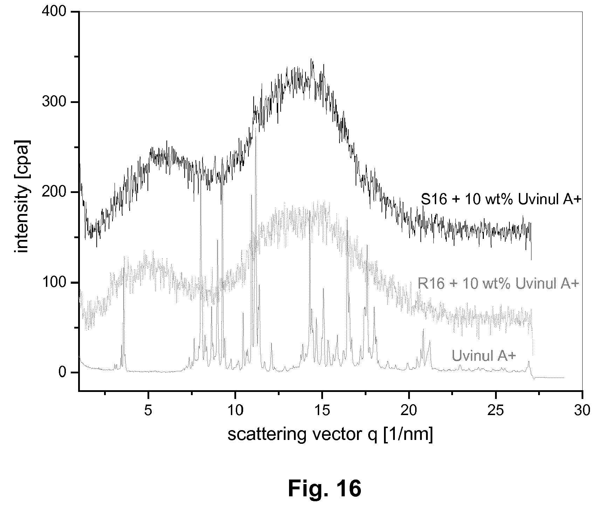

Im Gegensatz zum reinen Uvinul A+ zeigen sich in der R16-Protein / Uvinul A+-Formulierung in den Röntgenbeugungsspektren keine kristallinen Peaks. (Figur 16). Demnach ist davon auszugehen, dass der Wirkstoff amorph in die Fasern integriert ist. In dem S16-Protein mit Uvinul A+ konnten sehr schwache kristalline Signale detektiert werden. Es spricht dafür, dass der Effektstoff teilkristallin vorliegt.

Abweichend vom oben dargestellten Vorgehen wurde die Freisetzungskinetik des Wirkstoffes aus R16- bzw. S16-Protein / Uvinul A+-Flächengebilde folgendermaßen bestimmt. Es wurden jeweils 10 mg der R16- bzw. 5 mg der S16-Protein / Uvinul A+-Flächengebilde in 5 mM Kaliumphosphatpuffer mit 0,25 % [w/v] Proteinase K inkubiert. Für jeden geplanten Probennahmezeitpunkt wurde jeweils ein Ansatz zusammengestellt. Die Ansätze wurden bei 37 °C und 400 Upm im Thermomixer (Fa. Eppendorf) inkubiert. Die Quantifizierung des freigesetzten Uvinul A+ erfolgte zu den jeweiligen Zeitpunkten nach Abtrennung der noch intakten R16- bzw. S16-Protein-Flächengebilde durch Extraktion des Überstandes mit THF und anschließende absorptionsphotometrische Bestimmung bei 352 nm.

Zur Bestimmung der Beladungsdichte mit dem Wirkstoff wurden alle für die Freisetzungkinetik angesetzten Proben quantitativ mit THF extrahiert. In den Proben, bei denen kein vollständiger Abbau der Proteinflächengebilde erfolgt war, wurde auch das abgetrennte Proteinflächengebilde mit THF extrahiert und anschließend Uvinul A+ absorptionsphotometrisch quantifiziert. Beide Werte (Überstand und Pellet) wurden addiert, um die gesamte Beladungsdichte zu ermitteln. Aus den Beladungsdichten aller Zeitpunkte wurde anschließend die mittlere Beladungsdichte bestimmt. Es zeigte sich, dass im R16-Protein-Flächengebilde eine Uvinul A+-Beladungsdichte von etwa 33,5 % [w/w], im S16-Protein-Flächengebilde von etwa 49,6 % [w/w] vorlag.

Nach 24 h wurde aus dem R16-Flächengebilde im Kontrollansatz (Puffer ohne Proteinase K) lediglich 7,5 % Uvinul A+ freigesetzt. Aus dem S16-Flächengebilde wurde in diesem Zeitraum im Kontrollansatz 9,5 % Wirkstoff freigesetzt. In beiden Ansätzen blieben die Proteinflächengebilde aber intakt. Im gleichen Zeitraum war im Proteinase K-versetzten Ansatz das S16-Flächengebilde vollständig abgebaut. Der Proteinase K gesteuerte Abbau des R16-Flächengebildes erfolgte hingegen deutlich langsamer. Nach 24 h waren hier noch einzelne Reste des R16-Flächengebildes im Ansatz zu erkennen.

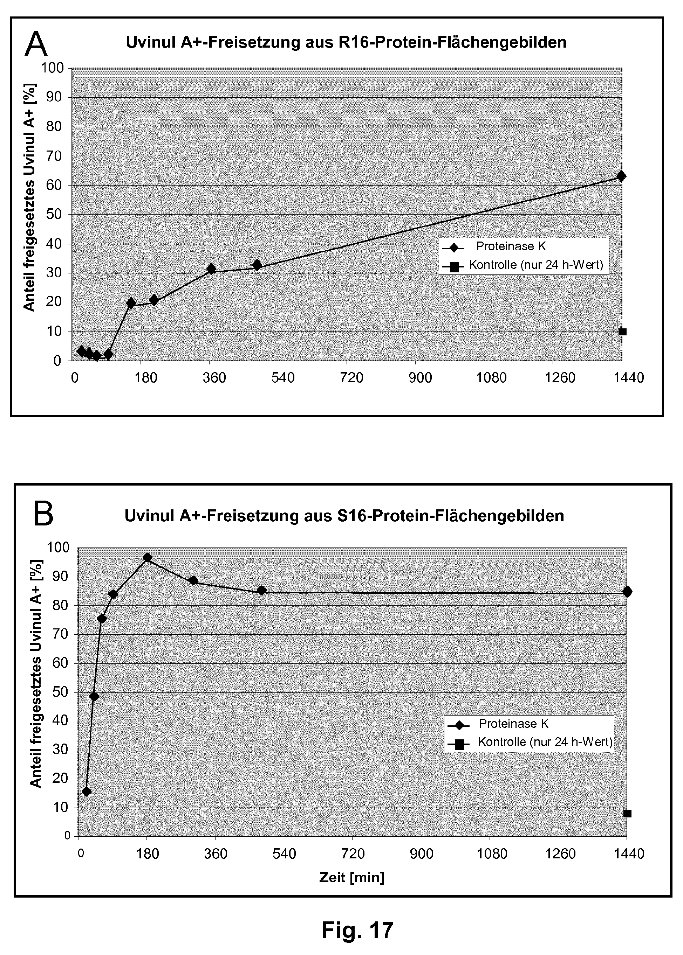

Im Proteinase K-enthaltenden R16-Protein-Ansatz waren nach 24 h etwa 63 % [w/w] des Uvinul A+ freigesetzt (Figur 17). Im S16-Protein-Ansatz hingegen wurde der gesamte Wirkstoff Uvinul A+ schon nach etwa 3 Stunden freigesetzt (Figur 17).

Weitere Ausführungsformen der Erfindung betreffen:
1. Wirkstoffhaltiges Faserflächengebilde, umfassend einen faserförmigen, polymeren löslichen und/ oder abbaubaren Wirkstoffträger und wenigstens einen, mit dem Träger assoziierten, und von dem Faserflächengebilde freisetzbaren Wirkstoff,
   wobei der Träger als Polymerkomponente wenigstens ein Biopolymer umfasst, das gegebenenfalls zusätzlich chemisch und/oder enzymatisch modifiziert ist.
2. Faserflächengebilde nach Ausführungsform 1, wobei das Faserflächengebilde erhältlich ist mittels eines Spinnverfahrens, insbesondere durch Elektroverspinnung einer elektroverspinnbaren Lösung, welche wenigstens ein Biopolymer und wenigstens einen Wirkstoff umfasst.
3. Faserflächengebilde nach einer der vorhergehenden Ausführungsformen, wobei der wenigstens eine Wirkstoff in amorpher, teilkristalliner oder kristalliner Form vorliegt.
4. Faserflächengebilde nach einer der vorhergehenden Ausführungsformen, wobei der Wirkstoff in den Träger integriert und/oder daran adsorbiert ist.
5. Faserflächengebilde nach einer der vorhergehenden Ausführungsformen, wobei das Biopolymer ein Protein, insbesondere ein amphiphiles, selbstassemblierendes Protein, ist.
6. Faserflächengebilde nach Ausführungsform 5, wobei es sich bei den amphiphilen selbstassemblierenden Proteinen um Microbead-bildende Proteine handelt.
7. Faserflächengebilde nach Ausführungsform 5, wobei es sich bei den amphiphilen selbstassemblierenden Proteinen um intrinsisch entfaltete Proteine handelt.
8. Faserflächengebilde nach Ausführungsform 5, wobei es sich bei den amphiphilen selbstassemblierenden Proteinen um Seidenproteine handelt.
9. Faserflächengebilde nach Ausführungsform 8, wobei es sich bei den amphiphilen selbstassemblierenden Proteinen um Spinnenseidenproteine handelt.
10. Faserflächengebilde nach Ausführungsform 9, wobei das Spinnenseidenprotein das C16-Spinnenseidenprotein umfassend eine Aminosäuresequenz gemäß SEQ ID NO: 2 ist, oder ein davon abgeleitetes verspinnbares Protein mit einer Sequenzidentität von wenigstens etwa 60%
11. Faserflächengebilde nach Ausführungsform 8, wobei das Seidenprotein ausgewählt ist unter dem R16-Protein umfassend eine Aminosäuresequenz gemäß SEQ ID NO: 4 und dem S16-Protein umfassend eine Aminosäuresequenz gemäß SEQ ID NO: 6; oder ein von diesen Proteinen abgeleitetes verspinnbares Protein mit einer Sequenzidentität von wenigstens etwa 60%.
12. Faserflächengebilde nach einer der vorhergehenden Ausführungsformen, wobei wenigstens ein pharmazeutischer Wirkstoff enthalten ist.
13. Faserflächengebilde nach Ausführungsform 12, wobei der Wirkstoff ein husten- und schleimlösender Wirkstoff ist.
14. Faserflächengebilde nach Ausführungsform 13, wobei der Wirkstoff Guaiacol Glyceryl Ether oder ein Derivat davon ist.
15. Faserflächengebilde nach einer der Ausführungsformen 1 bis 11, wobei der Wirkstoff ein Pflanzenschutzwirkstoff ist.
16. Faserflächengebilde nach einer der Ausführungsformen 1 bis 11, wobei der Wirkstoff ein haut- und/oder haarkosmetischer Wirkstoff ist.
17. Faserflächengebilde nach einer der vorhergehenden Ausführungsformen, wobei der Träger wenigstens eine weitere Polymerkomponente umfasst, ausgewählt unter synthetischen Polymeren.
18. Faserflächengebilde nach Ausführungsform 17, wobei das synthetische Polymer ein Homo- oder Copolymer ist.
19. Faserflächengebilde nach einer der vorhergehenden Ausführungsformen, wobei der polymere Träger ein Kompositpolymer ist, ausgewählt ist unter
   a. Mischungen aus mindestens 2 mischbaren Biopolymeren;
   b. Mischungen aus mindestens 2 nicht mischbaren Biopolymeren;
   c. Mischungen aus mindestens einem synthetischen Homo- oder Copolymer und mindestens einem Biopolymer, die miteinander mischbar sind;
   d. Mischungen aus mindestens einem synthetischen Homo- oder Copolymer und mindestens einem Biopolymer, die miteinander nicht mischbar sind.
20. Faserflächengebilde nach einer der Ausführungsformen 17 bis 19, wobei die synthetische Polymerkomponente eine Molmasse im Bereich von etwa 500 bis 10.000.000 aufweist.
21. Faserflächengebilde nach einer der vorhergehenden Ausführungsformen, wobei Durchmesser der Wirkstoffträgerfasern 10 nm bis 100 µm, wie 50 nm bis 10 µm, oder 100 nm bis 2 µm, beträgt
22. Faserflächengebilde nach einer der vorhergehenden Ausführungsformen, wobei die Wirkstoffbeladung etwa 0.01 bis 80 Gew.-%, bezogen auf den Feststoffgehalt des Faserflächengebildes beträgt.
23. Faserflächengebilde nach einer der vorhergehenden Ausführungsformen, ausgewählt unter Polymerfasern, Polymerfilmen und Polymervliesstoffen.
24. Faserflächengebilde nach einer der vorhergehenden Ausführungsformen, wobei Trägerpolymerkomponenten und Wirkstoffe nicht-kovalent wechselwirken.
25. Wirkstoffhaltige Formulierung, umfassend ein Faserflächengebilde nach einer der vorhergehenden Ausführungsformen in prozessierter Form, gegebenenfalls in Kombination mit wenigstens einem weiteren Formulierungshilfsmittel.
26. Formulierung nach Ausführungsform 25, umfassend das Faserflächengebilde in zerkleinerter oder nicht-zerkleinerter Form.
27. Formulierung nach Ausführungsform 25 oder 26, umfassend das Faserflächengebilde in kompaktierter Form, in Pulverform oder aufgetragen auf ein Trägersubstrat.
28. Formulierung nach einer der Ausführungsformen 25 bis 27, ausgewählt unter kosmetischen, human- und tierpharmazeutischen, agrochemischen Formulierungen, Nahrungs- und Futtermittelzusätzen.
29. Verwendung eines wirkstoffhaltigen Faserflächengebildes nach einer der Ausführungsformen 1 bis 24 zur Herstellung einer wirkstoffhaltigen Formulierung nach einer der Ausführungsformen 25 bis 28.
30. Verwendung einer wirkstoffhaltigen Formulierung nach einem der Ausführungsformen 25 bis 28 zur kontrollierten Abgabe eines darin enthaltenen Wirkstoffs.
31. Verfahren zur Herstellung eines Faserflächengebildes nach einer der Ausführungsformen 1 bis 24, wobei man
   a.wenigstens einen Wirkstoff zusammen mit wenigstens einer Biopolymerkomponente in einer gemeinsamen flüssigen Phase mischt und
   b.anschließend die Einbettung des Wirkstoffes in die Biopolymerfaser mittels Spinnverfahren durchführt.
32. Verfahren nach Ausführungsform 31, wobei man wenigstens einen Wirkstoff und die Biopolymerkomponente in einer Lösungsmittelphase mischt und aus dieser Mischung verspinnt.
33. Verfahren nach Ausführungsform 31, wobei man wenigstens einen Wirkstoff und die Biopolymerkomponente in einem Gemisch aus wenigstens zwei miteinander mischbaren Lösungsmitteln mischt, wobei Wirkstoffe und Polymere mindestens in einem der Lösungsmittel löslich sind, und man aus dieser Mischung verspinnt.
34. Verfahren nach einer der Ausführungsformen 31 bis 33, wobei das Biopolymer ein amphiphiles, selbstassemblierendes Protein ist, das man mit wenigstens einem Wirkstoff in Ameisensäure mischt und anschließend aus dieser Mischung verspinnt
35. Verfahren nach einer der Ausführungsformen 31 bis 34, wobei es sich bei dem Spinnverfahren um ein Elektrospinnverfahren oder um ein Zentrifugen(Rotor)spinnverfahren handelt.
36. Verfahren nach einer der Ausführungsformen 31 bis 35 wobei man bei einer Temperatur im Bereich von etwa 5 bis 50°C arbeitet.
37. Faserflächengebilde nach einer der Ausführungsformen 1 bis 24, welches im wesentlichen frei ist von niedermolekularen Wirkstoffen.
38. Verwendung eines Faserflächengebildes nach Ausführungsform 37, zu Herstellung einer wirkstoffhaltigen oder wirkstofffreien Formulierung.
39. Verwendung nach Ausführungsform 38, wobei die Formulierung ausgewählt ist unter kosmetischen, human- und tierpharmazeutischen, agrochemischen Formulierungen, Nahrungs- und Futtermittelzusätzen.
40. Faserflächengebilde nach Ausführungsform 37, umfassend einen faserförmigen, polymeren löslichen und/ oder abbaubaren Träger, wobei der Träger als Polymerkomponente wenigstens ein Biopolymer umfasst, das gegebenenfalls zusätzlich chemisch und/oder enzymatisch modifiziert ist, und wobei das Biopolymer ein amphiphiles, selbstassemblierendes Protein, ist.
41. Faserflächengebilde nach Ausführungsform 37 oder 40, wobei das Biopolymer ein Seidenprotein ist, ausgewählt unter dem R16-Protein, umfassend eine Aminosäuresequenz gemäß SEQ ID NO: 4, und dem S16-Protein, umfassend eine Aminosäuresequenz gemäß SEQ ID NO: 6; oder ein von diesen Proteinen abgeleitetes verspinnbares Protein mit einer Sequenzidentität von wenigstens etwa 60%.
42. Verwendung eines Faserflächengebildes nach Ausführungsform 41, zur Herstellung von (medizinischen) Wundversorgungsprodukten und Hygieneartikeln.
43. Wundversorgungsprodukte, hergestellt unter Verwendung eines Faserflächengebildes nach Ausführungsform 41.
44. Hygieneartikel, hergestellt unter Verwendung eines Faserflächengebildes nach Ausführungsform 41.

Auf die Offenbarung der hierin zitierten Druckschriften wird ausdrücklich Bezug genommen.

## Patentansprüche

1. Wirkstoffhaltiges Faserflächengebilde, umfassend einen faserförmigen, polymeren löslichen und/ oder abbaubaren Wirkstoffträger und wenigstens einen, mit dem Träger assoziierten, und von dem Faserflächengebilde freisetzbaren Wirkstoff,
wobei der Träger als Polymerkomponente wenigstens ein Biopolymer umfasst, das gegebenenfalls zusätzlich chemisch und/oder enzymatisch modifiziert ist; und gegebenenfalls
(1) der Träger wenigstens eine weitere Polymerkomponente umfasst, ausgewählt unter synthetischen Polymeren; und/oder
(2) der Träger ein Kompositpolymer ist, ausgewählt ist unter:
a) Mischungen aus mindestens 2 mischbaren Biopolymeren;
b) Mischungen aus mindestens 2 nicht mischbaren Biopolymeren;
c) Mischungen aus mindestens einem synthetischen Homo- oder Copoly-mer und mindestens einem Biopolymer, die miteinander mischbar sind;
d) Mischungen aus mindestens einem synthetischen Homo- oder Copoly-mer und mindestens einem Biopolymer, die miteinander nicht mischbar sind.

2. Faserflächengebilde nach Anspruch 1, wobei der wenigstens eine Wirkstoff in amorpher, teilkristalliner oder kristalliner Form vorliegt.

3. Faserflächengebilde nach einem der vorhergehenden Ansprüche, wobei der Wirkstoff in den Träger integriert und/oder daran adsorbiert ist.

4. Faserflächengebilde nach einem der vorhergehenden Ansprüche, wobei das Biopolymer ausgewählt ist unter dem C16-Spinnenseidenprotein, umfassend eine Aminosäuresequenz gemäß SEQ ID NO: 2;
dem R16-Protein, umfassend eine Aminosäuresequenz gemäß SEQ ID NO: 4; dem S16-Protein, umfassend eine Aminosäuresequenz gemäß SEQ ID NO: 6; oder einem von diesen Proteinen abgeleiteten verspinnbaren Protein mit einer Sequenzidentität von wenigstens etwa 60%.

5. Faserflächengebilde nach einem der Ansprüche 1 bis 4 wobei die synthetische Polymerkomponente eine Molmasse im Bereich von etwa 500 bis 10.000.000 aufweist.

6. Faserflächengebilde nach einem der vorhergehenden Ansprüche, wobei die Wirkstoffbeladung etwa 0.01 bis 80 Gew.-%, bezogen auf den Feststoffgehalt des Faserflächengebildes beträgt.

7. Faserflächengebilde nach einem der vorhergehenden Ansprüche, ausgewählt unter Polymerfasern, Polymerfilmen und Polymervliesstoffen.

8. Faserflächengebilde nach einem der vorhergehenden Ansprüche, wobei Trägerpolymerkomponenten und Wirkstoffe nicht-kovalent wechselwirken.

9. Wirkstoffhaltige Formulierung, umfassend ein Faserflächengebilde nach einem der vorhergehenden Ansprüche in prozessierter Form, gegebenenfalls in Kombination mit wenigstens einem weiteren Formulierungshilfsmittel.

10. Verwendung einer wirkstoffhaltigen Formulierung nach Anspruch 9 zur kontrollierten Abgabe eines darin enthaltenen Wirkstoffs.

11. Verfahren zur Herstellung eines Faserflächengebildes nach einem der Ansprüche 1 bis 8, wobei man
a) wenigstens einen Wirkstoff zusammen mit wenigstens einer Biopolymerkomponente in einer gemeinsamen flüssigen Phase mischt und
b) anschließend die Einbettung des Wirkstoffes in die Biopolymerfaser mittels Spinnverfahren durchführt.

12. Faserflächengebilde nach einem der Ansprüche 1 bis 8, welches im wesentlichen frei ist von niedermolekularen Wirkstoffen.

13. Verwendung eines Faserflächengebildes nach Anspruch 12, zu Herstellung einer wirkstoffhaltigen oder wirkstofffreien Formulierung.

14. Verwendung eines Faserflächengebildes nach Anspruch 12 zur Herstellung von (medizinischen) Wundversorgungsprodukten und Hygieneartikeln.

15. Wundversorgungsprodukte oder Hygieneartikel, hergestellt unter Verwendung eines Faserflächengebildes nach Anspruch 12.


SEQUENCE LISTING

<110>  BASF SE

<120>  Wirkstoffhaltige Fasernflächengebilde auf Basis von Biopolymeren,
        ihre Anwendungen und Verfahren zur ihrer Herstellung

<130>  M/49169

<160>  6

<170>  PatentIn version 3.3

<210>  1
<211>  1731
<212>  DNA
<213>  Artificial

<220>
<223>  Synthetisches Spinnenseidenprotein


<220>
<221>  CDS
<222>  (1)..(1731)

<400>  1
atg gct agc atg act ggt gga cag caa atg ggt cgc gga tcc atg ggt        48
Met Ala Ser Met Thr Gly Gly Gln Gln Met Gly Arg Gly Ser Met Gly
1               5                   10                  15

tct agc gcg gct gca gcc gcg gca gct gcg tcc ggc ccg ggt ggc tac        96
Ser Ser Ala Ala Ala Ala Ala Ala Ala Ala Ser Gly Pro Gly Gly Tyr
            20                  25                  30

ggt ccg gaa aac cag ggt cca tct ggc ccg ggt ggc tac ggt cct ggc       144
Gly Pro Glu Asn Gln Gly Pro Ser Gly Pro Gly Gly Tyr Gly Pro Gly
        35                  40                  45

ggt ccg ggt tct agc gcg gct gca gcc gcg gca gct gcg tcc ggc ccg       192
Gly Pro Gly Ser Ser Ala Ala Ala Ala Ala Ala Ala Ala Ser Gly Pro
    50                  55                  60

ggt ggc tac ggt ccg gaa aac cag ggt cca tct ggc ccg ggt ggc tac       240
Gly Gly Tyr Gly Pro Glu Asn Gln Gly Pro Ser Gly Pro Gly Gly Tyr
65                  70                  75                  80

ggt cct ggc ggt ccg ggt tct agc gcg gct gca gcc gcg gca gct gcg       288
Gly Pro Gly Gly Pro Gly Ser Ser Ala Ala Ala Ala Ala Ala Ala Ala
                85                  90                  95

tcc ggc ccg ggt ggc tac ggt ccg gaa aac cag ggt cca tct ggc ccg       336
Ser Gly Pro Gly Gly Tyr Gly Pro Glu Asn Gln Gly Pro Ser Gly Pro
            100                 105                 110

ggt ggc tac ggt cct ggc ggt ccg ggt tct agc gcg gct gca gcc gcg       384
Gly Gly Tyr Gly Pro Gly Gly Pro Gly Ser Ser Ala Ala Ala Ala Ala
        115                 120                 125

gca gct gcg tcc ggc ccg ggt ggc tac ggt ccg gaa aac cag ggt cca       432
Ala Ala Ala Ser Gly Pro Gly Gly Tyr Gly Pro Glu Asn Gln Gly Pro
    130                 135                 140

tct ggc ccg ggt ggc tac ggt cct ggc ggt ccg ggt tct agc gcg gct 480
Ser Gly Pro Gly Gly Tyr Gly Pro Gly Gly Pro Gly Ser Ser Ala Ala
145                 150                 155                 160

gca gcc gcg gca gct gcg tcc ggc ccg ggt ggc tac ggt ccg gaa aac 528
Ala Ala Ala Ala Ala Ala Ser Gly Pro Gly Gly Tyr Gly Pro Glu Asn
                165                 170                 175

cag ggt cca tct ggc ccg ggt ggc tac ggt cct ggc ggt ccg ggt tct 576
Gln Gly Pro Ser Gly Pro Gly Gly Tyr Gly Pro Gly Gly Pro Gly Ser
            180                 185                 190

agc gcg gct gca gcc gcg gca gct gcg tcc ggc ccg ggt ggc tac ggt 624
Ser Ala Ala Ala Ala Ala Ala Ala Ala Ser Gly Pro Gly Gly Tyr Gly
        195                 200                 205

ccg gaa aac cag ggt cca tct ggc ccg ggt ggc tac ggt cct ggc ggt 672
Pro Glu Asn Gln Gly Pro Ser Gly Pro Gly Gly Tyr Gly Pro Gly Gly
    210                 215                 220

ccg ggt tct agc gcg gct gca gcc gcg gca gct gcg tcc ggc ccg ggt 720
Pro Gly Ser Ser Ala Ala Ala Ala Ala Ala Ala Ala Ser Gly Pro Gly
225                 230                 235                 240

ggc tac ggt ccg gaa aac cag ggt cca tct ggc ccg ggt ggc tac ggt 768
Gly Tyr Gly Pro Glu Asn Gln Gly Pro Ser Gly Pro Gly Gly Tyr Gly
                245                 250                 255

cct ggc ggt ccg ggt tct agc gcg gct gca gcc gcg gca gct gcg tcc 816
Pro Gly Gly Pro Gly Ser Ser Ala Ala Ala Ala Ala Ala Ala Ala Ser
            260                 265                 270

ggc ccg ggt ggc tac ggt ccg gaa aac cag ggt cca tct ggc ccg ggt 864
Gly Pro Gly Gly Tyr Gly Pro Glu Asn Gln Gly Pro Ser Gly Pro Gly
        275                 280                 285

ggc tac ggt cct ggc ggt ccg ggt tct agc gcg gct gca gcc gcg gca 912
Gly Tyr Gly Pro Gly Gly Pro Gly Ser Ser Ala Ala Ala Ala Ala Ala
    290                 295                 300

gct gcg tcc ggc ccg ggt ggc tac ggt ccg gaa aac cag ggt cca tct 960
Ala Ala Ser Gly Pro Gly Gly Tyr Gly Pro Glu Asn Gln Gly Pro Ser
305                 310                 315                 320

ggc ccg ggt ggc tac ggt cct ggc ggt ccg ggt tct agc gcg gct gca 1008
Gly Pro Gly Gly Tyr Gly Pro Gly Gly Pro Gly Ser Ser Ala Ala Ala
                325                 330                 335

gcc gcg gca gct gcg tcc ggc ccg ggt ggc tac ggt ccg gaa aac cag 1056
Ala Ala Ala Ala Ala Ser Gly Pro Gly Gly Tyr Gly Pro Glu Asn Gln
            340                 345                 350

ggt cca tct ggc ccg ggt ggc tac ggt cct ggc ggt ccg ggt tct agc 1104
Gly Pro Ser Gly Pro Gly Gly Tyr Gly Pro Gly Gly Pro Gly Ser Ser
        355                 360                 365

gcg gct gca gcc gcg gca gct gcg tcc ggc ccg ggt ggc tac ggt ccg 1152
Ala Ala Ala Ala Ala Ala Ala Ala Ser Gly Pro Gly Gly Tyr Gly Pro
    370                 375                 380

gaa aac cag ggt cca tct ggc ccg ggt ggc tac ggt cct ggc ggt ccg 1200
Glu Asn Gln Gly Pro Ser Gly Pro Gly Gly Tyr Gly Pro Gly Gly Pro
385                 390                 395                 400

ggt tct agc gcg gct gca gcc gcg gca gct gcg tcc ggc ccg ggt ggc 1248
Gly Ser Ser Ala Ala Ala Ala Ala Ala Ala Ala Ser Gly Pro Gly Gly
                405                 410                 415

tac ggt ccg gaa aac cag ggt cca tct ggc ccg ggt ggc tac ggt cct 1296
Tyr Gly Pro Glu Asn Gln Gly Pro Ser Gly Pro Gly Gly Tyr Gly Pro
            420                 425                 430

ggc ggt ccg ggt tct agc gcg gct gca gcc gcg gca gct gcg tcc ggc 1344
Gly Gly Pro Gly Ser Ser Ala Ala Ala Ala Ala Ala Ala Ala Ser Gly
        435                 440                 445

ccg ggt ggc tac ggt ccg gaa aac cag ggt cca tct ggc ccg ggt ggc 1392
Pro Gly Gly Tyr Gly Pro Glu Asn Gln Gly Pro Ser Gly Pro Gly Gly
    450                 455                 460

tac ggt cct ggc ggt ccg ggt tct agc gcg gct gca gcc gcg gca gct 1440
Tyr Gly Pro Gly Gly Pro Gly Ser Ser Ala Ala Ala Ala Ala Ala Ala
465                 470                 475                 480

gcg tcc ggc ccg ggt ggc tac ggt ccg gaa aac cag ggt cca tct ggc 1488
Ala Ser Gly Pro Gly Gly Tyr Gly Pro Glu Asn Gln Gly Pro Ser Gly
                485                 490                 495

ccg ggt ggc tac ggt cct ggc ggt ccg ggt tct agc gcg gct gca gcc 1536
Pro Gly Gly Tyr Gly Pro Gly Gly Pro Gly Ser Ser Ala Ala Ala Ala
            500                 505                 510

gcg gca gct gcg tcc ggc ccg ggt ggc tac ggt ccg gaa aac cag ggt 1584
Ala Ala Ala Ala Ser Gly Pro Gly Gly Tyr Gly Pro Glu Asn Gln Gly
        515                 520                 525

cca tct ggc ccg ggt ggc tac ggt cct ggc ggt ccg ggt tct agc gcg 1632
Pro Ser Gly Pro Gly Gly Tyr Gly Pro Gly Gly Pro Gly Ser Ser Ala
    530                 535                 540

gct gca gcc gcg gca gct gcg tcc ggc ccg ggt ggc tac ggt ccg gaa 1680
Ala Ala Ala Ala Ala Ala Ala Ser Gly Pro Gly Gly Tyr Gly Pro Glu
545                 550                 555                 560

aac cag ggt cca tct ggc ccg ggt ggc tac ggt cct ggc ggt ccg ggc 1728
Asn Gln Gly Pro Ser Gly Pro Gly Gly Tyr Gly Pro Gly Gly Pro Gly
                565                 570                 575

taa 1731

<210> 2
<211> 576
<212> PRT
<213> Artificial

<220>
<223> Synthetic Construct

<400> 2

Met Ala Ser Met Thr Gly Gly Gln Gln Met Gly Arg Gly Ser Met Gly
1               5                   10                  15

Ser Ser Ala Ala Ala Ala Ala Ala Ala Ala Ser Gly Pro Gly Gly Tyr

20                          25                          30

Gly Pro Glu Asn Gln Gly Pro Ser Gly Pro Gly Gly Tyr Gly Pro Gly
            35                          40                          45

Gly Pro Gly Ser Ser Ala Ala Ala Ala Ala Ala Ala Ala Ser Gly Pro
      50                          55                          60

Gly Gly Tyr Gly Pro Glu Asn Gln Gly Pro Ser Gly Pro Gly Gly Tyr
65                          70                          75                          80

Gly Pro Gly Gly Pro Gly Ser Ser Ala Ala Ala Ala Ala Ala Ala Ala
                        85                          90                          95

Ser Gly Pro Gly Gly Tyr Gly Pro Glu Asn Gln Gly Pro Ser Gly Pro
                  100                         105                         110

Gly Gly Tyr Gly Pro Gly Gly Pro Gly Ser Ser Ala Ala Ala Ala Ala
            115                         120                         125

Ala Ala Ala Ser Gly Pro Gly Gly Tyr Gly Pro Glu Asn Gln Gly Pro
      130                         135                         140

Ser Gly Pro Gly Gly Tyr Gly Pro Gly Gly Pro Gly Ser Ser Ala Ala
145                         150                         155                         160

Ala Ala Ala Ala Ala Ala Ser Gly Pro Gly Gly Tyr Gly Pro Glu Asn
                        165                         170                         175

Gln Gly Pro Ser Gly Pro Gly Gly Tyr Gly Pro Gly Gly Pro Gly Ser
                  180                         185                         190

Ser Ala Ala Ala Ala Ala Ala Ala Ala Ser Gly Pro Gly Gly Tyr Gly
            195                         200                         205

Pro Glu Asn Gln Gly Pro Ser Gly Pro Gly Gly Tyr Gly Pro Gly Gly
      210                         215                         220

Pro Gly Ser Ser Ala Ala Ala Ala Ala Ala Ala Ala Ser Gly Pro Gly
225                         230                         235                         240

Gly Tyr Gly Pro Glu Asn Gln Gly Pro Ser Gly Pro Gly Gly Tyr Gly
                        245                         250                         255

Pro Gly Gly Pro Gly Ser Ser Ala Ala Ala Ala Ala Ala Ala Ala Ser
                  260                         265                         270

Gly Pro Gly Gly Tyr Gly Pro Glu Asn Gln Gly Pro Ser Gly Pro Gly
        275                 280                 285

Gly Tyr Gly Pro Gly Gly Pro Gly Ser Ser Ala Ala Ala Ala Ala Ala
    290                 295                 300

Ala Ala Ser Gly Pro Gly Gly Tyr Gly Pro Glu Asn Gln Gly Pro Ser
305                 310                 315                 320

Gly Pro Gly Gly Tyr Gly Pro Gly Gly Pro Gly Ser Ser Ala Ala Ala
                325                 330                 335

Ala Ala Ala Ala Ala Ser Gly Pro Gly Gly Tyr Gly Pro Glu Asn Gln
            340                 345                 350

Gly Pro Ser Gly Pro Gly Gly Tyr Gly Pro Gly Gly Pro Gly Ser Ser
        355                 360                 365

Ala Ala Ala Ala Ala Ala Ala Ala Ser Gly Pro Gly Gly Tyr Gly Pro
    370                 375                 380

Glu Asn Gln Gly Pro Ser Gly Pro Gly Gly Tyr Gly Pro Gly Gly Pro
385                 390                 395                 400

Gly Ser Ser Ala Ala Ala Ala Ala Ala Ala Ala Ser Gly Pro Gly Gly
                405                 410                 415

Tyr Gly Pro Glu Asn Gln Gly Pro Ser Gly Pro Gly Gly Tyr Gly Pro
            420                 425                 430

Gly Gly Pro Gly Ser Ser Ala Ala Ala Ala Ala Ala Ala Ala Ser Gly
        435                 440                 445

Pro Gly Gly Tyr Gly Pro Glu Asn Gln Gly Pro Ser Gly Pro Gly Gly
    450                 455                 460

Tyr Gly Pro Gly Gly Pro Gly Ser Ser Ala Ala Ala Ala Ala Ala Ala
465                 470                 475                 480

Ala Ser Gly Pro Gly Gly Tyr Gly Pro Glu Asn Gln Gly Pro Ser Gly
                485                 490                 495

Pro Gly Gly Tyr Gly Pro Gly Gly Pro Gly Ser Ser Ala Ala Ala Ala
            500                 505                 510

Ala Ala Ala Ala Ser Gly Pro Gly Gly Tyr Gly Pro Glu Asn Gln Gly
        515                 520                 525

Pro Ser Gly Pro Gly Gly Tyr Gly Pro Gly Gly Pro Gly Ser Ser Ala
    530                 535                 540

Ala Ala Ala Ala Ala Ala Ala Ser Gly Pro Gly Gly Tyr Gly Pro Glu
545                 550                 555                 560

Asn Gln Gly Pro Ser Gly Pro Gly Gly Tyr Gly Pro Gly Gly Pro Gly
                565                 570                 575

<210>  3
<211>  1683
<212>  DNA
<213>  Artificial

<220>
<223>  R16 Seidenprotein

<220>
<221>  CDS
<222>  (1)..(1683)

<400>  3
atg gct agc atg act ggt gga cag caa atg ggt cgc gga tcc atg ggc      48
Met Ala Ser Met Thr Gly Gly Gln Gln Met Gly Arg Gly Ser Met Gly
1               5                   10                  15

ccg ggt tct agc gcg gct gca gcc gcg gca gct gcg tcc ggc ccg ggt      96
Pro Gly Ser Ser Ala Ala Ala Ala Ala Ala Ala Ala Ser Gly Pro Gly
            20                  25                  30

cag ggc cag ggt cag ggt caa ggc cag ggt ggc cgt cct tct gac acc      144
Gln Gly Gln Gly Gln Gly Gln Gly Gln Gly Gly Arg Pro Ser Asp Thr
        35                  40                  45

tac ggc ccg ggt tct agc gcg gct gca gcc gcg gca gct gcg tcc ggc      192
Tyr Gly Pro Gly Ser Ser Ala Ala Ala Ala Ala Ala Ala Ala Ser Gly
    50                  55                  60

ccg ggt cag ggc cag ggt cag ggt caa ggc cag ggt ggc cgt cct tct      240
Pro Gly Gln Gly Gln Gly Gln Gly Gln Gly Gln Gly Gly Arg Pro Ser
65                  70                  75                  80

gac acc tac ggc ccg ggt tct agc gcg gct gca gcc gcg gca gct gcg      288
Asp Thr Tyr Gly Pro Gly Ser Ser Ala Ala Ala Ala Ala Ala Ala Ala
                85                  90                  95

tcc ggc ccg ggt cag ggc cag ggt cag ggt caa ggc cag ggt ggc cgt      336
Ser Gly Pro Gly Gln Gly Gln Gly Gln Gly Gln Gly Gln Gly Gly Arg
            100                 105                 110

cct tct gac acc tac ggc ccg ggt tct agc gcg gct gca gcc gcg gca      384
Pro Ser Asp Thr Tyr Gly Pro Gly Ser Ser Ala Ala Ala Ala Ala Ala
        115                 120                 125

gct gcg tcc ggc ccg ggt cag ggc cag ggt cag ggt caa ggc cag ggt      432
Ala Ala Ser Gly Pro Gly Gln Gly Gln Gly Gln Gly Gln Gly Gln Gly
    130                 135                 140

ggc cgt cct tct gac acc tac ggc ccg ggt tct agc gcg gct gca gcc 480
Gly Arg Pro Ser Asp Thr Tyr Gly Pro Gly Ser Ser Ala Ala Ala Ala
145                 150                 155                 160

gcg gca gct gcg tcc ggc ccg ggt cag ggc cag ggt cag ggt caa ggc 528
Ala Ala Ala Ala Ser Gly Pro Gly Gln Gly Gln Gly Gln Gly Gln Gly
                165                 170                 175

cag ggt ggc cgt cct tct gac acc tac ggc ccg ggt tct agc gcg gct 576
Gln Gly Gly Arg Pro Ser Asp Thr Tyr Gly Pro Gly Ser Ser Ala Ala
            180                 185                 190

gca gcc gcg gca gct gcg tcc ggc ccg ggt cag ggc cag ggt cag ggt 624
Ala Ala Ala Ala Ala Ala Ser Gly Pro Gly Gln Gly Gln Gly Gln Gly
        195                 200                 205

caa ggc cag ggt ggc cgt cct tct gac acc tac ggc ccg ggt tct agc 672
Gln Gly Gln Gly Gly Arg Pro Ser Asp Thr Tyr Gly Pro Gly Ser Ser
    210                 215                 220

gcg gct gca gcc gcg gca gct gcg tcc ggc ccg ggt cag ggc cag ggt 720
Ala Ala Ala Ala Ala Ala Ala Ala Ser Gly Pro Gly Gln Gly Gln Gly
225                 230                 235                 240

cag ggt caa ggc cag ggt ggc cgt cct tct gac acc tac ggc ccg ggt 768
Gln Gly Gln Gly Gln Gly Gly Arg Pro Ser Asp Thr Tyr Gly Pro Gly
                245                 250                 255

tct agc gcg gct gca gcc gcg gca gct gcg tcc ggc ccg ggt cag ggc 816
Ser Ser Ala Ala Ala Ala Ala Ala Ala Ala Ser Gly Pro Gly Gln Gly
            260                 265                 270

cag ggt cag ggt caa ggc cag ggt ggc cgt cct tct gac acc tac ggc 864
Gln Gly Gln Gly Gln Gly Gln Gly Gly Arg Pro Ser Asp Thr Tyr Gly
        275                 280                 285

ccg ggt tct agc gcg gct gca gcc gcg gca gct gcg tcc ggc ccg ggt 912
Pro Gly Ser Ser Ala Ala Ala Ala Ala Ala Ala Ala Ser Gly Pro Gly
    290                 295                 300

cag ggc cag ggt cag ggt caa ggc cag ggt ggc cgt cct tct gac acc 960
Gln Gly Gln Gly Gln Gly Gln Gly Gln Gly Gly Arg Pro Ser Asp Thr
305                 310                 315                 320

tac ggc ccg ggt tct agc gcg gct gca gcc gcg gca gct gcg tcc ggc 1008
Tyr Gly Pro Gly Ser Ser Ala Ala Ala Ala Ala Ala Ala Ala Ser Gly
                325                 330                 335

ccg ggt cag ggc cag ggt cag ggt caa ggc cag ggt ggc cgt cct tct 1056
Pro Gly Gln Gly Gln Gly Gln Gly Gln Gly Gln Gly Gly Arg Pro Ser
            340                 345                 350

gac acc tac ggc ccg ggt tct agc gcg gct gca gcc gcg gca gct gcg 1104
Asp Thr Tyr Gly Pro Gly Ser Ser Ala Ala Ala Ala Ala Ala Ala Ala
        355                 360                 365

tcc ggc ccg ggt cag ggc cag ggt cag ggt caa ggc cag ggt ggc cgt 1152
Ser Gly Pro Gly Gln Gly Gln Gly Gln Gly Gln Gly Gln Gly Gly Arg
    370                 375                 380

cct tct gac acc tac ggc ccg ggt tct agc gcg gct gca gcc gcg gca 1200
Pro Ser Asp Thr Tyr Gly Pro Gly Ser Ser Ala Ala Ala Ala Ala Ala
385                 390                 395                 400

gct gcg tcc ggc ccg ggt cag ggc cag ggt cag ggt caa ggc cag ggt      1248
Ala Ala Ser Gly Pro Gly Gln Gly Gln Gly Gln Gly Gln Gly Gln Gly
                405                 410                 415

ggc cgt cct tct gac acc tac ggc ccg ggt tct agc gcg gct gca gcc      1296
Gly Arg Pro Ser Asp Thr Tyr Gly Pro Gly Ser Ser Ala Ala Ala Ala
            420                 425                 430

gcg gca gct gcg tcc ggc ccg ggt cag ggc cag ggt cag ggt caa ggc      1344
Ala Ala Ala Ala Ser Gly Pro Gly Gln Gly Gln Gly Gln Gly Gln Gly
        435                 440                 445

cag ggt ggc cgt cct tct gac acc tac ggc ccg ggt tct agc gcg gct      1392
Gln Gly Gly Arg Pro Ser Asp Thr Tyr Gly Pro Gly Ser Ser Ala Ala
    450                 455                 460

gca gcc gcg gca gct gcg tcc ggc ccg ggt cag ggc cag ggt cag ggt      1440
Ala Ala Ala Ala Ala Ala Ser Gly Pro Gly Gln Gly Gln Gly Gln Gly
465                 470                 475                 480

caa ggc cag ggt ggc cgt cct tct gac acc tac ggc ccg ggt tct agc      1488
Gln Gly Gln Gly Gly Arg Pro Ser Asp Thr Tyr Gly Pro Gly Ser Ser
                485                 490                 495

gcg gct gca gcc gcg gca gct gcg tcc ggc ccg ggt cag ggc cag ggt      1536
Ala Ala Ala Ala Ala Ala Ala Ala Ser Gly Pro Gly Gln Gly Gln Gly
            500                 505                 510

cag ggt caa ggc cag ggt ggc cgt cct tct gac acc tac ggc ccg ggt      1584
Gln Gly Gln Gly Gln Gly Gly Arg Pro Ser Asp Thr Tyr Gly Pro Gly
        515                 520                 525

tct agc gcg gct gca gcc gcg gca gct gcg tcc ggc ccg ggt cag ggc      1632
Ser Ser Ala Ala Ala Ala Ala Ala Ala Ala Ser Gly Pro Gly Gln Gly
    530                 535                 540

cag ggt cag ggt caa ggc cag ggt ggc cgt cct tct gac acc tac ggc      1680
Gln Gly Gln Gly Gln Gly Gln Gly Gly Arg Pro Ser Asp Thr Tyr Gly
545                 550                 555                 560

taa                                                                  1683


<210>  4
<211>  560
<212>  PRT
<213>  Artificial

<220>
<223>  Synthetic Construct

<400>  4

Met Ala Ser Met Thr Gly Gly Gln Gln Met Gly Arg Gly Ser Met Gly
1               5                   10                  15


Pro Gly Ser Ser Ala Ala Ala Ala Ala Ala Ala Ala Ser Gly Pro Gly
            20                  25                  30


Gln Gly Gln Gly Gln Gly Gln Gly Gln Gly Gly Arg Pro Ser Asp Thr

        35                  40                  45

Tyr Gly Pro Gly Ser Ser Ala Ala Ala Ala Ala Ala Ala Ala Ser Gly
    50                  55                  60

Pro Gly Gln Gly Gln Gly Gln Gly Gln Gly Gln Gly Gly Arg Pro Ser
65                  70                  75                  80

Asp Thr Tyr Gly Pro Gly Ser Ser Ala Ala Ala Ala Ala Ala Ala Ala
                85                  90                  95

Ser Gly Pro Gly Gln Gly Gln Gly Gln Gly Gln Gly Gln Gly Gly Arg
            100                 105                 110

Pro Ser Asp Thr Tyr Gly Pro Gly Ser Ser Ala Ala Ala Ala Ala Ala
        115                 120                 125

Ala Ala Ser Gly Pro Gly Gln Gly Gln Gly Gln Gly Gln Gly Gln Gly
    130                 135                 140

Gly Arg Pro Ser Asp Thr Tyr Gly Pro Gly Ser Ser Ala Ala Ala Ala
145                 150                 155                 160

Ala Ala Ala Ala Ser Gly Pro Gly Gln Gly Gln Gly Gln Gly Gln Gly
                165                 170                 175

Gln Gly Gly Arg Pro Ser Asp Thr Tyr Gly Pro Gly Ser Ser Ala Ala
            180                 185                 190

Ala Ala Ala Ala Ala Ala Ser Gly Pro Gly Gln Gly Gln Gly Gln Gly
        195                 200                 205

Gln Gly Gln Gly Gly Arg Pro Ser Asp Thr Tyr Gly Pro Gly Ser Ser
    210                 215                 220

Ala Ala Ala Ala Ala Ala Ala Ala Ser Gly Pro Gly Gln Gly Gln Gly
225                 230                 235                 240

Gln Gly Gln Gly Gln Gly Gly Arg Pro Ser Asp Thr Tyr Gly Pro Gly
                245                 250                 255

Ser Ser Ala Ala Ala Ala Ala Ala Ala Ala Ser Gly Pro Gly Gln Gly
            260                 265                 270

Gln Gly Gln Gly Gln Gly Gln Gly Gly Arg Pro Ser Asp Thr Tyr Gly
        275                 280                 285

Pro Gly Ser Ser Ala Ala Ala Ala Ala Ala Ala Ala Ser Gly Pro Gly
    290                 295                 300

Gln Gly Gln Gly Gln Gly Gln Gly Gln Gly Gly Arg Pro Ser Asp Thr
305                 310                 315                 320

Tyr Gly Pro Gly Ser Ser Ala Ala Ala Ala Ala Ala Ala Ala Ser Gly
                325                 330                 335

Pro Gly Gln Gly Gln Gly Gln Gly Gln Gly Gln Gly Gly Arg Pro Ser
            340                 345                 350

Asp Thr Tyr Gly Pro Gly Ser Ser Ala Ala Ala Ala Ala Ala Ala Ala
        355                 360                 365

Ser Gly Pro Gly Gln Gly Gln Gly Gln Gly Gln Gly Gln Gly Gly Arg
    370                 375                 380

Pro Ser Asp Thr Tyr Gly Pro Gly Ser Ser Ala Ala Ala Ala Ala Ala
385                 390                 395                 400

Ala Ala Ser Gly Pro Gly Gln Gly Gln Gly Gln Gly Gln Gly Gln Gly
                405                 410                 415

Gly Arg Pro Ser Asp Thr Tyr Gly Pro Gly Ser Ser Ala Ala Ala Ala
            420                 425                 430

Ala Ala Ala Ala Ser Gly Pro Gly Gln Gly Gln Gly Gln Gly Gln Gly
        435                 440                 445

Gln Gly Gly Arg Pro Ser Asp Thr Tyr Gly Pro Gly Ser Ser Ala Ala
    450                 455                 460

Ala Ala Ala Ala Ala Ala Ser Gly Pro Gly Gln Gly Gln Gly Gln Gly
465                 470                 475                 480

Gln Gly Gln Gly Gly Arg Pro Ser Asp Thr Tyr Gly Pro Gly Ser Ser
                485                 490                 495

Ala Ala Ala Ala Ala Ala Ala Ala Ser Gly Pro Gly Gln Gly Gln Gly
            500                 505                 510

Gln Gly Gln Gly Gln Gly Gly Arg Pro Ser Asp Thr Tyr Gly Pro Gly
        515                 520                 525

Ser Ser Ala Ala Ala Ala Ala Ala Ala Ala Ser Gly Pro Gly Gln Gly
    530                 535                 540

Gln Gly Gln Gly Gln Gly Gln Gly Gly Arg Pro Ser Asp Thr Tyr Gly
545                 550                 555                 560

<210>  5
<211>  1923
<212>  DNA
<213>  Artificial

<220>
<223>  S16 Seidenprotein

<220>
<221>  CDS
<222>  (1)..(1923)

<400>  5
atg gct agc atg act ggt gga cag caa atg ggt cgc gga tcc atg ggt      48
Met Ala Ser Met Thr Gly Gly Gln Gln Met Gly Arg Gly Ser Met Gly
1               5                   10                  15

tct gcg gct gca gcc gcg gca gct gcg ggt ccg ggc ggt ggc aac ggt      96
Ser Ala Ala Ala Ala Ala Ala Ala Ala Gly Pro Gly Gly Gly Asn Gly
            20                  25                  30

ggc cgt ccg tct gac acc tac ggt gcg ccg ggt ggc ggt aac ggt ggc      144
Gly Arg Pro Ser Asp Thr Tyr Gly Ala Pro Gly Gly Gly Asn Gly Gly
        35                  40                  45

cgt cct tct tcc tct tac ggt tct gcg gct gca gcc gcg gca gct gcg      192
Arg Pro Ser Ser Ser Tyr Gly Ser Ala Ala Ala Ala Ala Ala Ala Ala
    50                  55                  60

ggt ccg ggc ggt ggc aac ggt ggc cgt ccg tct gac acc tac ggt gcg      240
Gly Pro Gly Gly Gly Asn Gly Gly Arg Pro Ser Asp Thr Tyr Gly Ala
65                  70                  75                  80

ccg ggt ggc ggt aac ggt ggc cgt cct tct tcc tct tac ggt tct gcg      288
Pro Gly Gly Gly Asn Gly Gly Arg Pro Ser Ser Ser Tyr Gly Ser Ala
                85                  90                  95

gct gca gcc gcg gca gct gcg ggt ccg ggc ggt ggc aac ggt ggc cgt      336
Ala Ala Ala Ala Ala Ala Ala Gly Pro Gly Gly Gly Asn Gly Gly Arg
            100                 105                 110

ccg tct gac acc tac ggt gcg ccg ggt ggc ggt aac ggt ggc cgt cct      384
Pro Ser Asp Thr Tyr Gly Ala Pro Gly Gly Gly Asn Gly Gly Arg Pro
        115                 120                 125

tct tcc tct tac ggt tct gcg gct gca gcc gcg gca gct gcg ggt ccg      432
Ser Ser Ser Tyr Gly Ser Ala Ala Ala Ala Ala Ala Ala Ala Gly Pro
    130                 135                 140

ggc ggt ggc aac ggt ggc cgt ccg tct gac acc tac ggt gcg ccg ggt      480
Gly Gly Gly Asn Gly Gly Arg Pro Ser Asp Thr Tyr Gly Ala Pro Gly
145                 150                 155                 160

ggc ggt aac ggt ggc cgt cct tct tcc tct tac ggt tct gcg gct gca      528
Gly Gly Asn Gly Gly Arg Pro Ser Ser Ser Tyr Gly Ser Ala Ala Ala
                165                 170                 175

gcc gcg gca gct gcg ggt ccg ggc ggt ggc aac ggt ggc cgt ccg tct     576
Ala Ala Ala Ala Ala Gly Pro Gly Gly Gly Asn Gly Gly Arg Pro Ser
            180                 185                 190

gac acc tac ggt gcg ccg ggt ggc ggt aac ggt ggc cgt cct tct tcc     624
Asp Thr Tyr Gly Ala Pro Gly Gly Gly Asn Gly Gly Arg Pro Ser Ser
        195                 200                 205

tct tac ggt tct gcg gct gca gcc gcg gca gct gcg ggt ccg ggc ggt     672
Ser Tyr Gly Ser Ala Ala Ala Ala Ala Ala Ala Ala Gly Pro Gly Gly
    210                 215                 220

ggc aac ggt ggc cgt ccg tct gac acc tac ggt gcg ccg ggt ggc ggt     720
Gly Asn Gly Gly Arg Pro Ser Asp Thr Tyr Gly Ala Pro Gly Gly Gly
225                 230                 235                 240

aac ggt ggc cgt cct tct tcc tct tac ggt tct gcg gct gca gcc gcg     768
Asn Gly Gly Arg Pro Ser Ser Ser Tyr Gly Ser Ala Ala Ala Ala Ala
                245                 250                 255

gca gct gcg ggt ccg ggc ggt ggc aac ggt ggc cgt ccg tct gac acc     816
Ala Ala Ala Gly Pro Gly Gly Gly Asn Gly Gly Arg Pro Ser Asp Thr
            260                 265                 270

tac ggt gcg ccg ggt ggc ggt aac ggt ggc cgt cct tct tcc tct tac     864
Tyr Gly Ala Pro Gly Gly Gly Asn Gly Gly Arg Pro Ser Ser Ser Tyr
        275                 280                 285

ggt tct gcg gct gca gcc gcg gca gct gcg ggt ccg ggc ggt ggc aac     912
Gly Ser Ala Ala Ala Ala Ala Ala Ala Ala Gly Pro Gly Gly Gly Asn
    290                 295                 300

ggt ggc cgt ccg tct gac acc tac ggt gcg ccg ggt ggc ggt aac ggt     960
Gly Gly Arg Pro Ser Asp Thr Tyr Gly Ala Pro Gly Gly Gly Asn Gly
305                 310                 315                 320

ggc cgt cct tct tcc tct tac ggt tct gcg gct gca gcc gcg gca gct     1008
Gly Arg Pro Ser Ser Ser Tyr Gly Ser Ala Ala Ala Ala Ala Ala Ala
                325                 330                 335

gcg ggt ccg ggc ggt ggc aac ggt ggc cgt ccg tct gac acc tac ggt     1056
Ala Gly Pro Gly Gly Gly Asn Gly Gly Arg Pro Ser Asp Thr Tyr Gly
            340                 345                 350

gcg ccg ggt ggc ggt aac ggt ggc cgt cct tct tcc tct tac ggt tct     1104
Ala Pro Gly Gly Gly Asn Gly Gly Arg Pro Ser Ser Ser Tyr Gly Ser
        355                 360                 365

gcg gct gca gcc gcg gca gct gcg ggt ccg ggc ggt ggc aac ggt ggc     1152
Ala Ala Ala Ala Ala Ala Ala Ala Gly Pro Gly Gly Gly Asn Gly Gly
    370                 375                 380

cgt ccg tct gac acc tac ggt gcg ccg ggt ggc ggt aac ggt ggc cgt     1200
Arg Pro Ser Asp Thr Tyr Gly Ala Pro Gly Gly Gly Asn Gly Gly Arg
385                 390                 395                 400

cct tct tcc tct tac ggt tct gcg gct gca gcc gcg gca gct gcg ggt     1248
Pro Ser Ser Ser Tyr Gly Ser Ala Ala Ala Ala Ala Ala Ala Ala Gly
                405                 410                 415

ccg ggc ggt ggc aac ggt ggc cgt ccg tct gac acc tac ggt gcg ccg     1296
Pro Gly Gly Gly Asn Gly Gly Arg Pro Ser Asp Thr Tyr Gly Ala Pro
            420                 425                 430

ggt ggc ggt aac ggt ggc cgt cct tct tcc tct tac ggt tct gcg gct      1344
Gly Gly Gly Asn Gly Gly Arg Pro Ser Ser Ser Tyr Gly Ser Ala Ala
        435                 440                 445

gca gcc gcg gca gct gcg ggt ccg ggc ggt ggc aac ggt ggc cgt ccg      1392
Ala Ala Ala Ala Ala Ala Gly Pro Gly Gly Gly Asn Gly Gly Arg Pro
    450                 455                 460

tct gac acc tac ggt gcg ccg ggt ggc ggt aac ggt ggc cgt cct tct      1440
Ser Asp Thr Tyr Gly Ala Pro Gly Gly Gly Asn Gly Gly Arg Pro Ser
465                 470                 475                 480

tcc tct tac ggt tct gcg gct gca gcc gcg gca gct gcg ggt ccg ggc      1488
Ser Ser Tyr Gly Ser Ala Ala Ala Ala Ala Ala Ala Ala Gly Pro Gly
                485                 490                 495

ggt ggc aac ggt ggc cgt ccg tct gac acc tac ggt gcg ccg ggt ggc      1536
Gly Gly Asn Gly Gly Arg Pro Ser Asp Thr Tyr Gly Ala Pro Gly Gly
            500                 505                 510

ggt aac ggt ggc cgt cct tct tcc tct tac ggt tct gcg gct gca gcc      1584
Gly Asn Gly Gly Arg Pro Ser Ser Ser Tyr Gly Ser Ala Ala Ala Ala
        515                 520                 525

gcg gca gct gcg ggt ccg ggc ggt ggc aac ggt ggc cgt ccg tct gac      1632
Ala Ala Ala Ala Gly Pro Gly Gly Gly Asn Gly Gly Arg Pro Ser Asp
    530                 535                 540

acc tac ggt gcg ccg ggt ggc ggt aac ggt ggc cgt cct tct tcc tct      1680
Thr Tyr Gly Ala Pro Gly Gly Gly Asn Gly Gly Arg Pro Ser Ser Ser
545                 550                 555                 560

tac ggt tct gcg gct gca gcc gcg gca gct gcg ggt ccg ggc ggt ggc      1728
Tyr Gly Ser Ala Ala Ala Ala Ala Ala Ala Ala Gly Pro Gly Gly Gly
                565                 570                 575

aac ggt ggc cgt ccg tct gac acc tac ggt gcg ccg ggt ggc ggt aac      1776
Asn Gly Gly Arg Pro Ser Asp Thr Tyr Gly Ala Pro Gly Gly Gly Asn
            580                 585                 590

ggt ggc cgt cct tct tcc tct tac ggt tct gcg gct gca gcc gcg gca      1824
Gly Gly Arg Pro Ser Ser Ser Tyr Gly Ser Ala Ala Ala Ala Ala Ala
        595                 600                 605

gct gcg ggt ccg ggc ggt ggc aac ggt ggc cgt ccg tct gac acc tac      1872
Ala Ala Gly Pro Gly Gly Gly Asn Gly Gly Arg Pro Ser Asp Thr Tyr
    610                 615                 620

ggt gcg ccg ggt ggc ggt aac ggt ggc cgt cct tct tcc tct tac ggc      1920
Gly Ala Pro Gly Gly Gly Asn Gly Gly Arg Pro Ser Ser Ser Tyr Gly
625                 630                 635                 640

taa                                                                  1923


<210>  6
<211>  640
<212>  PRT
<213>  Artificial

<220>
<223>  Synthetic Construct

<400>  6

Met Ala Ser Met Thr Gly Gly Gln Gln Met Gly Arg Gly Ser Met Gly
1               5                   10                  15

Ser Ala Ala Ala Ala Ala Ala Ala Ala Gly Pro Gly Gly Gly Asn Gly
            20                  25                  30

Gly Arg Pro Ser Asp Thr Tyr Gly Ala Pro Gly Gly Gly Asn Gly Gly
        35                  40                  45

Arg Pro Ser Ser Ser Tyr Gly Ser Ala Ala Ala Ala Ala Ala Ala Ala
    50                  55                  60

Gly Pro Gly Gly Gly Asn Gly Gly Arg Pro Ser Asp Thr Tyr Gly Ala
65                  70                  75                  80

Pro Gly Gly Gly Asn Gly Gly Arg Pro Ser Ser Ser Tyr Gly Ser Ala
                85                  90                  95

Ala Ala Ala Ala Ala Ala Ala Gly Pro Gly Gly Gly Asn Gly Gly Arg
            100                 105                 110

Pro Ser Asp Thr Tyr Gly Ala Pro Gly Gly Gly Asn Gly Gly Arg Pro
        115                 120                 125

Ser Ser Ser Tyr Gly Ser Ala Ala Ala Ala Ala Ala Ala Ala Gly Pro
    130                 135                 140

Gly Gly Gly Asn Gly Gly Arg Pro Ser Asp Thr Tyr Gly Ala Pro Gly
145                 150                 155                 160

Gly Gly Asn Gly Gly Arg Pro Ser Ser Ser Tyr Gly Ser Ala Ala Ala
                165                 170                 175

Ala Ala Ala Ala Ala Gly Pro Gly Gly Gly Asn Gly Gly Arg Pro Ser
            180                 185                 190

Asp Thr Tyr Gly Ala Pro Gly Gly Gly Asn Gly Gly Arg Pro Ser Ser
        195                 200                 205

Ser Tyr Gly Ser Ala Ala Ala Ala Ala Ala Ala Ala Gly Pro Gly Gly
    210                 215                 220

Gly Asn Gly Gly Arg Pro Ser Asp Thr Tyr Gly Ala Pro Gly Gly Gly
225                 230                 235                 240

Asn Gly Gly Arg Pro Ser Ser Ser Tyr Gly Ser Ala Ala Ala Ala Ala
                245                 250                 255

Ala Ala Ala Gly Pro Gly Gly Gly Asn Gly Gly Arg Pro Ser Asp Thr
            260                 265                 270

Tyr Gly Ala Pro Gly Gly Gly Asn Gly Gly Arg Pro Ser Ser Ser Tyr
        275                 280                 285

Gly Ser Ala Ala Ala Ala Ala Ala Ala Ala Gly Pro Gly Gly Gly Asn
    290                 295                 300

Gly Gly Arg Pro Ser Asp Thr Tyr Gly Ala Pro Gly Gly Gly Asn Gly
305                 310                 315                 320

Gly Arg Pro Ser Ser Ser Tyr Gly Ser Ala Ala Ala Ala Ala Ala Ala
                325                 330                 335

Ala Gly Pro Gly Gly Gly Asn Gly Gly Arg Pro Ser Asp Thr Tyr Gly
            340                 345                 350

Ala Pro Gly Gly Gly Asn Gly Gly Arg Pro Ser Ser Ser Tyr Gly Ser
        355                 360                 365

Ala Ala Ala Ala Ala Ala Ala Ala Gly Pro Gly Gly Gly Asn Gly Gly
    370                 375                 380

Arg Pro Ser Asp Thr Tyr Gly Ala Pro Gly Gly Gly Asn Gly Gly Arg
385                 390                 395                 400

Pro Ser Ser Ser Tyr Gly Ser Ala Ala Ala Ala Ala Ala Ala Ala Gly
                405                 410                 415

Pro Gly Gly Gly Asn Gly Gly Arg Pro Ser Asp Thr Tyr Gly Ala Pro
            420                 425                 430

Gly Gly Gly Asn Gly Gly Arg Pro Ser Ser Ser Tyr Gly Ser Ala Ala
        435                 440                 445

Ala Ala Ala Ala Ala Ala Gly Pro Gly Gly Gly Asn Gly Gly Arg Pro
    450                 455                 460

Ser Asp Thr Tyr Gly Ala Pro Gly Gly Gly Asn Gly Gly Arg Pro Ser
465                 470                 475                 480

Ser Ser Tyr Gly Ser Ala Ala Ala Ala Ala Ala Ala Ala Gly Pro Gly
                485                 490                 495

Gly Gly Asn Gly Gly Arg Pro Ser Asp Thr Tyr Gly Ala Pro Gly Gly
            500                 505                 510

Gly Asn Gly Gly Arg Pro Ser Ser Ser Tyr Gly Ser Ala Ala Ala Ala
        515                 520                 525

Ala Ala Ala Ala Gly Pro Gly Gly Gly Asn Gly Gly Arg Pro Ser Asp
    530                 535                 540

Thr Tyr Gly Ala Pro Gly Gly Gly Asn Gly Gly Arg Pro Ser Ser Ser
545                 550                 555                 560

Tyr Gly Ser Ala Ala Ala Ala Ala Ala Ala Ala Gly Pro Gly Gly Gly
                565                 570                 575

Asn Gly Gly Arg Pro Ser Asp Thr Tyr Gly Ala Pro Gly Gly Gly Asn
            580                 585                 590

Gly Gly Arg Pro Ser Ser Ser Tyr Gly Ser Ala Ala Ala Ala Ala Ala
        595                 600                 605

Ala Ala Gly Pro Gly Gly Gly Asn Gly Gly Arg Pro Ser Asp Thr Tyr
    610                 615                 620

Gly Ala Pro Gly Gly Gly Asn Gly Gly Arg Pro Ser Ser Ser Tyr Gly
625                 630                 635                 640